# EUROPEAN PATENT APPLICATION

(11) **EP 2 607 352 A1**
(43) Date of publication of application: **26.06.2013**
(21) Application number: 11075275.5
(22) Date of filing: 22.12.2011
(51) Int. Cl.: C07D 211/60, A61K 31/445, A61P 25/24

(54) **Pipecolate-diketoamides for treatment of psychiatric disorders**

(71) Applicant: Max-Planck-Gesellschaft zur Förderung der Wissenschaften e.V., 80539 München (DE)
(72) Inventor: Gopalakrishnan, Ranganath, 380052 Gujarat (IN); Hausch, Felix, 80796 Munich (DE)
(74) Representative: Arth, Hans-Lothar

(57) **Abstract**

The present invention relates to compounds having a pipecolate diketoamide scaffold, pharmaceutically acceptable salts of these compounds and pharmaceutical compositions containing at least one of these compounds together with pharmaceutically acceptable carrier, excipient and/or diluents. Said pipecolate diketoamide compounds can be used for prophylaxis and/or treatment of psychiatric disorders and neurodegenerative diseases, disorders and conditions.

## Description

The present invention relates to pipecolate diketoamide derivatives and stereoisomeric forms, solvates, hydrates and/or pharmaceutically acceptable salts of these compounds as well as pharmaceutical compositions containing at least one of these pipecolate diketoamide derivatives together with pharmaceutically acceptable carrier, excipient and/or diluents. Said pipecolate diketoamide derivatives have been identified as specific inhibitors of the FK506 binding proteins (FKBP's), especially FKBP-51 and FKBP-52, and are useful for the treatment of psychiatric disorders and neurodegenerative diseases, disorders and conditions, for treating vision disorders and/or improving vision; for treating memory impairment and/or enhancing memory performance and for treating alopecia and promoting hair growth.

### Background of the invention

The FK506-binding protein (FKBP) family of immunophilins consists of proteins with a variety of protein-protein interaction domains and versatile cellular functions. This highly conserved protein family binds with immunosuppressive drugs, such as FK506 and rapamycin. This protein family displays peptidyl propyl isomerase (PPlase) activity as seen with cyclophilins and parvulins. FKBP12, a 12kD protein is the most widely studied member of this family.

The immunosuppressant drugs FK506, rapamycin, and cyclosporin are well known as potent T-cell specific immunosuppressants, and are effective against autoimmunity, transplant or graft rejection, inflammation, allergic responses, other autoimmune or immune-mediated diseases, and infectious diseases.

FK506 and rapamycin apart from binding to FKBP12 also interact and inhibit calcineurin(CaN) and mTOR respectively thereby mediating their immunosuppressive action.

The high molecular weight multidomain homologs of FKBP12 (FKBP51/52) act as co chaperons for the heat shock protein (Hsp90) and modulate the signal transduction of the glucocorticoid receptor by participating in the Heat shock protein 90 (Hsp90) - steroid receptor complex.

In this complex, FKBP 51 and 52 modulate the binding competence and signalling of steroid hormone receptors and thereby regulate the cellular responsiveness to circulating hormone levels. This is supported by a natural animal model (squirrel monkey) and by knockout mice, where the essential role of FKPB 51 and 52 on the Glucocorticoid Receptor (GR) activity have been clearly demonstrated. Moreover, polymorphisms in the FKBP51-encoding gene of psychiatric patients have been associated with a faster response to antidepressants, with a higher incidence in depressive episodes and with a higher susceptibility for peritraumatic dissociation.

The immunosuppressive compounds, like FK506, disclosed in the prior art suppress the immune system, by definition, and also exhibit other toxic side effects. Accordingly, there is a need for non-immunosuppressant, small molecule compounds, and compositions and methods for use of such compounds, that are useful in treating psychiatric disorders and neurodegenerative diseases, disorders and conditions.

Further studies led to α-ketoamide analogs of FK506 devoid of immunosuppressive activity. By far there has not been any investigation on the activity of these compounds concerning the larger FKBP's (FKBP51 and 52).

It is the object of the present invention to provide compounds and/or pharmaceutically acceptable salts thereof which inhibit FKBP 51 and FKBP 52 but which show no immunosuppressive activity and are non toxic.

A further aspect of the invention is to provide compounds and/or pharmaceutically acceptable salts thereof which can be used as pharmaceutically active agents, especially for the treatment of psychiatric disorders and neurodegenerative diseases, disorders and conditions, for treating vision disorders and/or improving vision; for treating memory impairment and/or enhancing memory performance and for treating alopecia, as well as compositions comprising at least one of those compounds and/or pharmaceutically acceptable salts thereof as pharmaceutically active ingredients.

The object of the present invention is solved by the teaching of the independent claims. Further advantageous features, aspects and details of the invention are evident from the dependent claims, the description, and the examples of the present application.

### Description of the invention

The diketoamide derivatives according to the present invention are represented by the following general formula (I): 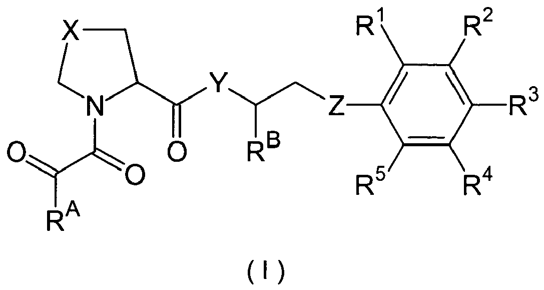 wherein
**X** represents -CH₂-, -CH₂-CH₂-, -CH=CH-, -CH₂-S-;
**Y** represents -NH-, -O-;
**Z** represents -CH₂-, -O-;
**R^{A}** represents -CH₃, -C₂H₅, -C₃H₇, -CH(CH₃)₂, -C₄H₉, -CH₂-CH(CH₃)₂, -_{CH}(CH₃)-C₂H₅, -C(CH₃)₃, -C₅H₁₁, -CH(CH₃)-C₃H₇, -CH₂-CH(CH₃)-C₂H₅, -CH(CH₃)-CH(CH₃)₂, -C(CH₃)₂-C₂H₅, -CH₂-C(CH₃)₃, -CH(C₂H₅)₂, -C₂H₄-CH(CH₃)₂, -C₆H₁₃, -C₇H₁₅, -C₈H₁₇, -C₃H₆-CH(CH₃)₂, -C₂H₄-CH(CH₃)-C₂H₅, -CH(CH₃)-C₄H₉, -CH₂-CH(CH₃)-C₃H₇, -CH(CH₃)-CH₂-CH(CH₃)₂, -CH(CH₃)-CH(CH₃)-C₂H₅, -CH₂-CH(CH₃)-CH(CH₃)₂, -CH₂-C(CH₃)₂-C₂H₅, -C(CH₃)₂-C₃H₇, -C(CH₃)₂-CH(CH₃)₂, -C₂H₄-C(CH₃)₃, -CH(CH₃)-C(CH₃)₃, -CH=CH₂, -CH₂-CH=CH₂, -C(CH₃)=CH₂, -CH=CH-CH₃, -C₂H₄-CH=CH₂, -CH₂-CH=CH-CH₃, -CH=CH-C₂H₅, -CH₂-C(CH₃)=CH₂, -CH(CH₃)-CH=CH, -CH=C(CH₃)₂, -C(CH₃)=CH-CH₃, -CH=CH-CH=CH₂, -C₃H₆-CH=CH₂, -C₂H₄-CH=CH-CH₃, -CH₂-CH=CH-C₂H₅, -CH=CH-C₃H₇, -CH₂-CH=CH-CH=CH₂, -CH=CH-CH=CH-CH₃, -CH=CH-CH₂-CH=CH₂, -C(CH₃)=CH-CH=CH₂, -CH=C(CH₃)-CH=CH₂, -CH=CH-C(CH₃)=CH₂, -C₂H₄-C(CH₃)=CH₂, -CH₂-CH(CH₃)-CH=CH₂, -CH(CH₃)-CH₂-CH=CH₂, -CH₂-CH=C(CH₃)₂, -CH₂-C(CH₃)=CH-CH₃, -CH(CH₃)-CH=CH-CH₃, -CH=CH-CH(CH₃)₂, -CH=C(CH₃)-C₂H₅, -C(CH₃)=CH-C₂H₅, -C(CH₃)=C(CH₃)₂, -C(CH₃)₂-CH=CH₂, -CH(CH₃)-C(CH₃)=CH₂, -C(CH₃)=CH-CH=CH₂, -CH=C(CH₃)-CH=CH₂, -CH=CH-C(CH₃)=CH₂, -C₄H₈-CH=CH₂, -C₃H₆-CH=CH-CH₃, -C₂H₄-CH=CH-C₂H₅, -CH₂-CH=CH-C₃H₇, -CH=CH-C₄H₉, -C₃H₆-C(CH₃)=CH₂, -C₂H₄-CH(CH₃)-CH=CH₂, -CH₂-CH(CH₃)-CH₂-CH=CH₂, -C₂H₄-CH=C(CH₃)₂, -CH(CH₃)-C₂H₄-CH=CH₂, -C₂H₄-C(CH₃)=CH-CH₃, -CH₂-CH(CH₃)-CH=CH-CH₃, -CH(CH₃)-CH₂-CH=CH-CH₃, -CH₂-CH=CH-CH(CH₃)₂, -CH₂-CH=C(CH₃)-C₂H₅, -CH₂-C(CH₃)=CH-C₂H₅, -CH(CH₃)-CH=CH-C₂H₅, -CH=CH-CH₂-CH(CH₃)₂, -CH=CH-CH(CH₃)-C₂H₅, -CH=C(CH₃)-C₃H₇, -C(CH₃)=CH-C₃H₇, -CH₂-CH(CH₃)-C(CH₃)=CH₂, -C[C(CH₃)₃]=CH₂, -CH(CH₃)-CH₂-C(CH₃)=CH₂, -CH(CH₃)-CH(CH₃)-CH=CH₂, -CH=CH-C₂H₄-CH=CH₂, -CH₂-C(CH₃)₂-CH=CH₂, -C(CH₃)₂-CH₂-CH=CH₂, -CH₂-C(CH₃)=C(CH₃)₂, -CH(CH₃)-CH=C(CH₃)₂, -C(CH₃)₂-CH=CH-CH₃, -CH=CH-CH₂-CH=CH-CH₃, -CH(CH₃)-C(CH₃)=CH-CH₃, -CH=C(CH₃)-CH(CH₃)₂, -C(CH₃)=CH-CH(CH₃)₂, -C(CH₃)=C(CH₃)-C₂H₅, -CH=CH-C(CH₃)₃, -C(CH₃)₂-C(CH₃)=CH₂, -CH(C₂H₅)-C(CH₃)=CH₂, -C(CH₃)(C₂H₅)-CH=CH₂, -CH(CH₃)-C(C₂H₅)=CH₂, -CH₂-C(C₃H₇)=CH₂, -CH₂-C(C₂H₅)=CH-CH₃, -CH(C₂H₅)-CH=CH-CH₃, -C(C₄H₉)=CH₂, -C(C₃H₇)=CH-CH₃, -C(C₂H₅)=CH-C₂H₅, -C(C₂H₅)=C(CH₃)₂, -C[CH(CH₃)(C₂H₅)]=CH₂, -C[CH₂-CH(CH₃)₂]=CH₂, -C₂H₄-CH=CH-CH=CH₂, -CH₂-CH=CH-CH₂-CH=CH₂, -C₃H₆-C=C-CH₃, -CH₂-CH=CH-CH=CH-CH₃, -CH=CH-CH=CH-C₂H₅, -CH₂-CH=CH-C(CH₃)=CH₂, -CH₂-CH=C(CH₃)-CH=CH₂, -CH₂-C(CH₃)=CH-CH=CH₂, -CH(CH₃)-CH₂-C≡CH, -CH(CH₃)-CH=CH-CH=CH₂, -CH=CH-CH₂-C(CH₃)=CH₂, -CH(CH₃)-C=C-CH₃, -CH=CH-CH(CH₃)-CH=CH₂, -CH=C(CH₃)-CH₂-CH=CH₂, -C₂H₄-CH(CH₃)-C≡CH, -C(CH₃)=CH-CH₂-CH=CH₂, -CH=CH-CH=C(CH₃)₂, -CH₂-CH(CH₃)-CH₂-C≡CH, -CH=CH-C(CH₃)=CH-CH₃, -CH=C(CH₃)-CH=CH-CH₃, -CH₂-CH(CH₃)-C≡CH, -C(CH₃)=CH-CH=CH-CH₃, -CH=C(CH₃)-C(CH₃)=CH₂, -C(CH₃)=CH-C(CH₃)=CH₂, -C(CH₃)=C(CH₃)-CH=CH₂, -CH=CH-CH=CH-CH=CH₂, -C≡CH, -C≡C-CH₃, -CH₂-C≡CH, -C₂H₄-C≡CH, -CH₂-C≡C-CH₃, -C≡C-C₂H₅, -C₃H₆-C≡CH, -C₂H₄-C≡C-CH₃, -CH₂-C≡C-C₂H₅, -C≡C-C₃H₇, -CH(CH₃)-C≡CH, -C₄H₈-C≡CH, -C₂H₄-C≡C-C₂H₅, -CH₂-C≡C-C₃H₇, -C≡C-C₄H₉, -C≡C-C(CH₃)₃, -CH(CH₃)-C₂H₄-C≡CH, -CH₂-CH(CH₃)-C≡C-CH₃. -CH(CH₃)-CH₂-C≡C-CH₃, -CH(CH₃)-C≡C-C₂H₅, -CH₂-C≡C-CH(CH₃)₂, -C≡C-CH(CH₃)-C₂H₅, -C≡C-CH₂-CH(CH₃)₂, -CH(C₂H₅)-C≡C-CH₃, -C(CH₃)₂-C≡C-CH₃, -CH(C₂H₅)-CH₂-C≡CH, -CH₂-CH(C₂H₅)-C≡CH, -C(CH₃)₂-CH₂-C≡CH, -CH₂-C(CH₃)₂-C≡CH, -CH(CH₃)-CH(CH₃)-C≡CH, -CH(C₃H₇)-C≡CH, -C(CH₃)(C₂H₅)-C≡CH, -CH₂-CH(C≡CH)₂, -C≡C-C≡CH, -CH₂-C≡C-C≡CH, -C≡C-C≡C-CH₃, -CH(C≡CH)_{Z}, -C₂H₄-C≡C-C≡CH, -CH₂-C≡C-CH₂-C≡CH, -C≡C-C₂H₄-C≡CH, -CH₂-C≡C-C≡C-CH₃, -C≡C-CH₂-C≡C-CH₃, -C≡C-C≡C-C₂H₅, -C(C≡CH)₂-CH₃, -C≡C-CH(CH₃)-C≡CH, -CH(CH₃)-C≡C-C≡CH, -CH(C≡CH)-CH₂-C≡CH, -CH(C≡CH)-C≡C-CH₃, 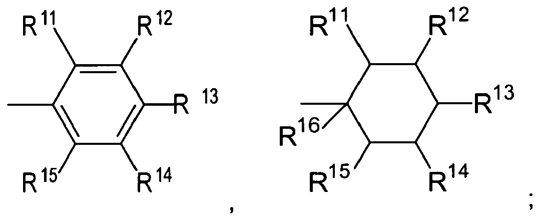 R^{B} represents the following -H or 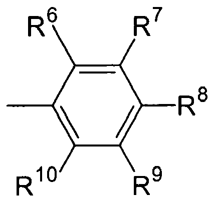 R¹-R¹⁶ represent independently of each other -H, -OH, -OCH₃, -OC₂H₅, -OC₃H₇, -O-cyclo-C₃H₅, -OCH(CH₃)₂, -OC(CH₃)₃, -OC₄H₉, -OCH₂-COOH, -O-(CH₂)ₙ-COOR^{c}, -OPh, -OCH₂-Ph, -OCPh₃, -CH₂-OCH₃, -CH₂-OH, -C₂H₄-OCH₃, -C₃H₆-OCH₃, -CH₂-OC₂H₅, -C₂H₄-OC₂H₅, -C₃H₆-OC₂H₅, -CH₂-OC₃H₇, -C₂H₄-OC₃H₇, -C₃H₆-OC₃H₇, -CH₂-O-cyclo-C₃H₅, -C₂H₄-O-cyclo-C₃H₅, -C₃H₆-O-cyclo-C₃H₅, -CH₂-OCH(CH₃)₂, -C₂H₄-OCH(CH₃)₂, -C₃H₆-OCH(CH₃)₂, -CH₂-OC(CH₃)₃. -C₂H₄-OC(CH₃)₃, -C₃H₆-OC(CH₃)₃, -CH₂-OC₄H₉, -C₂H₄-OC₄H₉, -C₃H₆-OC₄H₉, -CH₂-OPh, -C₂H₄-OPh, -C₃H₆-OPh, -CH₂-OCH₂-Ph, -C₂H₄-OCH₂-Ph, -C₃H₆-OCH₂-Ph, -SH, -SCH₃, -SC₂H₅, -SC₃H₇, -S-cyclo-C₃H₅, -SCH(CH₃)₂, -SC(CH₃)₃, -NO₂, -F, -CI, -Br, -I, -P(O)(OH)₂, -P(O)(OCH₃)₂, -P(O)(OC₂H₅)₂, -P(O)(OCH(CH₃)₂)₂, -C(OH)[P(O)(OH)₂]₂, -Si(CH₃)₂(C(CH₃)₃), -Si(C₂H₅)₃, -Si(CH₃)₃, -N₃, -CN, -OCN, -NCO, -SCN, -NCS, -CHO, -COCH₃, -COC₂H₅, -COC₃H₇, -CO-cyclo-C₃H₅, -COCH(CH₃)₂, -COC(CH₃)₃, -COOH, -COCN, -COOCH₃, -COOC₂H₅, -COOC₃H₇, -COO-cyclo-C₃H₅, -COOCH(CH₃)₂, -COOC(CH₃)₃, -OOC-CH₃, -OOC-C₂H₅, -OOC-C₃H₇, -OOC-cyclo-C₃H₅, -OOC-CH(CH₃)₂, -OOC-C(CH₃)₃, -CONH₂, -CONHCH₃, -CONHC₂H₅, -CONHC₃H₇, -CONH-cyclo-C₃H₅, -CONH[CH(CH₃)₂], -CONH[C(CH₃)₃], -CON(CH₃)₂, -CON(C₂H₅)₂, -CON(C₃H₇)₂, -CON(cyclo-C₃H₅)₂, -CON[CH(CH₃)₂]₂, -CON[C(CH₃)₃]₂, -NHCOCH₃, -NHCOC₂H₅, -NHCOC₃H₇, -NHCO-cyclo-C₃H₅, -NHCO-CH(CH₃)₂, -NHCO-C(CH₃)₃, -NHCO-OCH₃, -NHCO-OC₂H₅, -NHCO-OC₃H₇, -NHCO-O-cyclo-C₃H₅, -NHCO-OCH(CH₃)₂, -NHCO-OC(CH₃)₃, -NH₂, -NHCH₃, -NHC₂H₅, -NHC₃H₇, -NH-cyclo-C₃H₅, -NHCH(CH₃)₂, -NHC(CH₃)₃, -N(CH₃)₂, -N(C₂H₅)₂, -N(C₃H₇)₂, -N(cyclo-C₃H₅)₂, -N[CH(CH₃)₂]₂, -N[C(CH₃)₃]₂, -SOCH₃, -SOC₂H₅, -SOC₃H₇, -SO-cyclo-C₃H₅, -SOCH(CH₃)₂, -SOC(CH₃)₃, -SO₂CH₃, -SO₂C₂H₅, -SO₂C₃H₇, -SO₂-cyclo-C₃H₅, -SO₂CH(CH₃)₂, -SO₂C(CH₃)₃, -SO₃H, -SO₃CH₃, -SO₃C₂H₅, -SO₃C₃H₇, -S0₃-cyclo-C₃H₅, -SO₃CH(CH₃)₂, -SO₃C(CH₃)₃, -SO₂NH₂, -SO₂NHCH₃, -SO₂NHC₂H₅, -SO₂NHC₃H₇, -SO₂NH-cyclo-C₃H₅, -SO₂NHCH(CH₃)₂, -SO₂NHC(CH₃)₃, -SO₂N(CH₃)₂, -SO₂N(C₂H₅)₂, -SO₂N(C₃H₇)₂, -SO₂N(cyclo-C₃H₅)₂, -SO₂N[CH(CH₃)₂]₂, -SO₂N[C(CH₃)₃]₂, -O-S(=O)CH₃, -O-S(=O)C₂H₅, -O-S(=O)C₃H₇, -O-S(=O)-cyclo-C₃H₅, -O-S(=O)CH(CH₃)₂, -O-S(=O)C(CH₃)₃, , -S(=O)(=NH)CH₃, -S(=O)(=NH)C₂H₅, -S(=O)(=NH)C₃H₇, -S(=O)(=NH)-cyclo-C₃H₅, -S(=O)(=NH)CH(CH₃)₂, -S(=O)(=NH)C(CH₃)₃, -NH-SO₂-CH₃, -NH-SO₂-C₂H₅, -NH-SO₂-C₃H₇, -NH-SO₂-cyclo-C₃H₅, -NH-SO₂-CH(CH₃)₂, -NH-SO₂-C(CH₃)₃, -O-SO₂-CH₃, -O-SO₂-C₂H₅, -O-SO₂-C₃H₇, -O-SO₂-cyclo-C₃H₅, -O-SO₂-CH(CH₃)₂, -O-SO₂-C(CH₃)₃, -OCF₃, -CH₂-OCF₃. -C₂H₄-OCF₃, -C₃H₆-OCF₃, -OC₂F₅, -CH₂-OC₂F₅, -C₂H₄-OC₂F₅, -C₃H₆-OC₂F₅, -O-COOCH₃, -O-COOC₂H₅, -O-COOC₃H₇, -O-COO-cyclo-C₃H₅, -O-COOCH(CH₃)₂, -O-COOC(CH₃)₃, -NH-CO-NH₂, -NH-CO-NHCH₃, -NH-CO-NHC₂H₅, -NH-CS-N(C₃H₇)₂, -NH-CO-NHC₃H₇, -NH-CO-N(C₃H₇)₂, -NH-CO-NH[CH(CH₃)₂], -NH-CO-NH[C(CH₃)₃], -NH-CO-N(CH₃)₂, -NH-CO-N(C₂H₅)₂, -NH-CO-NH-cyclo-C₃H₅, -NH-CO-N(cyclo-C₃H₅)₂, -NH-CO-N[CH(CH₃)₂]₂, -NH-CS-N(C₂H₅)₂, -NH-CO-N[C(CH₃)₃]₂, -NH-CS-NH₂, -NH-CS-NHCH₃, -NH-CS-N(CH₃)₂, -NH-CS-NHC₂H₅, -NH-CS-NHC₃H₇, -NH-CS-NH-cyclo-C₃H₅, -NH-CS-NH[CH(CH₃)₂], -NH-CS-NH[C(CH₃)₃], -NH-CS-N(cyclo-C₃H₅)₂, -NH-CS-N[CH(CH₃)₂]₂, -NH-CS-N[C(CH₃)₃]₂, -NH-C(=NH)-NH₂, -NH-C(=NH)-NHCH₃, -NH-C(=NH)-NHC₂H₅, -NH-C(=NH)-NHC₃H₇, -O-CO-NH-cyclo-C₃H₅, -NH-C(=NH)-NH-cyclo-C₃H₅, -NH-C(=NH)-NH[CH(CH₃)₂], -O-CO-NH[CH(CH₃)₂], -NH-C(=NH)-NH[C(CH₃)₃], -NH-C(=NH)-N(CH₃)₂, -NH-C(=NH)-N(C₂H₅)₂, -NH-C(=NH)-N(C₃H₇)₂, -NH-C(=NH)-N(cyclo-C₃H₅)₂, -O-CO-NHC₃H₇, -NH-C(=NH)-N[CH(CH₃)₂]₂, -NH-C(=NH)-N[C(CH₃)₃]₂. -O-CO-NH₂, -O-CO-NHCH₃, -O-CO-NHC₂H₅, -O-CO-NH[C(CH₃)₃], -O-CO-N(CH₃)₂, -O-CO-N(C₂H₅)₂, -O-CO-N(C₃H₇)_{2,} -O-CO-N(cyclo-C₃H₅)₂, -O-CO-N[CH(CH₃)₂]₂, -O-CO-N[C(CH₃)₃]₂, -O-CO-OCH₃, -O-CO-OC₂H₅, -O-CO-OC₃H₇, -O-CO-O-cyclo-C₃H₅, -O-CO-OCH(CH₃)₂, -O-CO-OC(CH₃)₃, -CH₂F, -CHF₂, -CF₃, -CH₂Cl, -CH₂Br, -CH₂l, -CH₂-CH₂F, -CH₂-CHF₂, -CH₂-CF₃, -CH₂-CH₂Cl, -CH₂-CH₂Br, -CH₂-CH₂l, -cyclo-C₈H₁₅, -Ph, -CH₂-Ph, -CH₂-CH₂-Ph, -CH=CH-Ph, -CPh₃, -CH₃, -C₂H₅, -C₃H₇, -CH(CH₃)₂, -C₄H₉, -CH₂-CH(CH₃)₂, -CH(CH₃)-C₂H₅, -C(CH₃)₃, -C₅H₁₁, -CH(CH₃)-C₃H₇, -CH₂-CH(CH₃)-C₂H₅, -CH(CH₃)-CH(CH₃)₂, -C(CH₃)₂-C₂H₅, -CH₂-C(CH₃)₃, -CH(C₂H₅)₂, -C₂H₄-CH(CH₃)₂, -C₆H₁₃, -C₇H₁₅, -C₈H₁₇, -C₃H₆-CH(CH₃)₂, -C₂H₄-CH(CH₃)-C₂H₅, -CH(CH₃)-C₄H₉, -CH₂-CH(CH₃)-C₃H₇, -CH(CH₃)-CH₂-CH(CH₃)₂, -CH(CH₃)-CH(CH₃)-C₂H₅, -CH₂-CH(CH₃)-CH(CH₃)₂, -CH₂-C(CH₃)₂-C₂H₅, -C(CH₃)₂-C₃H₇, -C(CH₃)₂-CH(CH₃)₂, -C₂H₄-C(CH₃)₃, -CH(CH₃)-C(CH₃)₃, -CH=CH₂, -CH₂-CH=CH₂, -C(CH₃)=CH₂, -CH=CH-CH₃, -C₂H₄-CH=CH₂, -CH₂-CH=CH-CH₃, -CH=CH-C₂H₅, -CH₂-C(CH₃)=CH₂, -CH(CH₃)-CH=CH, -CH=C(CH₃)₂, -C(CH₃)=CH-CH₃, -CH=CH-CH=CH₂, -C₃H₆-CH=CH₂, -C₂H₄-CH=CH-CH₃, -CH₂-CH=CH-C₂H₅, -CH=CH-C₃H₇, -CH₂-CH=CH-CH=CH₂, -CH=CH-CH=CH-CH₃, -CH=CH-CH₂-CH=CH₂, -C(CH₃)=CH-CH=CH₂, -CH=C(CH₃)-CH=CH₂, -CH=CH-C(CH₃)=CH₂, -C₂H₄-C(CH₃)=CH₂, -CH₂-CH(CH₃)-CH=CH₂, -CH(CH₃)-CH₂-CH=CH₂, -CH₂-CH=C(CH₃)₂, -CH₂-C(CH₃)=CH-CH₃, -CH(CH₃)-CH=CH-CH₃, -CH=CH-CH(CH₃)₂, -CH=C(CH₃)-C₂H₅, -C(CH₃)=CH-C₂H₅, -C(CH₃)=C(CH₃)₂, -C(CH₃)₂-CH=CH₂, -CH(CH₃)-C(CH₃)=CH₂, -C(CH₃)=CH-CH=CH₂, -CH=C(CH₃)-CH=CH₂, -CH=CH-C(CH₃)=CH₂, -C₄H₈-CH=CH₂, -C₃H₆-CH=CH-CH₃, -C₂H₄-CH=CH-C₂H₅, -CH₂-CH=CH-C₃H₇, -CH=CH-C₄H₉, -C₃H₆-C(CH₃)=CH₂, -C₂H₄-CH(CH₃)-CH=CH₂, -CH₂-CH(CH₃)-CH₂-CH=CH₂, -C₂H₄-CH=C(CH₃)₂, -CH(CH₃)-C₂H₄-CH=CH₂, -C₂H₄-C(CH₃)=CH-CH₃, -CH₂-CH(CH₃)-CH=CH-CH₃, -CH(CH₃)-CH₂-CH=CH-CH₃, -CH₂-CH=CH-CH(CH₃)₂, -CH₂-CH=C(CH₃)-C₂H₅, -CH₂-C(CH₃)=CH-C₂H₅, -CH(CH₃)-CH=CH-C₂H₅, -CH=CH-CH₂-CH(CH₃)₂, -CH=CH-CH(CH₃)-C₂H₅, -CH=C(CH₃)-C₃H₇, -C(CH₃)=CH-C₃H₇, -CH₂-CH(CH₃)-C(CH₃)=CH₂, -C[C(CH₃)₃]=CH₂, -CH(CH₃)-CH₂-C(CH₃)=CH₂, -CH(CH₃)-CH(CH₃)-CH=CH₂, -CH=CH-C₂H₄-CH=CH₂, -CH₂-C(CH₃)₂-CH=CH₂, -C(CH₃)₂-CH₂-CH=CH₂, -CH₂-C(CH₃)=C(CH₃)₂, -CH(CH₃)-CH=C(CH₃)₂, -C(CH₃)₂-CH=CH-CH₃, -CH=CH-CH₂-CH=CH-CH₃, -CH(CH₃)-C(CH₃)=CH-CH₃, -CH=C(CH₃)-CH(CH₃)₂, -C(CH₃)=CH-CH(CH₃)₂, -C(CH₃)=C(CH₃)-C₂H₅, -CH=CH-C(CH₃)₃, -C(CH₃)₂-C(CH₃)=CH₂, -CH(C₂H₅)-C(CH₃)=CH₂, -C(CH₃)(C₂H₅)-CH=CH₂, -CH(CH₃)-C(C₂H₅)=CH₂, -CH₂-C(C₃H₇)=CH₂, -CH₂-C(C₂H₅)=CH-CH₃, -CH(C₂H₅)-CH=CH-CH₃, -C(C₄H₉)=CH₂, -C(C₃H₇)=CH-CH₃, -C(C₂H₅)=CH-C₂H₅, -C(C₂H₅)=C(CH₃)₂, -C[CH(CH₃)(C₂H₅)]=CH₂, -C[CH₂-CH(CH₃)₂]=CH₂, -C₂H₄-CH=CH-CH=CH₂, -CH₂-CH=CH-CH₂-CH=CH₂, -C₃H₆-C≡C-CH₃, -CH₂-CH=CH-CH=CH-CH₃, -CH=CH-CH=CH-C₂H₅, -CH₂-CH=CH-C(CH₃)=CH₂, -CH₂-CH=C(CH₃)-CH=CH₂, -CH₂-C(CH₃)=CH-CH=CH₂, -CH(CH₃)-CH₂-C≡CH, -CH(CH₃)-CH=CH-CH=CH₂, -CH=CH-CH₂-C(CH₃)=CH₂, -CH(CH₃)-C≡C-CH₃, -CH=CH-CH(CH₃)-CH=CH₂, -CH=C(CH₃)-CH₂-CH=CH₂, -C₂H₄-CH(CH₃)-C≡CH, -C(CH₃)=CH-CH₂-CH=CH₂, -CH=CH-CH=C(CH₃)₂, -CH₂-CH(CH₃)-CH₂-C≡CH, -CH=CH-C(CH₃)=CH-CH₃, -CH=C(CH₃)-CH=CH-CH₃, -CH₂-CH(CH₃)-C≡CH, -C(CH₃)=CH-CH=CH-CH₃, -CH=C(CH₃)-C(CH₃)=CH₂, -C(CH₃)=CH-C(CH₃)=CH₂, -C(CH₃)=C(CH₃)-CH=CH₂, -CH=CH-CH=CH-CH=CH₂, -C≡CH, -C≡C-CH₃, -CH₂-C≡CH, -C₂H₄-C≡CH, -CH₂-C≡C-CH₃, -C≡C-C₂H₅, -C₃H₆-C≡CH, -C₂H₄-C≡C-CH₃, -CH₂-C≡C-C₂H₅, -C≡C-C₃H₇, -CH(CH₃)-C≡CH, -C₄H₈-C≡CH, -C₂H₄-C≡C-C₂H₅, -CH₂-C≡C-C₃H₇, -C≡C-C₄H₉, -C≡C-C(CH₃)₃, -CH(CH₃)-C₂H₄-C≡CH, -CH₂-CH(CH₃)-C≡C-CH₃, -CH(CH₃)-CH₂-C≡C-CH₃, -CH(CH₃)-C≡C-C₂H₅, -CH₂-C≡C-CH(CH₃)₂, -C≡C-CH(CH₃)-C₂H₅, -C≡C-CH₂-CH(CH₃)₂, -CH(C₂H₅)-C≡C-CH₃, -C(CH₃)₂-C≡C-CH₃, -CH(C₂H₅)-CH₂-C≡CH, -CH₂-CH(C₂H₅)-C≡CH, -C(CH₃)₂-CH₂-C≡CH, -CH₂-C(CH₃)₂-C≡CH, -CH(CH₃)-CH(CH₃)-C≡CH, -CH(C₃H₇)-C≡CH, -C(CH₃)(C₂H₅)-C≡CH, -CH₂-CH(C≡CH)₂, -C≡C-C≡CH, -CH₂-C≡C-C≡CH, -C≡C-C≡C-CH₃, -CH(C≡CH)₂, -C₂H₄-C≡C-C≡CH, -CH₂-C≡C-CH₂-C≡CH, -C≡C-C₂H₄-C≡CH, -CH₂-C≡C-C≡C-CH₃, -C≡C-CH₂-C≡C-CH₃, -C≡C-C≡C-C₂H₅, -C(C≡CH)₂-CH₃, -C≡C-CH(CH₃)-C≡CH, -CH(CH₃)-C≡C-C≡CH, -CH(C≡CH)-CH₂-C≡CH, -CH(C≡CH)-C≡C-CH₃, 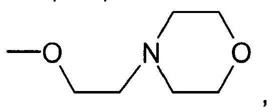 R^{C} represents -CH₂-OCH₃, -C₂H₄-OCH₃, -C₃H₆-OCH₃, -CH₂-OC₂H₅, -C₂H₄-OC₂H₅, -C₃H₅-OC₂H₅, -CH₂-OC₃H₇, -C₂H₄-OC₃H₇, -C₃H₆-OC₃H₇, -CH₂-O-cyclo-C₃H₅, -C₂H₄-O-cyclo-C₃H₅, -C₃H₆-O-cyclo-C₃H₅, -CH₂-OCH(CH₃)₂, -C₂H₄-OCH(CH₃)₂, -C₃H₆-OCH(CH₃)₂, -CH₂-OC(CH₃)₃, -C₂H₄-OC(CH₃)₃, -C₃H₆-OC(CH₃)₃, -CH₂-OC₄H₉, -C₂H₄-OC₄H₉, -C₃H₆-OC₄H₉, -CH₂-OPh, -C₂H₄-OPh, -C₃H₆-OPh, -CH₂-OCH₂-Ph, -C₂H₄-OCH₂-Ph, -C₃H₆-OCH₂-Ph, -CH₂F, -CHF₂, -CF₃, -CH₂Cl, -CH₂Br, -CH₂l, -CH₂-CH₂F, -CH₂-CHF₂, -CH₂-CF₃, -CH₂-CH₂Cl, -CH₂-CH₂Br, -CH₂-CH₂l, -cyclo-C₈H₁₅, -Ph, -CH₂-Ph, -CH₂-CH₂-Ph, -CH=CH-Ph, -CPh₃, -CH₃, -C₂H₅, -C₃H₇, -CH(CH₃)₂, -C₄H₉, -CH₂-CH(CH₃)₂, -CH(CH₃)-C₂H₅, -C(CH₃)₃, -C₅H₁₁, -CH(CH₃)-C₃H₇, -CH₂-CH(CH₃)-C₂H₅, -CH(CH₃)-CH(CH₃)₂, -C(CH₃)₂-C₂H₅, -CH₂-C(CH₃)₃, -CH(C₂H₅)₂, -C₂H₄-CH(CH₃)₂, -C₆H₁₃, -C₇H₁₅, -C₈H₁₇, -C₃H₆-CH(CH₃)₂, -C₂H₄-CH(CH₃)-C₂H₅. -CH(CH₃)-C₄H₉, -CH₂-CH(CH₃)-C₃H₇, -CH(CH₃)-CH₂-CH(CH₃)₂, -CH(CH₃)-CH(CH₃)-C₂H₅, -CH₂-CH(CH₃)-CH(CH₃)₂, -CH₂-C(CH₃)₂-C₂H₅, -C(CH₃)₂-C₃H₇, -C(CH₃)₂-CH(CH₃)₂, -C₂H₄-C(CH₃)₃, -CH(CH₃)-C(CH₃)₃. -CH=CH₂, -CH₂-CH=CH₂, -C(CH₃)=CH₂, -CH=CH-CH₃, -C₂H₄-CH=CH₂, -CH₂-CH=CH-CH₃, -CH=CH-C₂H₅, -CH₂-C(CH₃)=CH₂, -CH(CH₃)-CH=CH, -CH=C(CH₃)₂, -C(CH₃)=CH-CH₃, -CH=CH-CH=CH₂, - C₃H₆-CH=CH₂, -C₂H₄-CH=CH-CH₃, -CH₂-CH=CH-C₂H₅, -CH=CH-C₃H₇, -CH₂-CH=CH-CH=CH₂, -CH=CH-CH=CH-CH₃, -CH=CH-CH₂-CH=CH₂, -C(CH₃)=CH-CH=CH₂, -CH=C(CH₃)-CH=CH₂, -CH=CH-C(CH₃)=CH₂, -C₂H₄-C(CH₃)=CH₂, -CH₂-CH(CH₃)-CH=CH₂, -CH(CH₃)-CH₂-CH=CH₂, -CH₂-CH=C(CH₃)₂, -CH₂-C(CH₃)=CH-CH₃, -CH(CH₃)-CH=CH-CH₃, -CH=CH-CH(CH₃)₂, -CH=C(CH₃)-C₂H₅, -C(CH₃)=CH-C₂H₅, -C(CH₃)=C(CH₃)₂, -C(CH₃)₂-CH=CH₂, -CH(CH₃)-C(CH₃)=CH₂, -C(CH₃)=CH-CH=CH₂, -CH=C(CH₃)-CH=CH₂, -CH=CH-C(CH₃)=CH₂, -C₄H₈-CH=CH₂, -C₃H₆-CH=CH-CH₃, -C₂H₄-CH=CH-C₂H₅, -CH₂-CH=CH-C₃H₇, -CH=CH-C₄H₉, -C₃H₆-C(CH₃)=CH₂, -C₂H₄-CH(CH₃)-CH=CH₂, -CH₂-CH(CH₃)-CH₂-CH=CH₂, -C₂H₄-CH=C(CH₃)₂, -CH(CH₃)-C₂H₄-CH=CH₂, -C₂H₄-C(CH₃)=CH-CH₃, -CH₂-CH(CH₃)-CH=CH-CH₃, -CH(CH₃)-CH₂-CH=CH-CH₃, -CH₂-CH=CH-CH(CH₃)₂, -CH₂-CH=C(CH₃)-C₂H₅, -CH₂-C(CH₃)=CH-C₂H₅, -CH(CH₃)-CH=CH-C₂H₅, -CH=CH-CH₂-CH(CH₃)₂, -CH=CH-CH(CH₃)-C₂H₅, -CH=C(CH₃)-C₃H₇, -C(CH₃)=CH-C₃H₇, -CH₂-CH(CH₃)-C(CH₃)=CH₂, -C[C(CH₃)₃]=CH₂, -CH(CH₃)-CH₂-C(CH₃)=CH₂, -CH(CH₃)-CH(CH₃)-CH=CH₂, -CH=CH-C₂H₄-CH=CH₂, -CH₂-C(CH₃)₂-CH=CH₂, -C(CH₃)₂-CH₂-CH=CH₂, -CH₂-C(CH₃)=C(CH₃)₂, -CH(CH₃)-CH=C(CH₃)₂, -C(CH₃)₂-CH=CH-CH₃, -CH=CH-CH₂-CH=CH-CH₃, -CH(CH₃)-C(CH₃)=CH-CH₃, -CH=C(CH₃)-CH(CH₃)₂, -C(CH₃)=CH-CH(CH₃)₂, -C(CH₃)₌C(CH₃)-C₂H₅, -CH=CH-C(CH₃)₃, -C(CH₃)₂-C(CH₃)=CH₂, -CH(C₂H₅)-C(CH₃)=CH₂, -C(CH₃)(C₂H₅)-CH=CH₂, -CH(CH₃)-C(C₂H₅)=CH₂, -CH₂-C(C₃H₇)=CH₂, -CH₂-C(C₂H₅)=CH-CH₃, -CH(C₂H₅)-CH=CH-CH₃, -C(C₄H₉)=CH₂, -C(C₃H₇)=CH-CH₃, -C(C₂H₅)=CH-C₂H₅, -C(C₂H₅)=C(CH₃)₂, -C[CH(CH₃)(C₂H₅)]=CH₂, -C[CH₂-CH(CH₃)₂]=CH₂, -C₂H₄-CH=CH-CH=CH₂, -CH₂-CH=CH-CH₂-CH=CH₂, -C₃H₆-C≡C-CH₃, -CH₂-CH=CH-CH=CH-CH₃, -CH=CH-CH=CH-C₂H₅, -CH₂-CH=CH-C(CH₃)=CH₂, -CH₂-CH=C(CH₃)-CH=CH₂, -CH₂-C(CH₃)=CH-CH=CH_{2,} -CH(CH₃)-CH₂-C≡CH, -CH(CH₃)-CH=CH-CH=CH₂, -CH=CH-CH₂-C(CH₃)=CH₂, -CH(CH₃)-C≡C-CH₃, -CH=CH-CH(CH₃)-CH=CH₂, -CH=C(CH₃)-CH₂-CH=CH₂, -C₂H₄-CH(CH₃)-C≡CH, -C(CH₃)=CH-CH₂-CH=CH₂, -CH=CH-CH=C(CH₃)₂, -CH₂-CH(CH₃)-CH₂-C≡CH, -CH=CH-C(CH₃)=CH-CH₃, -CH=C(CH₃)-CH=CH-CH₃, -CH₂-CH(CH₃)-C≡CH, -C(CH₃)=CH-CH=CH-CH_{3,} -CH=C(CH₃)-C(CH₃)=CH₂, -C(CH₃)=CH-C(CH₃)=CH₂, -C(CH₃)=C(CH₃)-CH=CH₂, -CH=CH-CH=CH-CH=CH₂, -C≡CH, -C≡C-CH₃, -CH₂-C≡CH, -C₂H₄-C≡CH, -CH₂-C≡C-CH₃, -C≡C-C₂H₅, -C₃H₆-C≡CH, -C₂H₄-C≡C-CH₃, -CH₂-C≡C-C₂H₅, -C≡C-C₃H₇, -CH(CH₃)-C≡CH, -C₄H₈-C≡CH, -C₂H₄-C≡C-C₂H₅, -CH₂-C≡C-C₃H₇, -C≡C-C₄H₉, -C≡C-C(CH₃)₃, -CH(CH₃)-C₂H₄-C≡CH, -CH₂-CH(CH₃)-C≡C-CH₃, -CH(CH₃)-CH₂-C≡C-CH₃, -CH(CH₃)-C≡C-C₂H₅, -CH₂-C≡C-CH(CH₃)₂, -C≡C-CH(CH₃)-C₂H₅, -C≡C-CH₂-CH(CH₃)₂, -CH(C₂H₅)-C≡C-CH₃, -C(CH₃)₂-C≡C-CH₃, -CH(C₂H₅)-CH₂-C≡CH, -CH₂-CH(C₂H₅)-C≡CH, -C(CH₃)₂-CH₂-C≡CH, -CH₂-C(CH₃)₂-C≡CH, -CH(CH₃)-CH(CH₃)-C≡CH, -CH(C₃H₇)-C≡CH, -C(CH₃)(C₂H₅)-C≡CH, -CH₂-CH(C≡CH)₂, -C≡C-C≡CH, -CH₂-C≡C-C≡CH, -C≡C-C≡C-CH₃, -CH(C≡CH)₂, -C₂H₄-C≡C-C≡CH, -CH₂-C≡C-CH₂-C≡CH, -C≡C-C₂H₄-C≡CH, -CH₂-C≡C-C≡C-CH₃, -C≡C-CH₂-C≡C-CH₃, -C≡C-C≡C-C₂H₅, -C(C≡CH)₂-CH₃, -C≡C-CH(CH₃)-C≡CH, -CH(CH₃)-C≡C-C≡CH, -CH(C≡CH)-CH₂-C≡CH, -CH(C≡CH)-C≡C-CH₃;
n is 1, 2, 3 or 4,
and enantiomers, stereoisomeric forms, mixtures of enantiomers, anomers, deoxy-forms, diastereomers, mixtures of diastereomers, prodrugs, tautomers, hydrates, solvates and racemates of the above mentioned compounds and pharmaceutically acceptable salts thereof for use as an inhibitor of the FK506-binding protein 51 and 52 (FKBP51, FKBP52).

FKBP inhibitors as used herein is defined as compounds that inhibit the peptidyl-prolyl isomerase activity of FKBPs (PPlase inhbitors, also referred to as rotamase inhibitors) and compounds that displace FK506 or FK506 analogs form the PPlase active site of FKBPs.

The expression prodrug is defined as a pharmacological substance, a drug, which is administered in an inactive or significantly less active form. Once administered, the prodrug is metabolized in the body in vivo into the active compound.

The expression tautomer is defined as an organic compound that is interconvertible by a chemical reaction called tautomerization. Tautomerization can be catalyzed preferably by bases or acids or other suitable compounds.

Preferred substituents for **R^{A}** are: -C₅H₁₁, -CH(CH₃)-C₃H₇, -CH₂-CH(CH₃)-C₂H₅, -CH(CH₃)-CH(CH₃)₂, -C(CH₃)₂-C₂H₅, -CH₂-C(CH₃)₃, -CH(C₂H₅)₂, -C₂H₄-CH(CH₃)₂, 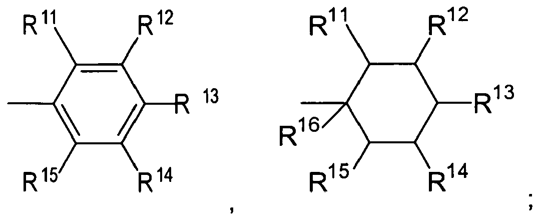 Preferred substituents for **R¹-R¹⁶** are: -H, -OH, -OCH₃, -OC₂H₅, -OC₃H₇, -O-cyclo-C₃H₅, -OCH(CH₃)₂, -OC(CH₃)₃, -OC₄H₉, -OCH₂-COOH, -O-(CH₂)ₙ-COO**R^{c}** (with n = 1, 2, 3 or 4), -CH₃, -CH₂-OH, -C₂H₅, -C₃H₇, -CH(CH₃)₂, -C₄H₉, -CH₂-CH(CH₃)₂, -CH(CH₃)-C₂H₅, -C(CH₃)₃, -C₅H₁₁, -CH(CH₃)-C₃H₇, -CH₂-CH(CH₃)-C₂H₅, -CH(CH₃)-CH(CH₃)₂, -C(CH₃)₂-C₂H₅, -CH₂-C(CH₃)₃, -CH(C₂H₅)₂, -C₂H₄-CH(CH₃)₂, -C₆H₁₃, -C₇H₁₅, -C₈H₁₇, -C₃H₆-CH(CH₃)₂, -C₂H₄-CH(CH₃)-C₂H₅, -CH(CH₃)-C₄H₉, -CH₂-CH(CH₃)-C₃H₇, -CH(CH₃)-CH₂-CH(CH₃)₂, -CH(CH₃)-CH(CH₃)-C₂H₅, -CH₂-CH(CH₃)-CH(CH₃)₂, -CH₂-C(CH₃)₂-C₂H₅, -C(CH₃)₂-C₃H₇, -C(CH₃)₂-CH(CH₃)₂, -C₂H₄-C(CH₃)₃, -CH(CH₃)-C(CH₃)₃,

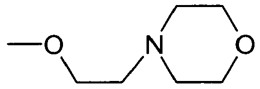

Preferred substituents for **R^{c}** are:
-CH₃, -C₂H₅, -C₃H₇, -CH(CH₃)₂, -C₄H₉, -CH₂-CH(CH₃)₂, -CH(CH₃)-C₂H₅, -C(CH₃)₃, -C₅H₁₁, -CH(CH₃)-C₃H₇, -CH₂-CH(CH₃)-C₂H₅, -CH(CH₃)-CH(CH₃)₂, -C(CH₃)₂-C₂H₅, -CH₂-C(CH₃)₃, -CH(C₂H₅)₂, -C₂H₄-CH(CH₃)₂, -C₆H₁₃, -C₇H₁₅, -C₈H₁₇, -C₃H₆-CH(CH₃)₂, -C₂H₄-CH(CH₃)-C₂H₅, -CH(CH₃)-C₄H₉, -CH₂-CH(CH₃)-C₃H₇, -CH(CH₃)-CH₂-CH(CH₃)₂, -CH(CH₃)-CH(CH₃)-C₂H₅, -CH₂-CH(CH₃)-CH(CH₃)₂, -CH₂-C(CH₃)₂-C₂H₅, -C(CH₃)₂-C₃H₇, -C(CH₃)₂-CH(CH₃)₂, -C₂H₄-C(CH₃)₃, -CH(CH₃)-C(CH₃)₃.

Preferred are the following compounds of the general formula (I):
2-(3-((R)-3-(3,4-dimethoxyphenyl)-1-((S)-1-(2-oxo-2-(3,4,5-trimethoxyphen yl)acetyl)piperidine-2-carbonyloxy)propyl)phenoxy)acetic acid
(S)-((R) -3-(3,4-dimethoxyphenyl) -1-(3-(2-morpholinoethoxy)phenyl) propyl) 1-(3,3-dimethyl-2-oxopentanoyl)pyrrolidine-2-carboxylate
(S)-((R) -3-(3,4-dimethoxyphenyl) -1-(3-(2-morpholinoethoxy) phenyl) propyl) 1-(3,3-dimethyl-2-oxopentanoyl)piperidine-2-carboxylate
2-(3-((R) -3-(3,4-dimethoxyphenyl) -1-((S)-1-(3,3-dimethyl-2-oxopentanoyl)-1,2,3,6-tetrahydropyridine-2-carbonyloxy) propyl)phenoxy)acetic acid
2-(3-((R)-3-(3,4-dimethoxyphenyl)-1-((S)-1-(3,3-dimethyl-2-oxopentanoyl)pyrro-lidine-2-carbonyloxy)propyl)phenoxy)acetic acid
2-(3-((R)-3-(3,4-dimethoxyphenyl)-1-((S)-1-(3,3-dimethyl-2-oxopentanoyl) piperidine-2-carbonyloxy)propyl)phenoxy)acetic acid
3,3-dimethyl-2-oxopentanamide (1R)-1-(3-aminophenyl) -3-(3,4-dimethoxyphenyl)propyl cyclohexanecarboxylate
2-{3-[(1R)-3-(3,4-dimethoxyphenyl)-1-({1-[2-oxo-2-(3,4,5-trimethoxyphenyl)acetyl]piperidin-2-yl}carbonyloxy)propyl]phenoxy}acetic acid
2-(3-((R)-3-(3,4-dimethoxyphenyl)-1-((S)-1-(2-((1S,2R)-1-hydroxy-2-methyl cyclohexyl)-2-oxoacetyl)piperidine-2-carbonyloxy)propyl)phenoxy)acetic acid
3,4-dimethoxyphenethyl 1-(2-((1S,2R)-1-hydroxy-2-methylcyclohexyl)-2-oxo-acetyl)-piperidine-2-carboxylate
2-(3,4-dimethoxyphenoxy)ethyl 1-(2-((1S,2R)-1-hydroxy-2-methylcyclohexyl)-2-oxoacetyl)piperidine-2-carboxylate
2-(3,4-dimethoxyphenoxy)ethyl 1-(2-((1 S,2R)-2-ethyl-1-hydroxycyclohexyl)-2-oxoacetyl)piperidine-2-carboxylate
(S)-((R)-3-(3,4-dimethoxyphenyl)-1-(3-(2-morpholinoethoxy)phenyl)propyl) 1-(2-((1S, 2R)-1-hydroxy-2-methylcyclohexyl)-2-oxoacetyl)piperidine-2-carboxylate
2-(3-((R)-3-(3,4-dimethoxyphenyl)-1-((S)-1-(2-((1S,2R)-2-ethyl-1-hydroxy-cyclohexyl)-2-oxoacetyl)piperidine-2-carbonyloxy)propyl)phenoxy)acetic acid
(S)-((R)-3-(3,4-dimethoxyphenyl)-1-(3-(2-morpholinoethoxy)phenyl)propyl)1-(2-((1S, 2R) -2-ethyl-1-hydroxycyclohexyl)-2-oxoacetyl)piperidine-2-carboxylate
2-(3-((R)-3-(3,4-dimethoxyphenyl)-1-((S)-1-(2-((1S,2S)-1-hydroxy-2-(hydroxyl methyl) cyclohexyl)-2-oxoacetyl)piperidine-2-carbonyloxy)propyl)phenoxy)acetic acid
2-(3-((R)-3-(3,4-dimethoxyphenyl)-1-((S)-1-(2-((1R,2R)-1-hydroxy-2-methyl cyclohexyl)-2-oxoacetyl)piperidine-2-carbonyloxy)propyl)phenoxy)acetic acid
2-(3-((R)-3-(3,4-dimethoxyphenyl)-1-((S)-1-(2-((1R,2R)-2-ethyl-1-hydroxy cyclo hexyl)-2-oxoacetyl)piperidine-2-carbonyloxy)propyl)phenoxy)acetic acid

Especially preferred are compounds of the subformula (II) and (III) of formula (I): 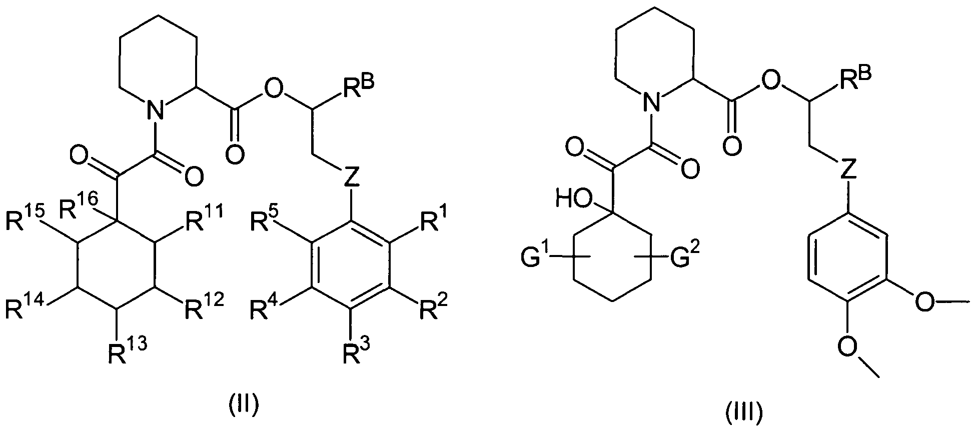 wherein Z and the substituents R¹ - R¹⁶ have the meanings as defined herein and
**R^{B}** represents -H or 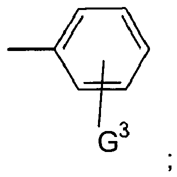 **G¹-G³** represent independently of each other **-G^{C}**, **-G^{D}**, -H, -OH, -OCH₃, -OC₂H₅, -OC₃H₇, -O-cyclo-C₃H₅, -OCH(CH₃)₂, -OC(CH₃)₃, -OC₄H₉, -OCH₂-COOH, -O-(CH₂)ₙ-COO**G^{C}** (with n = 1-4), -OPh, -OCH₂-Ph, -OCPh₃, -CH₂-OH, -SH, -SCH₃, -SC₂H₅, -SC₃H₇, -S-cyclo-C₃H₅, -SCH(CH₃)₂, -SC(CH₃)₃, -NO₂, -F, -Cl, -Br, -I, -P(O)(OH)₂, -P(O)(OCH₃)₂, -P(O)(OC₂H₅)₂, -P(O)(OCH(CH₃)₂)₂, -C(OH)[P(O)(OH)₂]₂, -Si(CH₃)₂(C(CH₃)₃), -Si(C₂H₅)₃, -Si(CH₃)₃, -N₃, -CN, -OCN, -NCO, -SCN, -NCS, -CHO, -COCH₃, -COC₂H₅, -COC₃H₇, -CO-cyclo-C₃H₅, -COCH(CH₃)₂, -COC(CH₃)₃, -COOH, -COCN, -COOCH₃, -COOC₂H₅, -COOC₃H₇, -COO-cyclo-C₃H₅, -COOCH(CH₃)₂, -COOC(CH₃)₃, -OOC-CH₃, -OOC-C₂H₅, -OOC-C₃H₇, -OOC-cyclo-C₃H₅, -OOC-CH(CH₃)₂, -OOC-C(CH₃)₃, -CONH₂, -CONHCH₃, -CONHC₂H₅, -CONHC₃H₇, -CONH-cyclo-C₃H₅, -CONH[CH(CH₃)₂], -CONH[C(CH₃)₃], -CON(CH₃)₂, -CON(C₂H₅)₂, -CON(C₃H₇)₂, -CON(cyclo-C₃H₅)₂, -CON[CH(CH₃)₂]₂, -CON[C(CH₃)₃]₂, -NHCOCH₃, -NHCOC₂H₅, -NHCOC₃H₇, -NHCO-cyclo-C₃H₅, -NHCO-CH(CH₃)₂, -NHCO-C(CH₃)₃, -NHCO-OCH₃, -NHCO-OC₂H₅, -NHCO-OC₃H₇, -NHCO-O-cyclo-C₃H₅, -NHCO-OCH(CH₃)₂, -NHCO-OC(CH₃)₃, -NH₂, -NHCH₃, -NHC₂H₅, -NHC₃H₇, -NH-cyclo-C₃H₅, -NHCH(CH₃)₂, -NHC(CH₃)₃, -N(CH₃)₂, -N(C₂H₅)₂, -N(C₃H₇)₂, -N(cyclo-C₃H₅)₂, -N[CH(CH₃)₂]₂, -N[C(CH₃)₃]₂, -SOCH₃, -SOC₂H₅, -SOC₃H₇, -SO-cyclo-C₃H₅, -SOCH(CH₃)₂, -SOC(CH₃)₃, -SO₂CH₃, -SO₂C₂H₅, -SO₂C₃H₇, -SO₂-cyclo-C₃H₅, -SO₂CH(CH₃)₂, -SO₂C(CH₃)₃, -SO₃H, -SO₃CH₃, -SO₃C₂H₅, -SO₃C₃H₇, -SO₃-cyclo-C₃H₅, -SO₃CH(CH₃)₂, -SO₃C(CH₃)₃, -SO₂NH₂, -SO₂NHCH₃, -SO₂NHC₂H₅, -SO₂NHC₃H₇, -SO₂NH-cyclo-C₃H₅, -SO₂NHCH(CH₃)₂, -SO₂NHC(CH₃)₃, -SO₂N(CH₃)₂, -SO₂N(C₂H₅)₂, -SO₂N(C₃H₇)₂, -SO₂N(cyclo-C₃H₅)₂, -SO₂N[CH(CH₃)₂]₂, -SO₂N[C(CH₃)₃]₂, -O-S(=O)CH₃, -O-S(=O)C₂H₅, -O-S(=O)C₃H₇, -O-S(=O)-cyclo-C₃H₅, -O-S(=O)CH(CH₃)₂, -O-S(=O)C(CH₃)₃, , -S(=O)(=NH)CH₃, -S(=O)(=NH)C₂H₅, -S(=O)(=NH)C₃H₇, -S(=O)(=NH)-cyclo-C₃H₅, -S(=O)(=NH)CH(CH₃)₂, -S(=O)(=NH)C(CH₃)₃, -NH-SO₂-CH₃, -NH-SO₂-C₂H₅, -NH-SO₂-C₃H₇, -NH-SO₂-cyclo-C₃H₅, -NH-SO₂-CH(CH₃)₂, -NH-SO₂-C(CH₃)₃, -O-SO₂-CH₃, -O-SO₂-C₂H₅, -O-SO₂-C₃H₇, -O-SO₂-cyclo-C₃H₅, -O-SO₂-CH(CH₃)₂, -O-SO₂-C(CH₃)₃, -OCF₃, -CH₂-OCF₃, -C₂H₄-OCF₃, -C₃H₆-OCF₃, -OC₂F₅, -CH₂-OC₂F₅, -C₂H₄-OC₂F₅, -C₃H₆-OC₂F₅, -O-COOCH₃, -O-COOC₂H₅, -O-COOC₃H₇, -O-COO-cyclo-C₃H₅, -O-COOCH(CH₃)₂, -O-COOC(CH₃)₃, -NH-CO-NH₂, -NH-CO-NHCH₃, -NH-CO-NHC₂H₅, -NH-CS-N(C₃H₇)₂, -NH-CO-NHC₃H₇, -NH-CO-N(C₃H₇)₂, -NH-CO-NH[CH(CH₃)₂], -NH-CO-NH[C(CH₃)₃], -NH-CO-N(CH₃)₂, -NH-CO-N(C₂H₅)₂, -NH-CO-NH-cyclo-C₃H₅, -NH-CO-N(cyclo-C₃H₅)₂, -NH-CO-N[CH(CH₃)₂]₂, -NH-CS-N(C₂H₅)₂, -NH-CO-N[C(CH₃)₃]₂, -NH-CS-NH₂, -NH-CS-NHCH₃, -NH-CS-N(CH₃)₂, -NH-CS-NHC₂H₅, -NH-CS-NHC₃H₇, -NH-CS-NH-cyclo-C₃H₅, -NH-CS-NH[CH(CH₃)₂], -NH-CS-NH[C(CH₃)₃], -NH-CS-N(cyclo-C₃H₅)₂, -NH-CS-N[CH(CH₃)₂]₂, -NH-CS-N[C(CH₃)₃]₂, -NH-C(=NH)-NH₂, -NH-C(=NH)-NHCH₃, -NH-C(=NH)-NHC₂H₅, -NH-C(=NH)-NHC₃H₇, -O-CO-NH-cyclo-C₃H₅, -NH-C(=NH)-NH-cyclo-C₃H₅, -NH-C(=NH)-NH[CH(CH₃)₂], -O-CO-NH[CH(CH₃)₂], -NH-C(=NH)-NH[C(CH₃)₃], -NH-C(=NH)-N(CH₃)₂, -NH-C(=NH)-N(C₂H₅)₂, -NH-C(=NH)-N(C₃H₇)₂, -NH-C(=NH)-N(cyclo-C₃H₅)₂, -O-CO-NHC₃H₇, -NH-C(=NH)-N[CH(CH₃)₂]₂, -NH-C(=NH)-N[C(CH₃)₃]₂, -O-CO-NH₂, -O-CO-NHCH₃, -O-CO-NHC₂H₅, -O-CO-NH[C(CH₃)₃], -O-CO-N(CH₃)₂, -O-CO-N(C₂H₅)₂, -O-CO-N(C₃H₇)₂, -O-CO-N(cyclo-C₃H₅)₂, -O-CO-N[CH(CH₃)₂]₂, -O-CO-N[C(CH₃)₃]₂, -O-CO-OCH₃, -O-CO-OC₂H₅, -O-CO-OC₃H₇, -O-CO-O-cyclo-C₃H₅, -O-CO-OCH(CH₃)₂, -O-CO-OC(CH₃)₃, 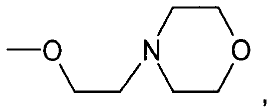 G^{C} and G^{D} represent independently of each other -CH₂-OCH₃, -C₂H₄-OCH₃, -C₃H₆-OCH₃, -CH₂-OC₂H₅, -C₂H₄-OC₂H₅, -C₃H₆-OC₂H₅, -CH₂-OC₃H₇, -C₂H₄-OC₃H₇, -C₃H₆-OC₃H₇, -CH₂-O-cyclo-C₃H₅, -C₂H₄-O-cyclo-C₃H₅, -C₃H₆-O-cyclo-C₃H₅, -CH₂-OCH(CH₃)₂, -C₂H₄-OCH(CH₃)₂, -C₃H₆-OCH(CH₃)₂, -CH₂-OC(CH₃)₃, -C₂H₄-OC(CH₃)₃, -C₃H₆-OC(CH₃)₃, -CH₂-OC₄H₉, -C₂H₄-OC₄H₉, -C₃H₆-OC₄H₉, -CH₂-OPh, -C₂H₄-OPh, -C₃H₆-OPh, -CH₂-OCH₂-Ph, -C₂H₄-OCH₂-Ph, -C₃H₆-OCH₂-Ph, -CH₂F, -CHF₂, -CF₃, -CH₂Cl, -CH₂Br, -CH₂l, -CH₂-CH₂F, -CH₂-CHF₂, -CH₂-CF₃, -CH₂-CH₂Cl, -CH₂-CH₂Br, -CH₂-CH₂l, -cyclo-C₈H₁₅, -Ph, -CH₂-Ph, -CH₂-CH₂-Ph, -CH=CH-Ph, -CPh₃, -CH₃, -C₂H₅, -C₃H₇, -CH(CH₃)₂, -C₄H₉, -CH₂-CH(CH₃)₂, -CH(CH₃)-C₂H₅, -C(CH₃)₃, -C₅H₁₁, -CH(CH₃)-C₃H₇, -CH₂-CH(CH₃)-C₂H₅, -CH(CH₃)-CH(CH₃)₂, -C(CH₃)₂-C₂H₅, -CH₂-C(CH₃)₃, -CH(C₂H₅)₂, -C₂H₄-CH(CH₃)₂, -C₆H₁₃, -C₇H₁₅, -C₈H₁₇, -C₃H₆-CH(CH₃)₂, -C₂H₄-CH(CH₃)-C₂H₅, -CH(CH₃)-C₄H₉, -CH₂-CH(CH₃)-C₃H₇, -CH(CH₃)-CH₂-CH(CH₃)₂, -CH(CH₃)-CH(CH₃)-C₂H₅, -CH₂-CH(CH₃)-CH(CH₃)₂, -CH₂-C(CH₃)₂-C₂H₅, -C(CH₃)₂-C₃H₇, -C(CH₃)₂-CH(CH₃)₂, -C₂H₄-C(CH₃)₃, -CH(CH₃)-C(CH₃)₃, -CH=CH₂, -CH₂-CH=CH₂, -C(CH₃)=CH₂, -CH=CH-CH₃, -C₂H₄-CH=CH₂, -CH₂-CH=CH-CH₃, -CH=CH-C₂H₅, -CH₂-C(CH₃)=CH₂, -CH(CH₃)-CH=CH, -CH=C(CH₃)₂, -C(CH₃)=CH-CH₃, -CH=CH-CH=CH₂, - C₃H₆-CH=CH₂, -C₂H₄-CH=CH-CH₃, -CH₂-CH=CH-C₂H₅, -CH=CH-C₃H₇, -CH₂-CH=CH-CH=CH₂, -CH=CH-CH=CH-CH₃, -CH=CH-CH₂-CH=CH₂, -C(CH₃)=CH-CH=CH₂, -CH=C(CH₃)-CH=CH₂, -CH=CH-C(CH₃)=CH₂, -C₂H₄-C(CH₃)=CH₂, -CH₂-CH(CH₃)-CH=CH₂, -CH(CH₃)-CH₂-CH=CH₂, -CH₂-CH=C(CH₃)₂, -CH₂-C(CH₃)=CH-CH₃, -CH(CH₃)-CH=CH-CH₃, -CH=CH-CH(CH₃)₂, -CH=C(CH₃)-C₂H₅, -C(CH₃)=CH-C₂H₅, -C(CH₃)=C(CH₃)₂, -C(CH₃)₂-CH=CH₂, -CH(CH₃)-C(CH₃)=CH₂, -C(CH₃)=CH-CH=CH₂, -CH=C(CH₃)-CH=CH₂, -CH=CH-C(CH₃)=CH₂, -C₄H₈-CH=CH₂, -C₃H₆-CH=CH-CH₃, -C₂H₄-CH=CH-C₂H₅, -CH₂-CH=CH-C₃H₇, -CH=CH-C₄H₉, -C₃H₆-C(CH₃)=CH₂, -C₂H₄-CH(CH₃)-CH=CH₂, -CH₂-CH(CH₃)-CH₂-CH=CH₂, -C₂H₄-CH=C(CH₃)₂, -CH(CH₃)-C₂H₄-CH=CH₂, -C₂H₄-C(CH₃)=CH-CH₃, -CH₂-CH(CH₃)-CH=CH-CH₃, -CH(CH₃)-CH₂-CH=CH-CH₃, -CH₂-CH=CH-CH(CH₃)₂, -CH₂-CH=C(CH₃)-C₂H₅, -CH₂-C(CH₃)=CH-C₂H₅, -CH(CH₃)-CH=CH-C₂H₅, -CH=CH-CH₂-CH(CH₃)₂, -CH=CH-CH(CH₃)-C₂H₅, -CH=C(CH₃)-C₃H₇, -C(CH₃)=CH-C₃H₇, -CH₂-CH(CH₃)-C(CH₃)=CH₂, -C[C(CH₃)₃]=CH₂, -CH(CH₃)-CH₂-C(CH₃)=CH₂, -CH(CH₃)-CH(CH₃)-CH=CH₂, -CH=CH-C₂H₄-CH=CH₂, -CH₂-C(CH₃)₂-CH=CH₂, -C(CH₃)₂-CH₂-CH=CH₂, -CH₂-C(CH₃)=C(CH₃)₂, -CH(CH₃)-CH=C(CH₃)₂, -C(CH₃)₂-CH=CH-CH₃, -CH=CH-CH₂-CH=CH-CH₃, -CH(CH₃)-C(CH₃)=CH-CH₃, -CH=C(CH₃)-CH(CH₃)₂, -C(CH₃)=CH-CH(CH₃)₂, -C(CH₃)=C(CH₃)-C₂H₅, -CH=CH-C(CH₃)₃, -C(CH₃)₂-C(CH₃)=CH₂, -CH(C₂H₅)-C(CH₃)=CH₂, -C(CH₃)(C₂H₅)-CH=CH₂, -CH(CH₃)-C(C₂H₅)=CH₂, -CH₂-C(C₃H₇)=CH₂, -CH₂-C(C₂H₅)=CH-CH₃, -CH(C₂H₅)-CH=CH-CH₃, -C(C₄H₉)₌CH₂, -C(C₃H₇)=CH-CH₃, -C(C₂H₅)=CH-C₂H₅, -C(C₂H₅)=C(CH₃)₂, -C[CH(CH₃)(C₂H₅)]=CH₂, -C[CH₂-CH(CH₃)₂]=CH₂, -C₂H₄-CH=CH-CH=CH₂, -CH₂-CH=CH-CH₂-CH=CH₂, -C₃H₆-C≡C-CH₃, -CH₂-CH=CH-CH=CH-CH₃, -CH=CH-CH=CH-C₂H₅, -CH₂-CH=CH-C(CH₃)=CH₂, -CH₂-CH=C(CH₃)-CH=CH₂, -CH₂-C(CH₃)=CH-CH=CH₂, -CH(CH₃)-CH₂-C≡CH, -CH(CH₃)-CH=CH-CH=CH₂, -CH=CH-CH₂-C(CH₃)=CH₂, -CH(CH₃)-C≡C-CH₃, -CH=CH-CH(CH₃)-CH=CH₂, -CH=C(CH₃)-CH₂-CH=CH₂, -C₂H₄-CH(CH₃)-C≡CH, -C(CH₃)=CH-CH₂-CH=CH₂, -CH=CH-CH=C(CH₃)₂, -CH₂-CH(CH₃)-CH₂-C≡CH, -CH=CH-C(CH₃)=CH-CH₃, -CH=C(CH₃)-CH=CH-CH₃, -CH₂-CH(CH₃)-C≡CH, -C(CH₃)=CH-CH=CH-CH₃, -CH=C(CH₃)-C(CH₃)=CH₂, -C(CH₃)=CH-C(CH₃)=CH₂, -C(CH₃)=C(CH₃)-CH=CH₂, -CH=CH-CH=CH-CH=CH₂, -C≡CH, -C≡C-CH₃, -CH₂-C≡CH, -C₂H₄-C≡CH, -CH₂-C≡C-CH₃, -C≡C-C₂H₅, -C₃H₆-C≡CH, -C₂H₄-C≡C-CH₃, -CH₂-C≡C-C₂H₅, -C≡C-C₃H₇, -CH(CH₃)-C≡CH, -C₄H₈-C≡CH, -C₂H₄-C≡C-C₂H₅, -CH₂-C≡C-C₃H₇, -C≡C-C₄H₉, -C≡C-C(CH₃)₃, -CH(CH₃)-C₂H₄-C≡CH, -CH₂-CH(CH₃)-C≡C-CH₃, -CH(CH₃)-CH₂-C≡C-CH₃, -CH(CH₃)-C≡C-C₂H₅, -CH₂-C≡C-CH(CH₃)₂, -C≡C-CH(CH₃)-C₂H₅, -C≡C-CH₂-CH(CH₃)₂, -CH(C₂H₅)-C≡C-CH₃, -C(CH₃)₂-C≡C-CH₃, -CH(C₂H₅)-CH₂-C≡CH, -CH₂-CH(C₂H₅)-C≡CH, -C(CH₃)₂-CH₂-C≡CH, -CH₂-C(CH₃)₂-C≡CH, -CH(CH₃)-CH(CH₃)-C≡CH, -CH(C₃H₇)-C≡CH, -C(CH₃)(C₂H₅)-C≡CH, -CH₂-CH(C≡CH)₂, -C≡C-C≡CH, -CH₂-C≡C-C≡CH, -C≡C-C≡C-CH₃, -CH(C≡CH)₂, -C₂H₄-C≡C-C≡CH, -CH₂-C≡C-CH₂-C≡CH, -C≡C-C₂H₄-C≡CH, -CH₂-C≡C-C≡C-CH₃, -C≡C-CH₂-C≡C-CH₃, -C≡C-C≡C-C₂H₅, -C(C≡CH)₂-CH₃, -C≡C-CH(CH₃)-C≡CH, -CH(CH₃)-C≡C-C≡CH, -CH(C≡CH)-CH₂-C≡CH, -CH(C≡CH)-C≡C-CH₃; and enantiomers, stereoisomeric forms, mixtures of enantiomers, anomers, deoxy-forms, diastereomers, mixtures of diastereomers, prodrugs, tautomers, hydrates, solvates and racemates of the above mentioned compounds and pharmaceutically acceptable salts thereof.

The compounds falling under general formula (III) are novel so that the present invention also relates to compounds of the general formula (III)

Further preferred are compounds of the subformula (IV), (V) and (VI) of formula (I): 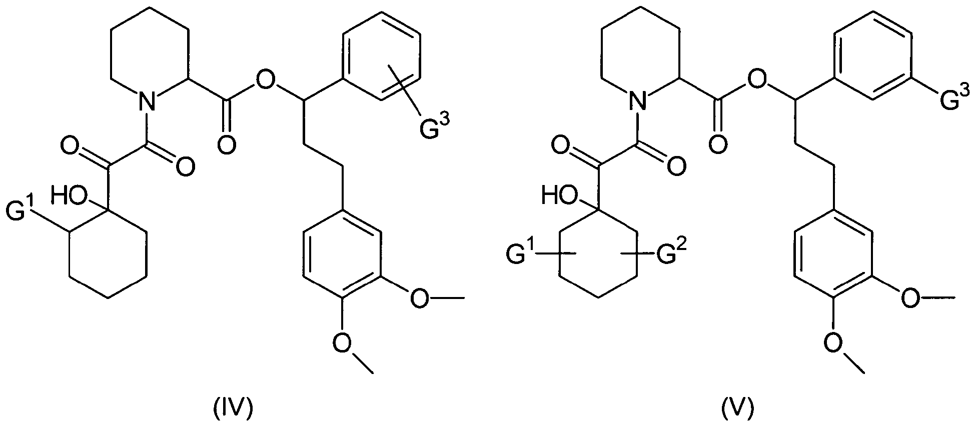 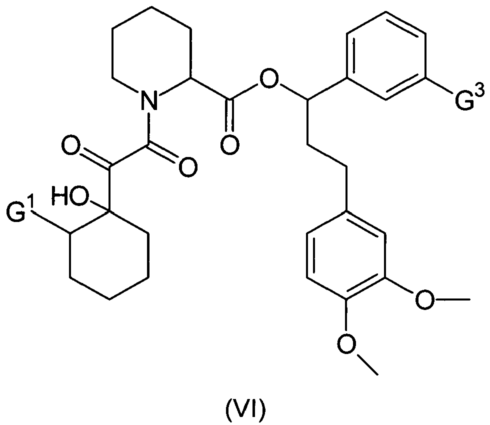 wherein **G1-G³** have the meanings as defined herein.

Preferred substituents for **G¹-G³** are: -**G^{C}, -G^{D}**, -H, -OH, -OCH₃, -OC₂H₅, -OC₃H₇, -O-cyclo-C₃H₅, -OCH(CH₃)₂, -OC(CH₃)₃, -OC₄H₉, -OCH₂-COOH, -O-(CH₂)ₙ-COO**G^{C}** (with n = 1-4), -CH₃, -C₂H₅, -C₃H₇, -CH(CH₃)₂, -C₄H₉, -CH₂-CH(CH₃)₂, -CH(CH₃)-C₂H₅, -C(CH₃)₃, -C₅H₁₁, -CH(CH₃)-C₃H₇, -CH₂-CH(CH₃)-C₂H₅, -CH(CH₃)-CH(CH₃)₂, -C(CH₃)₂-C₂H₅, -CH₂-C(CH₃)₃, -CH(C₂H₅)₂, -C₂H₄-CH(CH₃)₂, -C₆H₁₃, -C₇H₁₅, -C₈H₁₇, -C₃H₆-CH(CH₃)₂, -C₂H₄-CH(CH₃)-C₂H₅, -CH(CH₃)-C₄H₉, -CH₂-CH(CH₃)-C₃H₇, -CH(CH₃)-CH₂-CH(CH₃)₂, -CH(CH₃)-CH(CH₃)-C₂H₅, -CH₂-CH(CH₃)-CH(CH₃)₂, -CH₂-OH, -CH₂-C(CH₃)₂-C₂H₅, -C(CH₃)₂-C₃H₇, -C(CH₃)₂-CH(CH₃)₂, -C₂H₄-C(CH₃)₃, -CH(CH₃)-C(CH₃)₃,

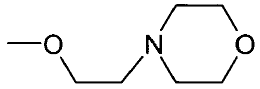

Preferred substituents for **G^{C}** and **G^{D}** are: -CH₂-OCH₃, -C₂H₄-OCH₃, -C₃H₆-OCH₃, -CH₂-OC₂H₅, -C₂H₄-OC₂H₅, -C₃H₆-OC₂H₅, -CH₂-OC₃H₇, -C₂H₄-OC₃H₇, -C₃H₆-OC₃H₇, -CH₃, -C₂H₅, -C₃H₇, -CH(CH₃)₂, -C₄H₉, -CH₂-CH(CH₃)₂, -CH(CH₃)-C₂H₅, -C(CH₃)₃, -C₅H₁₁, -CH(CH₃)-C₃H₇, -CH₂-CH(CH₃)-C₂H₅, -CH(CH₃)-CH(CH₃)₂, -C(CH₃)₂-C₂H₅, -CH₂-C(CH₃)₃, -CH(C₂H₅)₂, -C₂H₄-CH(CH₃)₂, -C₆H₁₃, -C₇H₁₅, -C₈H₁₇, -C₃H₆-CH(CH₃)₂, -C₂H₄-CH(CH₃)-C₂H₅, -CH(CH₃)-C₄H₉, -CH₂-CH(CH₃)-C₃H₇, -CH(CH₃)-CH₂-CH(CH₃)₂, -CH(CH₃)-CH(CH₃)-C₂H₅, -CH₂-CH(CH₃)-CH(CH₃)₂, -CH₂-C(CH₃)₂-C₂H₅, -C(CH₃)₂-C₃H₇, -C(CH₃)₂-CH(CH₃)₂, -C₂H₄-C(CH₃)₃, -CH(CH₃)-C(CH₃)₃.

In yet another preferred embodiment of the present invention, the compound according to the general formula (III) is selected from the group comprising or consisting of:
2-(3-((R)-3-(3,4-dimethoxyphenyl)-1-((S)-1-(2-((1S,2R)-1-hydroxy-2-methyl cyclo hexyl)-2-oxoacetyl)piperidine-2-carbonyloxy)propyl)phenoxy)acetic acid
3,4-dimethoxyphenethyl 1-(2-((1S,2R)-1-hydroxy-2-methylcyclohexyl)-2-oxoacetyl)-piperidine-2-carboxylate
2-(3,4-dimethoxyphenoxy)ethyl 1-(2-((1S,2R)-1-hydroxy-2-methylcyclohexyl)-2-oxo-acetyl)piperidine-2-carboxylate
2-(3,4-dimethoxyphenoxy)ethyl 1-(2-((1S,2R)-2-ethyl-1-hydroxycyclohexyl)-2-oxo-acetyl)piperidine-2-carboxylate
(S)-((R)-3-(3,4-dimethoxyphenyl)-1-(3-(2-morpholinoethoxy)phenyl)propyl) 1-(2-((1S, 2R)-1-hydroxy-2-methylcyclohexyl)-2-oxoacetyl)piperidine-2-carboxylate
2-(3-((R)-3-(3,4-dimethoxyphenyl)-1-((S)-1-(2-((1S,2R)-2-ethyl-1-hydroxy-cyclohexyl)-2-oxoacetyl)piperidine-2-carbonyloxy)propyl)phenoxy)acetic acid
(S)-((R)-3-(3,4-dimethoxyphenyl)-1-(3-(2-morpholinoethoxy)phenyl)propyl)1-(2-((1S, 2R)-2-ethyl-1-hydroxycyclohexyl)-2-oxoacetyl)piperidine-2-carboxylate
2-(3-((R)-3-(3,4-dimethoxyphenyl)-1-((S)-1-(2-((1S,2S)-1-hydroxy-2-(hydroxyl methyl) cyclohexyl)-2-oxoacetyl)piperidine-2-carbonyloxy)propyl)phenoxy)acetic acid
2-(3-((R)-3-(3,4-dimethoxyphenyl)-1-((S)-1-(2-((1R,2R)-1-hydroxy-2-methyl cyclohexyl)-2-oxoacetyl)piperidine-2-carbonyloxy)propyl)phenoxy)acetic acid
2-(3-((R)-3-(3,4-dimethoxyphenyl)-1-((S)-1-(2-((1R,2R)-2-ethyl-1-hydroxy cyclohexyl)-2-oxoacetyl)piperidine-2-carbonyloxy)propyl)phenoxy)acetic acid

The present invention also comprises pharmaceutically acceptable salts of the compounds according to the general formula (I), (II) and (III), all stereoisomeric forms of the compounds according to the general formula (I), (II) and (III), as well as solvates, especially hydrates or prodrugs thereof.

In case the compounds of the present invention bear basic and/or acidic substituents, they may form salts with organic or inorganic acids or bases. Examples of suitable acids for such acid addition salt formation are hydrochloric acid, hydrobromic acid, sulfuric acid, phosphoric acid, acetic acid, citric acid, oxalic acid, malonic acid, salicylic acid, p-aminosalicylic acid, malic acid, fumaric acid, succinic acid, ascorbic acid, maleic acid, sulfonic acid, phosphonic acid, perchloric acid, nitric acid, formic acid, propionic acid, gluconic acid, lactic acid, tartaric acid, hydroxymaleic acid, pyruvic acid, phenylacetic acid, benzoic acid, p-aminobenzoic acid, p-hydroxybenzoic acid, methanesulfonic acid, ethanesulfonic acid, nitrous acid, hydroxyethanesulfonic acid, ethylenesulfonic acid, p-toluenesulfonic acid, naphthylsulfonic acid, sulfanilic acid, camphorsulfonic acid, china acid, mandelic acid, o-methylmandelic acid, hydrogen-benzenesulfonic acid, picric acid, adipic acid, d-o-tolyltartaric acid, tartronic acid, (o, m, p)-toluic acid, naphthylamine sulfonic acid, and other mineral or carboxylic acids well known to those skilled in the art. The salts are prepared by contacting the free base form with a sufficient amount of the desired acid to produce a salt in the conventional manner.

Examples for suitable inorganic or organic bases are, for example, NaOH, KOH, NH₄OH, tetraalkylammonium hydroxide, lysine or arginine and the like. Salts may be prepared in a conventional manner using methods well known in the art, for example by treatment of a solution of the compound of the general formula (I) with a solution of an acid, selected out of the group mentioned above.

Some of the compounds of the present invention may be crystallised or recrystallised from solvents such as aqueous and organic solvents. In such cases solvates may be formed. This invention includes within its scope stoichiometric solvates including hydrates as well as compounds containing variable amounts of water that may be produced by processes such as lyophilisation.

Certain compounds of the general formula (I) may exist in the form of optical isomers if substituents with at least one asymmetric center are present, e.g. diastereoisomers and mixtures of isomers in all ratios, e.g. racemic mixtures. The invention includes all such forms, in particular the pure isomeric forms. The different isomeric forms may be separated or resolved one from the other by conventional methods, or any given isomer may be obtained by conventional synthetic methods or by stereospecific or asymmetric syntheses. Where a compound according to the general formula (I) contains an alkene moiety, the alkene can be presented as a cis or trans isomer or a mixture thereof. When an isomeric form of a compound of the invention is provided substantially free of other isomers, it will preferably contain less than 5% w/w, more preferably less than 2% w/w and especially less than 1 % w/w of the other isomers.

Another aspect of the present invention relates to the use of the inventive pipecolate diketoamide derivatives as drugs, i.e. as pharmaceutically active agents applicable in medicine.

Surprisingly it was found that the above-mentioned pipecolate diketoamide derivatives as well as the pharmaceutical compositions comprising said pipecolate diketoamide derivatives are useful for the specific inhibition of FKBP51 and/or FKBP 52.

Therefore one aspect of the present invention is that the compounds according to the general formula (I) are suitable for use as inhibitor of FK506-binding proteins (FKBP). It is preferred if said compound is suitable for use as inhibitor of the FK506-binding protein 51 (FKBP51) or 52 (FKBP52).

FKBP inhibitors as used herein are defined as compounds that inhibit the peptidylprolyl isomerase activity of FKBPs (PPlase inhbitors, also referred to as rotamase inhibitors) and compounds that displace FK506 or FK506 analogs form the PPlase active site of FKBPs.

Thus, the pipecolate diketoamide compounds of the present invention and especially the pipecolate diketoamide compounds according to the general formulae (I) to (VI) can be used for treatment and prophylaxis, or for the preparation of a pharmaceutical formulation for treatment and prophylaxis of psychiatric and neurodegenerative diseases, disorders and conditions, for neuroprotection or neuroregeneration, for the treatment of neurological disorders, for the treatment of diseases relating to neurodegeneration, for the treatment of cancers and especially steroid-hormone dependent cancers, for the treatment of glucocorticoid hyposensitivity syndromes and peripheral glucocorticoid resistance for the treatment of infectious diseases, for the treatment of alopecia and promoting hair growth, for the treatment or prevention of multi-drug resistance, for stimulating neurite growth, for the use as wound healing agents for treating wounds resulting from injury or surgery; for the use in antiglaucomatous medications for treating abnormally elevated intraocular pressure; for the use in limiting or preventing hemorrhage or neovascularization for treating macular degeneration, and for treating oxidative damage to eye tissues, for treating a vision disorder, for improving vision, for treating memory impairment or enhancing memory performance.

Preferably the pipecolate diketoamide compounds according to one of the general formulae (I) to (VI) are useful for or can be used for treatment and prophylaxis, or for the preparation of a pharmaceutical formulation for treatment and prophylaxis of
- psychiatric disorder, neurological disorder, cancer, transplant rejection or metabolic disorders, glucocorticoid hyposensitivity syndrome, alopecia, abnormally elevated intraocular pressure, macular degeneration, oxidative damage to eye tissues, vision disorder, memory impairment, and
- increase and/or support neuroprotection, neuroregeneration, and
- promote hair growth, and
- stimulate neurite growth, wound healing, antiglaucomatous medications, and
- improve vision, and
- enhance memory performance, and
- treat or prevent multi-drug resistance, and
- limit or prevent hemorrhage or neovascularization, and
- for the treatment of diseases relating to neurodegeneration.

The pipecolate diketoamide compounds of the present invention are preferably suitable for treatment, or for the preparation of a pharmaceutical formulation for prophylaxis and treatment of psychiatric diseases. It is especially preferred if this psychiatric diseases is an affective disorder or an anxiety disorder.

The affective disorder according to the invention is selected from the group comprising or consisting of depression, bipolar disorder, mania, substance induced mood disorder and seasonal affective disorder (SAD). Among the psychiatric diseases and disorders, the most preferred is depression, the most commonly diagnosed psychiatric disorder.

The anxiety disorder according to the invention is selected from the group comprising or consisting of generalized anxiety disorder, panic disorder, panic disorder with agoraphobia, phobias, obsessive-compulsive disorder, post-traumatic stress disorder, separation anxiety and childhood anxiety disorders.

Among the hundreds of different neurodegenerative disorders, the attention has been given especially to a handful, including Alzheimer's Disease, Parkinson's Disease, and amyotrophic lateral sclerosis.

Among the glucocorticoid hyposensitivity syndromes, the attention has been given to the group of related diseases enclosing resistant asthma, AIDS, rheumatoid arthritis, hypertension and obesity.

Among the cancers, the attention has been given to malignant melanoma, steroid-hormone dependent cancers or prostate cancer.

Among the hundreds of infectious diseases, the attention has been given to malaria and the Legionnaires' disease.

Among the vision disorders, the attention has been given to visual impairments; orbital disorders; disorders of the lacrimal apparatus; disorders of the eyelids; disorders of the conjunctiva; disorders of the Cornea; cataract; disorders of the uveal tract; disorders of the retina; disorders of the optic nerve or visual pathways; free radical induced eye disorders and diseases; immunologically-mediated eye disorders and diseases; eye injuries; and symptoms and complications of eye disease, eye disorder, or eye injury.

Therefore, another aspect of the present invention is directed to pharmaceutical compositions comprising at least one compound of the present invention as active ingredient, together with at least one pharmaceutically acceptable carrier, excipient and/or diluents. The pharmaceutical compositions of the present invention can be prepared in a conventional solid or liquid carrier or diluent and a conventional pharmaceutically-made adjuvant at suitable dosage level in a known way. The preferred preparations are adapted for oral application. These administration forms include, for example, pills, tablets, film tablets, coated tablets, capsules, powders and deposits.

Furthermore, the present invention also includes pharmaceutical preparations for parenteral application, including dermal, intradermal, intragastral, intracutan, intravasal, intravenous, intramuscular, intraperitoneal, intranasal, intravaginal, intrabuccal, percutan, rectal, subcutaneous, sublingual, topical, or transdermal application, which preparations in addition to typical vehicles and/or diluents contain at least one compound according to the present invention and/or a pharmaceutical acceptable salt thereof as active ingredient.

The pharmaceutical compositions according to the present invention containing at least one compound according to the present invention, and/or a pharmaceutical acceptable salt thereof as active ingredient will typically be administered together with suitable carrier materials selected with respect to the intended form of administration, i.e. for oral administration in the form of tablets, capsules (either solid filled, semi-solid filled or liquid filled), powders for constitution, extrudates, deposits, gels, elixirs, dispersable granules, syrups, suspensions, and the like, and consistent with conventional pharmaceutical practices. For example, for oral administration in the form of tablets or capsules, the active drug component may be combined with any oral non-toxic pharmaceutically acceptable carrier, preferably with an inert carrier like lactose, starch, sucrose, cellulose, magnesium stearate, dicalcium phosphate, calcium sulfate, talc, mannitol, ethyl alcohol (liquid filled capsules) and the like. Moreover, suitable binders, lubricants, disintegrating agents and coloring agents may also be incorporated into the tablet or capsule. Powders and tablets may contain about 5 to about 95 weight % of the benzothiophene-1,1-dioxide derived compound and/or the respective pharmaceutically active salt as active ingredient.

Suitable binders include starch, gelatin, natural sugars, corn sweeteners, natural and synthetic gums such as acacia, sodium alginate, carboxymethylcellulose, polyethylene glycol and waxes. Among suitable lubricants there may be mentioned boric acid, sodium benzoate, sodium acetate, sodium chloride, and the like. Suitable disintegrants include starch, methylcellulose, guar gum, and the like. Sweetening and flavoring agents as well as preservatives may also be included, where appropriate. The disintegrants, diluents, lubricants, binders etc. are discussed in more detail below.

Moreover, the pharmaceutical compositions of the present invention may be formulated in sustained release form to provide the rate controlled release of any one or more of the components or active ingredients to optimise the therapeutic effect(s), e.g. antihistaminic activity and the like. Suitable dosage forms for sustained release include tablets having layers of varying disintegration rates or controlled release polymeric matrices impregnated with the active components and shaped in tablet form or capsules containing such impregnated or encapsulated porous polymeric matrices.

Liquid form preparations include solutions, suspensions, and emulsions. As an example, there may be mentioned water or water/propylene glycol solutions for parenteral injections or addition of sweeteners and opacifiers for oral solutions, suspensions, and emulsions. Liquid form preparations may also include solutions for intranasal administration. Aerosol preparations suitable for inhalation may include solutions and solids in powder form, which may be present in combination with a pharmaceutically acceptable carrier such as an inert, compressed gas, e.g. nitrogen. For preparing suppositories, a low melting fat or wax, such as a mixture of fatty acid glycerides like cocoa butter is melted first, and the active ingredient is then dispersed homogeneously therein e.g. by stirring. The molten, homogeneous mixture is then poured into conveniently sized moulds, allowed to cool, and thereby solidified.

Also included are solid form preparations which are intended to be converted, shortly before use, to liquid form preparations for either oral or parenteral administration. Such liquid forms include solutions, suspensions, and emulsions.

The compounds according to the present invention may also be delivered transdermally. The transdermal compositions may have the form of a cream, a lotion, an aerosol and/or an emulsion and may be included in a transdermal patch of the matrix or reservoir type as is known in the art for this purpose.

The term capsule as recited herein refers to a specific container or enclosure made e.g. of methyl cellulose, polyvinyl alcohols, or denatured gelatins or starch for holding or containing compositions comprising the active ingredient(s). Capsules with hard shells are typically made of blended of relatively high gel strength gelatins from bones or pork skin. The capsule itself may contain small amounts of dyes, opaquing agents, plasticisers and/or preservatives. Under tablet a compressed or moulded solid dosage form is understood which comprises the active ingredients with suitable diluents. The tablet may be prepared by compression of mixtures or granulations obtained by wet granulation, dry granulation, or by compaction well known to a person of ordinary skill in the art.

Oral gels refer to the active ingredients dispersed or solubilised in a hydrophilic semi-solid matrix. Powders for constitution refers to powder blends containing the active ingredients and suitable diluents which can be suspended e.g. in water or in juice.

Suitable diluents are substances that usually make up the major portion of the composition or dosage form. Suitable diluents include sugars such as lactose, sucrose, mannitol, and sorbitol, starches derived from wheat, corn rice, and potato, and celluloses such as microcrystalline cellulose. The amount of diluent in the composition can range from about 5 to about 95 % by weight of the total composition, preferably from about 25 to about 75 weight %, and more preferably from about 30 to about 60 weight %.

The term disintegrants refers to materials added to the composition to support break apart (disintegrate) and release the pharmaceutically active ingredients of a medicament. Suitable disintegrants include starches, "cold water soluble" modified starches such as sodium carboxymethyl starch, natural and synthetic gums such as locust bean, karaya, guar, tragacanth and agar, cellulose derivatives such as methylcellulose and sodium carboxymethylcellulose, microcrystalline celluloses, and cross-linked microcrystalline celluloses such as sodium croscaramellose, alginates such as alginic acid and sodium alginate, clays such as bentonites, and effervescent mixtures. The amount of disintegrant in the composition may range from about 2 to about 20 weight % of the composition, more preferably from about 5 to about 10 weight %.

Binders are substances which bind or "glue" together powder particles and make them cohesive by forming granules, thus serving as the "adhesive" in the formulation. Binders add cohesive strength already available in the diluent or bulking agent. Suitable binders include sugars such as sucrose, starches derived from wheat corn rice and potato, natural gums such as acacia, gelatin and tragacanth, derivatives of seaweed such as alginic acid, sodium alginate and ammonium calcium alginate, cellulose materials such as methylcellulose, sodium carboxymethylcellulose and hydroxypropylmethylcellulose, polyvinylpyrrolidone, and inorganic compounds such as magnesium aluminum silicate. The amount of binder in the composition may range from about 2 to about 20 weight % of the composition, preferably from about 3 to about 10 weight %, and more preferably from about 3 to about 6 weight %. Lubricants refer to a class of substances which are added to the dosage form to enable the tablet granules etc. after being compressed to release from the mould or die by reducing friction or wear. Suitable lubricants include metallic stearates such as magnesium stearate, calcium stearate, or potassium stearate, stearic acid, high melting point waxes, and other water soluble lubricants such as sodium chloride, sodium benzoate, sodium acetate, sodium oleate, polyethylene glycols and D,L-leucine. Lubricants are usually added at the very last step before compression, since they must be present at the surface of the granules. The amount of lubricant in the composition may range from about 0.2 to about 5 weight % of the composition, preferably from about 0.5 to about 2 weight %, and more preferably from about 0.3 to about 1.5 weight % of the composition.

Glidents are materials that prevent caking of the components of the pharmaceutical composition and improve the flow characteristics of granulate so that flow is smooth and uniform. Suitable glidents include silicon dioxide and talc. The amount of glident in the composition may range from about 0.1 to about 5 weight % of the final composition, preferably from about 0.5 to about 2 weight %.

Coloring agents are excipients that provide coloration to the composition or the dosage form. Such excipients can include food grade dyes adsorbed onto a suitable adsorbent such as clay or aluminum oxide. The amount of the coloring agent may vary from about 0.1 to about 5 weight % of the composition, preferably from about 0.1 to about 1 weight %.

Said pharmaceutical compositions may further comprise at least one active pipecolate diketoamide compound of the general formula (I).

The pharmaceutical compositions may further comprise at least one further active agent. It is preferred if this active agent is selected from the group consisting of anti-depressantand other psychotropic drugs. It is further preferred if the anti-depressant is selected from amitriptyline, amioxide clomipramine, doxepine, duloxetine, imipramine trimipramine, mirtazapine, reboxetine, citaloprame, fluoxetine, moclobemide and sertraline.

### Examples

### Generic route to pipecolate-diketoamides

Route to structures according to the general formula (II):

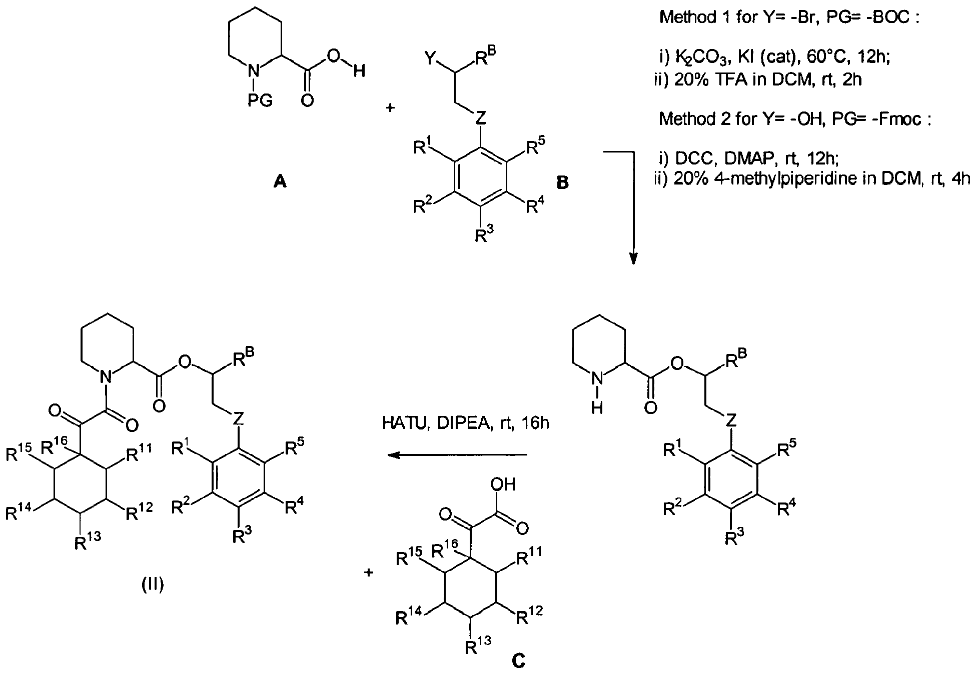

The protected piperidine-2-carboxylic acid **A** has been coupled with structure **B** by two methods according to the identity of moiety Y and the protective group PG.

Structure **B** has been synthesized from the corresponding phenols (Y= -Br, R^{B}= -H, Z= -O-):

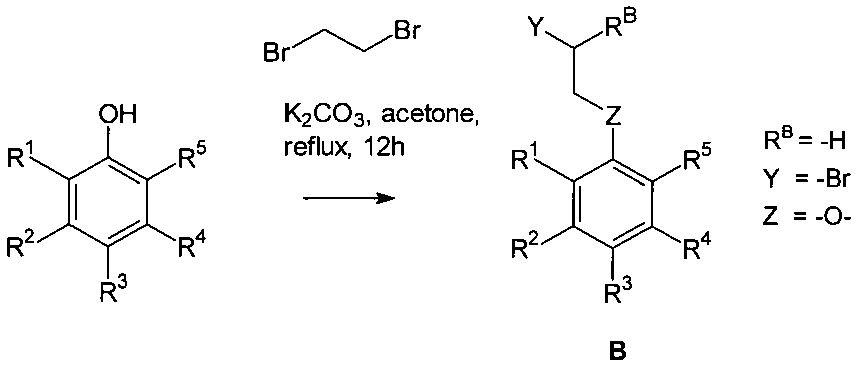

For Y= -OH structure **B** has been synthesized according to a literature procedure which is further described in Example 2.

### Coupling-Method 1:

To a solution of **B** (1.6 mmol), in acetone (10ml) was added (S)-1Boc-piperidine-2-carboxylic acid (1.30 mmol), K₂CO₃ (1.6 mmol), and KI (catalytic amount). The reaction mixture was stirred at 60°C for 12h. The mixture was filtered and the solid residue washed with ethyl acetate. The combined organic phases were washed with brine (30ml) and dried over MgSO₄. The solution was concentrated and then residue was purified by chromatography. The resulting pipecolic ester was treated with 20% TFA in DCM at rt. The mixture was allowed to stir for 2h. TFA and DCM was evaporated under reduced pressure.

### Coupling-Method 2:

A solution of B (5.0 mmol), (S)-1Fmoc-piperidine-2-carboxylic acid (5.5 mmol), and DMAP (0.5mmol) in 30mL DCM at room temperature was treated with DCC (5.5 mmol). The mixture was stirred for 12h after which time the organic solvent was dried and the solid was dissolved in diethyl ether and filtered through a plug of celite. The filtrate was concentrated and then flash chromatographed. The resulting pipecolic ester 3 was treated with 20% 4-methylpiperidine in DCM at rt. The mixture was allowed to stir for 4h. 4-methylpiperidine and DCM was evaporated under reduced pressure. Saturated NH₄Cl solution was added to the filtrate and the solution was stirred for 10 min. The organic phase was separated and the aqueous phase was extracted with ethyl acetate. The combined organic phases were washed with brine and dried over MgSO₄ and the residue purified by chromatogrpahy.

Structure **C** has been synthesized by the following procedure:

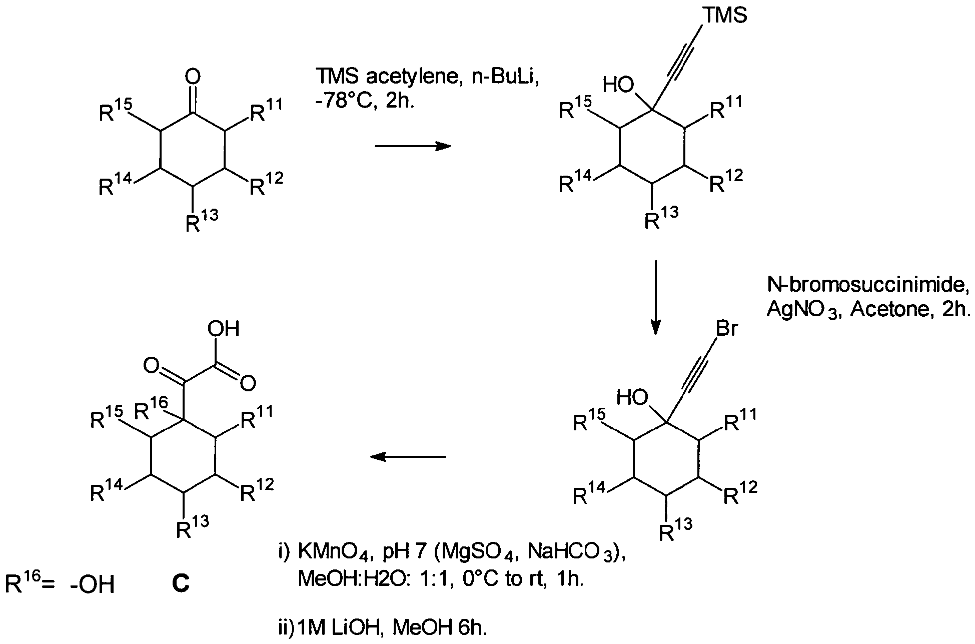

The THF solution of lithium reagent was generated by treatement of trimethylsilylacetylene (21 mmol) with n-BuLi (2M in hexane, 12ml) at -78°C. The solution was stirred for 0.5h at that temperature. To this mixture a THF (5ml) solution of 2-alkylcyclohexanone (18 mmol) was added and stirred for an additional 2h. Then the solution was quenched by addition of saturated aqueous NH₄Cl solution. The organic phase was separated and the aqueous phase was extracted with ethyl acetate. The combined organic phases were washed with brine (30ml) and dried over MgS04. The solution was concentrated and then flash chromatographed.

To an acetone solution of the resulting material (1.3 mmol), N-bromosuccinimide (1.5 mmol) and AgNO₃ (0.5 mmol) was added, and the resulting solution was stirred in darkness for 2h at rt. Acetone was evaporated under reduced pressure and the solids were removed by filtration through a celite pad. The combined organic phases were concentrated and subjected to purification by column chromatography. To a solution of the resulting material (1.1 mmol) in MeOH (5ml) a solution of NaHCO₃ (0.5 mmol) and MgSO₄ (2.0 mmol) in water (5ml) was added at 0°C. The mixture was stirred for 10 min vigorosuly before KMnO₄ (3.3 mmol) was added. The mixture was allowed to come to room temperature and stirred at this temperature for 1 h. The solids were removed by filtration through celite pad and washed with ethyl acetate. The organic phase was separated and the aqueous phase was extracted with ethyl acetate. The combined organic phases were washed with brine and dried over MgSO₄. The solution was concentrated and then flash chromatographed to afford the corresponding α-ketoesters .To the above synthesized α-ketoesters was added 1 M LiOH in MeOH: H₂O (1:1) and the reaction stirred for 6h at room temperature. The reaction was acidified to pH-2 by addition of 1 M HCl. The aqueous layer is extracted with ethyl acetate. The combined organic phases were washed with brine and dried over MgSO₄. The solution was concentrated under vacuo to furnish the free acid C.

The final step in the general procedure to synthesize the compounds of the general formula (II) comprises the following:

To a stirred solution of the free piperidine-amine in acetonitrile under argon was added sequentially N,N-Diisopropyl-ethylamine (DIPEA), HATU and the di-ketoacid **C.** The reaction mixture was stirred for 16h at room temperature. Saturated NH₄Cl solution was added to the reaction and the solution was stirred for 10 min. The organic phase was separated and the aqueous phase was extracted with ethyl acetate. The combined organic phases were washed with brine and dried over MgSO₄ and the residue purified by column chromatogrpahy to afford the final compounds of formula (II).

**Experimental conditions:** Chromatographic separations were performed either by manual flash chromatography or by automated flash chromatography using an Interchim- Puriflash 430 with a UV detector. Extracts were dried over MgSO₄, and the solvents were removed under reduced pressure. Merck F-254 (thickness 0.25mm) commercial plates were used for analytical TLC to follow the course of reaction. Silica gel 60 (Merck 70-230 mesh) was used for column chromatography. If not otherwise specified, ¹H NMR spectra, ¹³C NMR spectra, 2D HSQC, HMBC and COSY of all intermediates were obtained from the Department of Chemistry and Pharmacy, LMU, on a Bruker AC 300, a Bruker XL 400, or a Bruker AMX 600 at room temperature. Chemical shifts for ¹H, ¹³C are given in ppm (δ) relative to tetramethylsilane (TMS) as internal standard. Mass spectra (m/z) were recorded on a Thermo Finnigan LCQ DECA XP Plus mass spectrometer at the Max Planck Institute of Psychiatry, while the high resolution mass spectrometry was carried out at MPI for Biochemistry. (Microchemistry Core facility) on Varian Mat711 mass spectrometer. The purity of the compounds was verified by reversed phase HPLC (Jupiter 4 m Proteo 90 A, 250*4.6 mm, Phenomenex, Torrance, USA) by using gradient A (acetonitrile:water gradient of 0 - 100% in 45 min) unless otherwise specified. Solvents were brought from Roth, reagents were bought from Aldrich-Fluka unless otherwise noted.

**HPLC conditions for product analysis; Column:** Jupiter 4 m Proteo 90 A, 250 x 4.6 mm, Phenomenex, Torrance, USA, Wavelength: 224nm, 280nm Flow rate: 1ml/min, Buffer A: 0.1% TFA in 5% MeCN/Water, Buffer B: 0.1% TFA in 95% MeCN/water. Gradient A After 1 min elution with 100% buffer A,: a linear gradient of 0-100% buffer B was developed for 30 min. Finally the column was washed with 100% buffer B for 5min.

**LCMS conditions for product analysis; Column:** YMC Pack Pro C8, 100 x 4.6 mm, 3µM, Wavelength: 224nm, 280nm Flow rate: 1ml/min, Buffer A: 0.1 % HCOOH in 5% MeCN/Water, Buffer B: 0.1% HCOOH in 95% MeCN/water. Gradient A After 1 min elution with 100% buffer A,: a linear gradient of 0-100% buffer B was developed for 11 min. Finally the column was washed with 100% buffer B for 3min.

**Preparative HPLC conditions for product purification**, Compound was dissolved in 40% buffer B and the purification was carried out with a injection loop volume of 2ml, Column: Jupiter 10µ Proteo 90 A, 250 x 21.7 mm, 10micron Phenomenex, Torrance, USA, Wavelength: 224nm, Flow rate: 25ml/min, Buffer A: 0.1 % TFA in 5% MeOH/Water, Buffer B: 0.1 % TFA in 95% MeOH/water.

### Example 1

### Synthesis of building blocks C:

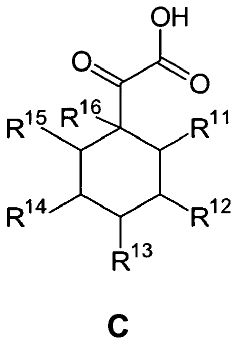

### Synthesis of precursor: (S)-2-((MOM)methyl)cyclohexanone

The (S)-2-(hydroxymethyl)cyclohexanone (**S-Alcohol**) was prepared as already described (A. Chen et al. / Tetrahedron 66 (2010) 1489-1495). To the above cyclohexanone (1.5 g, 11.7 mmol) in DCM was added N,N-Diisopropylethylamine(3.7g, 29.3 mmol). The mixture was allowed to stir at 0°C for 5 min before MOM-Cl (2g, 25.7 mmol) was added. The reaction was stirred for 3h. The mixture was then concentrated under reduced pressure and subjected to purification by column chromatography using Hexane: EtOAc 8:2 to afford (S)-2-((MOM)methyl)cyclohexanone (1.8g, 10.5 mmol, 90%) as a colorless liquid.
TLC (Hexane: EtOAc 8:2): Rf = 0.45
¹H NMR (300 MHz, CDCl₃) δ= 1.36-1.49 (m, 1H), 1.54-1.71 (m, 2H), 1.77-1.89 (m, 1 H), 1.95-2.06 (m, 1 H), 2.13-2.38 (m, 3H), 2.50-2.60 (m, 1 H), 3.29 (s, 3H), 3.41-3.47 (m, 1 H), 3.75-3.80 (m, 1H), 4.58 (d, 2H, J= 2.1 Hz). ¹³C NMR (75 MHz, CDCl₃) δ= 24.63, 27.62, 31.16, 41.99, 50.67, 55.08, 66.72, 96.62, 211.13
MS (ESI) m/z: 195.67 [M + Na]⁺, calculated 195.10 [M + Na]⁺.

### General procedure for the synthesis of 2-alkyl-1-((trimethylsilyl) ethynyl)cyclohexanoles:

The THF solution of lithium reagent was generated by treatement of trimethylsilylacetylene (3ml, 21.4 mmol) with n-BuLi (2M in hexane, 11.6ml) at -78°C. The solution was stirred for 0.5h at that temperature. To it was added a THF (5ml) solution of 2-alkylcyclohexanone (17.8 mmol) and stirred for an additional 2h. Then the solution was quenched by addition of saturated aqueous NH₄Cl solution. The organic phase was separated and the aqueous phase was extracted with ethyl acetate (3* 100ml). The combined organic phases were washed with brine (30ml) and dried over MgSO4. The solution was concentrated and then flash chromatographed using Hexane: EtOAc 9:1 to afford each of the two diasteromers.

(1 S,2R)-2-Methyl-1-((trimethylsilyl)ethynyl)cyclohexanol and (1 R,2S)-2-Methyl-1-((trimethylsilyl)ethynyl)cyclohexanol (1.2g, 33%) was obtained as a colorless liquid. TLC (Hexane: EtOAc 9:1): Rf = 0.36
¹H NMR (300 MHz, CDCl₃) δ= 0.17(s,9H), 1.06(d, 3H, J = 6.9 Hz), 1.23-1.33 (m, 1 H), 1.48-1.71 (m, 7p), 1.95-2.02 (m,1P). ¹³C NMR (75 MHz, CDCl₃) δ= 0.01, 16.00, 21.06, 25.02, 29.15, 39.15, 40.48, 69.77, 86.95, 110.61
HRMS 193.1390 [M - OH]⁺, calculated 193.1413 [M - OH]⁺.

(1S,2R)-2-Ethyl-1-((trimethylsilyl)ethynyl)cyclohexanol and (1 R,2S)-2-Ethyl-1 - ((trimethyls ilyl)ethynyl)cyclohexanol (1.65g, 46%) was obtained as a colorless liquid. TLC (Hexane: EtOAc 9:1): Rf = 0.40
¹H NMR (300 MHz, CDCl₃) δ= 0.17(s,9H), 0.86-0.96 (m, 6H), 1.12-2.42 (m, 22H). ¹³C NMR (75 MHz, CDCl₃) δ= 0.024, 11.68, 12.30, 21.23, 22.88, 24.80, 25.46, 28.00, 33.28, 39.47, 41.94, 47.47, 52.31, 70.32, 87.09, 110.85.
HRMS 208.2069 [M - OH] ⁺, calculated 208.1569 [M - OH] ⁺.
(1 S,2S)-2-((methoxymethoxy)methyl)-1-((trimethylsilyl)ethynyl)cyclohexanol. (2.1 g, 46%) was obtained as a colorless liquid.
TLC (Hexane: EtOAc 9:1): Rf = 0.45
¹H NMR (300 MHz, CDCl₃) δ= 0.17(s,9H), 1.31-2.04 (m, 9H), 3.40(s, 3H), 3.58 (dd, 1 H, J = 2.4, 9.3 Hz), 4.26 (dd, 1 H, J = 3.6, 9.6 Hz), 4.64 (dd, 2H, J = 6.6, 10.8 Hz). ¹³C NMR (75 MHz, CDCl₃) δ= 0.00, 20.70, 24.67, 25.57, 39.18, 45.07, 55.41, 69.77, 70.75, 87.06, 96.54, 110.30.
HRMS 254.2134 [M - OH] ⁺, calculated 254.2124 [M - OH] ⁺.

(1R,2R)-2-Methyl-1-((trimethylsilyl)ethynyl)cyclohexanol and (1S,2S)-2-Methyl-1-((trime thylsilyl)ethynyl)cyclohexanol (1.1 g, 31 %) was obtained as a colorless liquid . TLC (Hexane: EtOAc 9:1): Rf = 0.32 ¹H NMR (300 MHz, CDCl₃) δ= 0.18 (s,9H), 1.04 (d, 3H, J = 6.6 Hz), 1.17-1.32 (m, 2H), 1.41-1.73 (m, 6H), 1.96-2.02 (m, 1H).

¹³C NMR (75 MHz, CDCl₃) δ= 0.02, 16.03, 24.29, 25.50, 32.29, 40.63, 42.53, 73.42, 90.63, 106.80. HRMS 193.1278 [M - OH] ⁺, calculated 193.1413 [M - OH] ⁺.

(1R,2R)-2-Ethyl-1-((trimethylsilyl)ethynyl)cyclohexanol and (1S,2S)-2-Ethyl-1-((trimeth ylsilyl)ethynyl)cyclohexanol (1.3g, 37%) was obtained as a colorless liquid. TLC (Hexane: EtOAc 9:1): Rf = 0.37
¹H NMR (300 MHz, CDCl₃) δ= 0.19(s,9H), 0.94 (t, 3H, J= 7.2Hz), 1.04-1.30 (m, 4H), 1.43-1.74 (m, 4H), 1.80-2.01 (m, 3H). ¹³C NMR (75 MHz, CDCl₃) δ= 0.03, 11.93, 21.73, 24.15, 25.49, 28.36, 41.11, 49.56, 73.04, 90.46, 107.33.
HRMS 208.2069 [M - OH] ⁺, calculated 208.1569 [M - OH] ⁺.

### (1R,2S)-2-((methoxymethoxy)methyl)-1-((trimethylsilyl)ethynyl)cyclohexanol (1.9g, 41%) was obtained as a colorless liquid.

TLC (Hexane: EtOAc 9:1): Rf = 0.40
¹H NMR (300 MHz, CDCl₃) δ= δ= 0.18 (s,9H), 1.03-1.29 (m, 2H), 1.46-1.55 (m, 3H), 1.67-1.71 (m, 2H), 1.80-1.90 (m, 1H), 2.02-2.06 (m, 1H), 3.41 (s, 3H), 3.48 (dd, 1 H, J= 4.2, 9.6 Hz) 3.87 (T, 1H, J= 9.9 Hz), 4.64 (s, 2H). ¹³C NMR (75 MHz, CDCl₃) δ= 0.04, 23.16, 24.94, 26.29, 39.48, 46.06, 55.59, 71.68, 72.87, 90.49, 96.49, 106.78.
HRMS 254.2134 [M - OH] ⁺, calculated 254.2124 [M - OH] ⁺.

### General procedure for the synthesis of 2-alkyl-1-(bromoethynyl)cyclohexanoles:

To an acetone solution of 2-alkyl-1-((trimethylsilyl) ethynyl)cyclohexanoles (1.3 mmol), N-bromosuccinimide (1.5 mmol) and AgNO₃(0.5 mmol) was added, and the resulting solution was stirred in darkness for 2h at rt. Acetone was evaporated under reduced pressure and the solids were removed by filtration through a celite pad (washing with ether). The combined organic phase were concentrated and subjected to purification by column chromatography using Hexane: EtOAc 9:1 to yield 2-alkyl-1-(bromoethynyl)cyclohexanoles as yellow liquid.

(1S,2R)-1-(Bromoethynyl)-2-methylcyclohexanol and (1R,2S)-1-(Bromoethynyl)-2-methyl cyclohexanol (256 mg, 91 %) was obtained as a yellow liquid.
TLC (Hexane: EtOAc 9:1): Rf = 0.30
¹H NMR (300 MHz, CDCl₃) δ= 1.06 (d, 3H, J = 6.9 Hz), 1.29-1.73 (m, 8H),1.97-2.04 (m, 1 H). ¹³C NMR (75 MHz, CDCl₃) δ= 16.05, 20.94, 24.95, 29.09, 39.17, 40.52, 43.04, 70.79, 84.60.
HRMS m/z 199.0193, 201.0169 [M - OH] ⁺, calc. 199.0122, 201.0102 [M - OH]⁺.

(1S,2R)-1-(Bromoethynyl)-2-ethylcyclohexanol and (1 R,2S)-1-(Bromoethynyl)-2-ethylcyclo hexanol (264 mg, 88%)was obtained as a yellow liquid.
TLC (Hexane: EtOAc 9:1): Rf = 0.36
¹H NMR (300 MHz, CDCl₃) δ= 0.96 (d, 3H, J = 7.2 Hz), 1.13-1.30 (m, 3H), 1.36-1.45 (m, 1H), 1.50-1.74 (m, 5H), 1.83-2.02 (m, 2H). ¹³C NMR (75 MHz, CDCl₃) δ= 12.17, 21.10, 23.03, 24.81, 25.35, 39.55, 43.17, 47.78, 71.39, 84.74.
HRMS m/z 213.0268, 215.0245 [M - OH] ⁺, calculated 213.0279, 215.0259 [M - OH] ⁺

(1S,2S)-1-(bromoethynyl)-2-((methoxymethoxy)methyl)cyclohexanol (327 mg, 90%) was obtained (350mg) as a yellow liquid.
TLC (Hexane: EtOAc 9:1): Rf = 0.39
¹H NMR (300 MHz, CDCl₃) δ= 1.33-2.07 (m, 9H), 3.41 (s, 3H), 3.59 (dd, 1H, J = 2.4, 9.6 Hz), 4.24 (dd, 1H, J = 3.3, 9.6 Hz), 4.65 (dd, 2H, J = 6.6, 13.2 Hz).
¹³C NMR (75 MHz, CDCl₃) δ= 20.61, 24.67, 25.51, 39.17, 43.23, 45.09, 55.39, 70.66, 70.87, 84.39, 96.51.
HRMS m/z 259.0104, 261.0123[M - OH] ⁺, calculated 259.0334, 261.0314 [M - OH] ⁺.

(1R,2R)-1-(Bromoethynyl)-2-methylcyclohexanol and (1S,2S)-1-(Bromoethynyl)-2-methylcyclohexanol (254 mg, 90%) was obtained as a yellow liquid.
TLC (Hexane: EtOAc 9:1): Rf = 0.28
¹H NMR (300 MHz, CDCl₃) δ= 1.05 (d, 3H, J= 6.6 Hz), 1.20-1.29 (m, 2H), 1.48-1.75 (m, 6H), 2.00-2.05 (m, 1 H). ¹³C NMR (75 MHz, CDCl₃) δ= 16.66, 24,18, 25.43, 32.16, 40.63, 42.73, 45.35, 74.40, 81.18.
HRMS m/z 199.0193, 201.0169 [M - OH] ⁺, calc. 199.0122, 201.0102 [M - OH] ⁺.

(1R,2R)-1-(Bromoethynyl)-2-ethylcyclohexanol and (1S,2S)-1-(Bromoethynyl)-2-ethylcyclo hexanol (267 mg, 89%) was obtained as a yellow liquid.
TLC (Hexane: EtOAc 9:1): Rf = 0.32
¹H NMR (300 MHz, CDCl₃) δ= 0.97 (d, 3H, J = 7.2 Hz), 1.09-1.25 (m, 3H), 1.36-1.76 (m, 6H), 1.87-2.03 (m, 2H). ¹³C NMR (75 MHz, CDCl₃) δ= 12.57, 23.15, 24.97, 25.68, 25.55, 40.51, 45.60, 48.11, 75.20, 81.16.
HRMS m/z 213.0361, 215.0340 [M - OH] ⁺, calc. 213.0279, 215.0259 [M - OH] ⁺.

### General procedure for the synthesis of α-ketoesters:

To a solution of 2-alkyl-1-(bromoethynyl)cyclohexanoles (1.08 mmol) in MeOH (5ml) was added a solution of NaHCO₃ (45.5 mg, 0.54 mmol) and MgSO₄ (261 mg, 2.16 mmol) in water (5ml) at 0°C. The mixture was stirred for 10 min vigorosuly befpre KMnO₄ (513mg, 3.25 mmol) was added. The mixture was allowed to come to rt and stirred at this temperature for 1 h. The solids were removed by filtration through celite pad and washed with ethyl acetate. The organic phase was separated and the aqueous phase was extracted with ethyl acetate (3X 100ml). The combined organic phases were washed with brine (30ml) and dried over MgS04. The solution was concentrated and then flash chromatographed using Hexane: EtOAc 9:1 to afford the corresponding α-ketoesters.

Methyl 2-((1S,2R)-1-hydroxy-2-methylcyclohexyl)-2-oxoacetate and Methyl 2-((1R,2S)-1-hydroxy-2-methylcyclohexyl)-2-oxoacetate (117 mg, 65%) was obtained as a oily liquid.
TLC (Hexane: EtOAc 9:1): Rf = 0.61
¹H NMR (300 MHz, CDCl₃) δ= 0.81 (d, 3H, J = 6.6 Hz), 1.25-1.90 (m, 8H), 2.03- 2.16 (m, 1 H), 3.90 (s, 3H). ¹³C NMR (75 MHz, CDCl₃) δ= 16.00, 20.16, 25.35, 29.27, 34.99, 36.25, 52.74, 80.88, 163.61, 200.66.

Methyl 2-((1S,2R)-2-ethyl-1-hydroxycyclohexyl)-2-oxoacetate and Methyl 2-((1R,2S)-2-ethyl-1-hydroxycyclohexyl)-2-oxoacetate. (160 mg, 69%) was obtained as a oily liquid.
TLC (Hexane: EtOAc 9:1): Rf = 0.64

Methyl 2-((1S,2S)-1-hydroxy-2-((methoxymethoxy)methyl)cyclohexyl)-2-oxoacetate. (170 mg, 60%) was obtained as a oily liquid.
TLC (Hexane: EtOAc 9:1): Rf = 0.60

¹H NMR (300 MHz, CDCl₃) δ= 1.44-2.40 (m, 8H), 2.59-2.70 (m,1 H), 3.27(s, 3H), 3.38- 3.42 (m, 2H), 3.87 (s, 3H), 4.42 (dd, 2H, J = 6.6, 18.6 Hz).
¹³C NMR (75 MHz, CDCl₃) δ= 20.74, 23.65, 24.81, 35.62, 42.23, 52.54, 55.50, 68.34, 78.81, 96.24, 161.37, 197.77.
MS (ESI) m/z 282.73[M + Na]⁺, calculated 282.71 [M + Na] ⁺.

Methyl 2-((1 R,2R)-1-hydroxy-2-methylcyclohexyl)-2-oxoacetate and Methyl 2-((1S,2S)-1-hydroxy-2-methylcyclohexyl)-2-oxoacetate (85 mg, 47%) was obtained as a oily liquid.
TLC (Hexane: EtOAc 9:1): Rf = 0.58
¹H NMR (300 MHz, CDCl₃) δ= 0.76 (d, 3H, J = 3.6 Hz), 1.29-1.83 (m, 8H), 1.99- 2.11 (m, 1 H), 3.86 (s, 3H).
¹³C NMR (75 MHz, CDCl₃) δ= 15.96, 20.11, 25.31, 29.22, 34.95, 36.21, 52.68, 80.84, 163.62, 200.69.

Methyl 2-((1R,2R)-2-ethyl-1-hydroxycyclohexyl)-2-oxoacetate and Methyl 2-((1S,2S)-2-ethyl-1-hydroxycyclohexyl)-2-oxoacetate (127 mg, 55%) was obtained as a oily liquid.
TLC (Hexane: EtOAc 9:1): Rf = 0.61

### General procedure for the synthesis of 2-(1-hydroxy-2-alkylcyclohexyl)-2-oxoacetic acids:

To the above synthesized α-ketoesters was added 1 M LiOH in MeOH: H₂O (1:1) and the reaction stirred for 6h at rt. The reaction was acidified to pH-2 by addition of 1 M HCl. The aqueous layer is extracted with ethyl acetate (3x 20ml). The combined organic phases were washed with brine (30ml) and dried over MgSO4. The solution was concentrated under vacuo to furnish the free acid as an oily liquid.

2-((1S,2R)-1-Hydroxy-2-methylcyclohexyl)-2-oxoacetic acid and 2-((1R,2S)-1-Hydroxy-2-methylcyclohexyl)-2-oxoacetic acid (105 mg, overall yield for 2 steps 52%) was obtained as a oily liquid.
TLC (Hexane: EtOAc: TFA 9:1:0.1): Rf = 0.28
¹H NMR (400 MHz, CDCl₃) δ= 0.78 (d, 3H, J = 6.8 Hz), 1.33-1.95 (m, 8H), 2.15 - 2.24 (m, 1H). ¹³C NMR (100 MHz, CDCl₃) δ= 16.32, 20.32, 25.46, 29.41, 35.12, 36.57, 81.55, 162.81, 200.53.

2-((1S,2R)-2-Ethyl-1-hydroxycyclohexyl)-2-oxoacetic acid and 2-((1R,2S)-2-Ethyl-1-hydr oxycyclohexyl)-2-oxoacetic acid (141 mg, overall yield for 2 steps 65%) was obtained as a oily liquid.

TLC (Hexane: EtOAc: TFA 9:1:0.1): Rf = 0.26 ¹H NMR (400 MHz, CDCl₃) δ= 0.83 (t, 3H, J= 7.6Hz), 1.13-1.36 (m, 4H), 1.57-1.62 (m, 2H), 1.73-1.96 (m, 5H). ¹³C NMR (100 MHz, CDCl₃) δ= 11.82, 20.39, 23.95, 25.08, 25.46, 35.32, 43.14, 82.20, 164.12, 201.23.

2-((1S,2S)-1-hydroxy-2-((methoxymethoxy)methyl)cyclohexyl)-2-oxoacetic acid (170 mg, overall yield for 2 steps 59%) was obtained as a oily liquid.
TLC (Hexane: EtOAc: TFA 9:1:0.1): Rf = 0.26
¹H NMR (300 MHz, CDCl₃) δ= 1.44-1.67 (m, 7H), 2.70-2.80 (m, 1 H), 3.26-3.41 (m, 6H), 4.41-4.47 (m, 2H). ¹³C NMR (75 MHz, CDCl₃) δ= 20.59, 23.34, 24.53, 35.27, 42.79, 55.74, 68.03, 78.38, 96.12, 160.39, 196.63.

2-((1R,2R)-1-Hydroxy-2-methylcyclohexyl)-2-oxoacetic acid and 2-((1S,2S)-1-Hydroxy-2-methylcyclohexyl)-2-oxoacetic acid (68 mg, overall yield for 2 steps 34%) was obtained as a oily liquid.
TLC (Hexane: EtOAc: TFA 9:1:0.1): Rf = 0.24
¹H NMR (300 MHz, CDCl₃) δ= 0.76 (d, 3H, J = 6.6 Hz), 1.29-1.51 (m, 3H), 1.55-1.62 (m, 2H), 1.67-1.72 (m, 2H), 1.83-1.93 (m, 1H), 2.06-2.18 (m, 1H). ¹³C NMR (75 MHz, CDCl₃) δ= 16.00, 20.22, 25.37, 29.31, 34.88, 36.21, 80.82, 164.55, 201.85

2-((1R,2R)-2-Ethyl-1-hydroxycyclohexyl)-2-oxoacetic acid and 2-((1S,2S)-2-Ethyl-1-hydro xycyclohexyl)-2-oxoacetic acid (101 mg, overall yield for 2 steps 51%) was obtained as a oily liquid.
TLC (Hexane: EtOAc: TFA 9:1:0.1): Rf = 0.23
¹H NMR (300 MHz, CDCl₃) δ= 0.83 (t, 3H, J= 4.2Hz), 1.12-1.33 (m, 4H), 1.56-1.63 (m, 2H), 1.72-1.97 (m, 5H). ³C NMR (75 MHz, CDCl₃) δ= 11.79, 20.38, 23.94, 25.08, 25.47, 35.31, 43.15, 82.21, 164.10, 201.14

### Example 2

### Synthesis of building blocks B

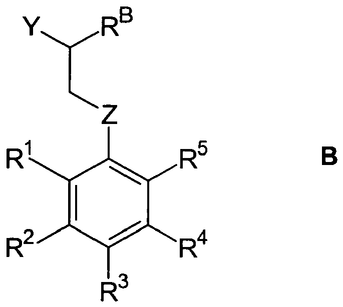

### Synthesis of tert-butyl 2-(3-(3-(3,4-dimethoxyphenyl) propanoyl) phenoxy) acetate.

A solution of the corresponding phenol (T. Keenan et al./Bioorg. Med. Chem. 6 (1998) 1309-1335) (5g, 15.46 mmol) and K₂CO₃ (4.8g, 34.9mmol) in acetone (30mL) was treated with tert-butyl bromoacetate (3.7g, 19.21 mmol) and allowed to stir at room temperature for 20h. After this time the reaction mixture was filtered, concentrated and flash chromatographed to afford tert-butyl 2-(3-(3-(3,4-dimethoxyphenyl)propanoyl)phenoxy)acetate (6.6g, 16.5mmol, 94%).
TLC (Hexane:EtOAc 8:2): RF = 0.50.
¹H NMR (400 MHz, CDCl₃) δ= 1.45 (s, 9H), 2.97 (t, 2H, *J* = 8 Hz), 3.22 (t, 2H, *J* = 8 Hz), 3.82 (s, 3H), 3.83 (s, 3H), 4.52 (s, 2H), 6.73-6.78 (m, 3H), 7.09 (dd, 1H, *J* = 0.8, 2.4 Hz), 7.33 (t, 1H, *J* = 8 Hz), 7.43 (m, 1H), 7.53 (dd, 1H, *J* = 1.2, 7.6).
¹³C NMR (100 MHz, CDCl₃) δ= 27.99, 29.77, 40.70, 55.80, 65.63, 82.55, 111.35, 111.81, 113.06, 119.99, 120.12, 121.39, 129.66, 133.77, 138.22, 147.38, 148.88, 158.12, 167.57, 198.77. MS (ESI) m/z 439.13 [M + K]⁺, calculated 439.15 [M + K]⁺.

### Synthesis of (R)-tert-butyl 2-(3-(3-(3,4-dimethoxyphenyl)-1-hydroxypropyl) phenoxy) acetate

A solution of tert-butyl 2-(3-(3-(3,4-dimethoxyphenyl)propanoyl)phenoxy)acetate (6.5g, 16.48mmol) in isopropanol was charged into the hydrogenation reactor (Highpressure laboratory autoclave Model IV from Roth) along with K₂CO₃ (2.3g, 16.48mmol). The reactor was flushed twice with argon and to the above solution was added Noyori catalyst (ABCR). The reactor was flushed with argon to remove any traces of air and was sealed. Hydrogen gas was flushed into the reactor twice, to flush out argon. The reaction was then stirred at room temperature with hydrogen gas held at 15 bar pressure for 6 days after which time the reaction was filtered through celite pad, and washed continuously with diethyl ether. The organic solvent was dried under vacuum to yield (R)-tert-butyl 2-(3-(3-(3,4-dimethoxyphenyl)-1-hydroxypropyl)phenoxy) acetate (6.2g. 15.42mmol, 94%).
TLC (Hexane:EtOAc 8:2): Rf = 0.3
¹H NMR (300 MHz, CDCl₃) δ= 1.47 (s, 9H), 2.03 (m, 2H), 2.642 (m, 2H), 3.83 (s, 3H), 3.84 (s, 3H), 4.49 (s, 2H), 4.66-4.61 (m, 1H), 6.70-6.80 (m, 4H), 6.95-6.91 (m, 2H), 7.24(t, 1H, *J* = 7.8 Hz). ¹³C NMR (75 MHz, CDCl₃) δ= 28.01, 31.56, 40.62, 55.79, 55.90, 65.62, 73.55, 82.32, 111.31, 111.80, 112.18, 113.53, 119.08, 120.19, 129.48, 134.40, 146.56, 147.16, 148.82, 158.07, 168.01. MS (ESI) m/z 425.20 [M + Na] ⁺, 441.17 [M + K] ⁺, calculated 425.19 [M + Na] ⁺, 441.17 [M + K] ⁺.

### Synthesis of 3-(3,4-Dimethoxyphenyl)-1-(3-(2-morpholinoethoxy)phenyl)-propan-1-one

A solution of the corresponding phenol (15.6 g, 59.5 mmol) in dry DMF (300 ml) under an atmosphere of nitrogen was treated with K₂CO₃ (33.2 g, 240 mmol) and 4-(2-chloroethyl)morpholine hydrochloride (11.1 g, 59.6 mmol). The mixture was heated with stirring at 90°C for 2 hours until TLC indicated complete conversion. The mixture was cooled to room temperature and poured into ice-cold water (3.2 I). The precipitate of the title compound was collected by filtration, washed with water (3 x 200 ml) and dried *in vacuo* to yield 3-(3,4-Dimethoxyphenyl)-1-(3-(2-morpholinoethoxy)phenyl)propan-1-one (17.7 g, 44.3 mmol, 74%) The product was used for the next step without further purification.
¹H NMR (300MHz, CDCl₃) δ= 2.49-2.54 (m, 4H), 2.75 (t, J = 5.7 Hz, 2H), 2.94 (t, J = 7.7 Hz, 2H), 3.20 (t, J = 7.7 Hz, 2H), 3.65-3.69 (m, 4H), 3.79 (s, 3H), 3.81 (s, 3H), 4.08 (t, J = 5.6 Hz, 2H), 6.69-6.74 (m, 3H), 7.04 (dd, J = 0.8 Hz, J = 2.6 Hz, 1 H), 7.29 (t, J = 7.9 Hz, 1 H), 7.41-7.49, (m, 2H).

### Synthesis of (R)-3-(3,4-Dimethoxyphenyl)-1-(3-(2-morpholinoethoxy)phenyl)-propan-1-ol

Dry THF (35 ml) was added under an atmosphere of nitrogen to 3-(3,4-Dimethoxyphenyl)-1-(3-(2-morpholinoethoxy)phenyl)propan-1-one (25 g, 62.6 mmol). The mixture was cooled to -20°C and a 1.8 M solution of (+)-B-chlorodiisopinocampheylborane [(+)-DIP chloride] in hexane (53 ml, 95.4 mmol) which had been diluted with dry THF (70 ml) was slowly added dropwise. The temperature was kept below -10°C and the mixture stirred for 3 hours. It was placed in a refrigerator overnight and another 0.2 equivalents of (+)-DIP chloride was added. After another day at -20°C the solvent was removed under reduced pressure, the residue treated with ether (170 ml) and the mixture cooled to 0°C. Diethanolamine (60 ml) was added and it was stirred for a while. The formed precipitate was removed by filtration and washed with ether. The combined filtrates were concentrated under reduced pressure, and the title compound was obtained from the residue by column chromatography as oil (silica gel; CH₂Cl₂ / MeOH gradient 100:0 to 94:6) to give (R)-3-(3,4-Dimethoxyphenyl)-1-(3-(2-morpholinoethoxy)phenyl)propan-1-ol (17.2 g, mmol, 68 %).
¹H NMR (300MHz, CDCl₃) δ= 1.84-2.08 (m, 2H), 2.27 (bs, 1 H), 2.45-2.52 (m, 4H), 2.52-2.67 (m, 2H), 2.70 (t, J = 5.8 Hz, 2H), 3.61-3.67 (m, 4H), 3.78 (2s, 6H), 4.02 (t, J = 5.8 Hz, 2H), 4.54-4.60 (m, 1H), 6.62-6.76 (m, 4H), 6.82-6.87 (m, 2H), 7.14-7.21 (m, 1H). MS (ESI) m/z 402 [M + H]⁺ ; 424 [M + Na]⁺, calc. 402 [M + H] ⁺, 424 [M + Na]⁺.

### Synthesis of 4-(2-bromoethoxy)-1,2-dimethoxybenzene

A solution of 3,4-dimethoxyphenol (500mg, 3.24 mmol) in acetone (5ml), was added to K₂CO₃ (538 mg, 3.89 mmol) and the reaction was stirred for 10min. Dibromoethane (2.4g, 12.97 mmol) was added to the reaction mixture before being heated to reflux for 12h. After the mixture was cooled to rt,1 M NaOH solution was added. The organic phase was separated and the aqueous phase was extracted with ethyl acetate (3X 30ml). The combined organic phases were washed with brine (30ml) and dried over MgSO₄ and the residue purified by chromatogrpahy using Hexane: EtOAc 7:3 to afford 4-(2-bromoethoxy)-1,2-dimethoxybenzene (510mg, 1.95 mmol, 60%)
TLC (Hexane:EtOAc 7:3): RF = 0.41
¹H NMR (300 MHz, CDCl₃) δ= 3.64 (t, 2H, J= 6.3 Hz), 3.85 (s,3H), 3.87 (s,3H), 4.26 (t, 2H, J= 6.3 Hz), 6.42 (dd, 1H, J= 2.7, 8.7 Hz), 6.57 (d, 1H, J= 2.7Hz), 6.79 (d, !H, J= 8.7Hz). ¹³C NMR (75 MHz, CDCl₃) δ= 20.30, 55.89, 56.42, 65.59, 101.37, 104,27, 111.75, 144.09, 149.97, 152.05
MS (ESI) m/z 261.18 [M + H] ⁺, calculated 261.26 [M + H] ⁺.

### Example 3

### Synthesis of building blocks A

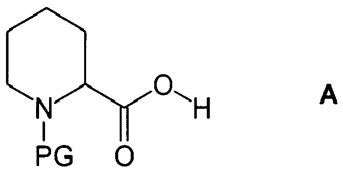

### Synthesis of (S)-1-(((9H-fluoren-9-yl)methoxy)carbonyl)piperidine-2-carboxylic acid

A solution of the L-pipecolic acid (3.6g, 38.7 mmol) in 40ml of 10% sodium carbonate was dissolved in round bottom flask and allowed to stir for 5 min at rt. To this solution was added F-moc succinamide (8.5g, 34.8 mmol) dissolved in 35 ml dioxane and the reaction was stirred overnight. After overnight stirring water was added and the aqueous layer was extracted with ethyl acetate. The aqueous layer was made acidic (pH-2) by addition of concentrated HCl. The acidic layer was extracted with ethyl acetate (3x 40ml). The organic phase was washed with 1 N HCl followed by brine, dried over MgSO₄ and concentrated under vacuo to yield a oily colorless liquid. The oily liquid was dissolved in ether and cooled to get fluffy white solid which was washed with hexane and dried to yield (S)-1-(((9H-fluoren-9-yl)methoxy)carbonyl)piperidine-2-carboxylic acid (8.2g, 38.7mmol, 83%).
TLC (Hexane:EtOAc: TFA 1:1: 0.2): RF = 0.60
HPLC (Gradient A) retention time= 24.6-24.8 min
¹H NMR (300 MHz, CDCl₃) δ=1.28-1.53 (m, 2H), 1.69-1.82 (m, 3H), 2.19-2.37 (m, 1 H), 3.15 (t, 1H, J= 13.2Hz), 4.05-4.33 (m, 2H), 4.37-4.49 (m, 2H), 4.76-5.05(m, 1 H), 7.28-7.41 (m, 4H), 7.55-7.62 (m, 2H), 1.77 (s, 2H).
¹³C NMR (75 MHz, CDCl₃) δ= 20.72, 24.70, 26.55, 41.94, 47.25, 54.19, 67.86, 119.97, 125.08, 127.07, 127.68, 141.33, 143.89, 156.65, 177.36
MS (ESI) m/z 352.66 [M + H]⁺, calculated 352.40 [M + H]⁺.

### Example 4

### Methods for the coupling of pipecolic acids A with building blocks B:

### Synthesis of (S)-1-tert-butyl 2-[2-(3,4-dimethoxyphenyl)ethyl] piperidine-1,2-dicarboxylate

To a solution of 4-(2-bromoethyl)-1,2-dimethoxybenzene (385 mg, 1.57 mmol), in acetone (10ml) was added (S)-1-Boc-piperidine-2-carboxylic acid (300mg, 1.30 mmol), K₂CO₃ (217 mg, 1.57 mmol), and KI (catalytic amount). The reaction mixture was stirred at 60°C for 12h. The mixture was filtered and the solid residue washed with ethyl acetate (3 X 30 ml). The combined organic phases were washed with brine (30ml) and dried over MgS04. The solution was concentrated and then residue was purified by chromatography using Hexane: EtOAc 8:2 to afford (S)-1-tert-butyl 2-[2-(3,4-dimethoxyphenyl)ethyl] piperidine-1,2-dicarboxylate (440mg, 1.12 mmol, 85%). TLC (Hexane:EtOAc 8:2): RF = 0.46
¹H NMR (300 MHz, CDCl₃) δ= 1.46 (s,1H), 1.58-1.59 (m, 3H), 2.17 (d, 1H, j= 13.2 Hz), 2.91 (t, 2H, j= 6.9 Hz), 3.87 (s, 3H), 3.89 (s, 3H), 3.91-4.17 (m, 2H), 4.35 (t, 2H, J= 6.9 Hz), 4.71-4.88(m, 1 H), 6.75-6.83(m, 3H). ¹³C NMR (75 MHz, CDCl₃) δ= 28.36, 34.75, 55.80, 55.94, 65.53, 79.89, 111.35, 112.11, 120.90, 147.78, 148.95.
HRMS m/z 294.1694 [M - Boc + H] ⁺, 394.2277 [M + H] ⁺, 416.2083 [M + Na] ⁺, calculated 294.1716 [M - Boc + H] ⁺, 394.2151 [M + H] ⁺, 416.2044 [M + Na] ⁺.

### Synthesis of (S)-3,4-dimethoxyphenethyl piperidine-2-carboxylate

(S)-1-tert-butyl 2-[2-(3,4-dimethoxyphenyl)ethyl] piperidine-1,2-dicarboxylate (390 mg, 0.991 mmol) was treated with 20% TFA in DCM at rt. The mixture was allowed to stir for 2h. TFA and DCM was evaporated under reduced pressure to yield (S)-3,4-dimethoxyphenethyl piperidine-2-carboxylate (280mg, 0.95mmol, 96.2%).
TLC (Hexane: EtOAc: TEA 7:2.8: 0.2): Rf = 0.24.
¹H NMR (300 MHz, CDCl₃) δ= 1.57 (s, 1 H), 1.84 (s, 4H), 2.18 (d, 1 H, J= 12.9 Hz), 3.54 (s, 1 H), 3.86 (s, 3H), 3.87 (s, 3H), 3.89-3.96 (m, 1 H), 4.37 (d, 3H, J= 7.8 Hz), 6.73 (d, 2H, J= 6.9 Hz), 6.81 (d, 1 H, J= 9.3 Hz).
¹³C NMR (75 MHz, CDCl₃) δ= 21.48, 21.71, 25.52, 34.18, 44.15, 55.83, 55.89, 56.84, 66.97, 111.42, 112.06, 120.81, 129.18, 147.94, 149.00.
MS (ESI) m/z 294.17 [M + H] ⁺, calculated 294.41 [M + H]⁺.

### Synthesis of (S)-1-tert-butyl 2-(2-(3,4-dimethoxyphenoxy)ethyl)-piperidine-1,2-dicarboxylate

To a solution of 4-(2-bromoethoxy)-1,2-dimethoxybenzene (200mg, 0.76 mmol), in acetone (10ml) was added (S)-1Boc-piperidine-2-carboxylic acid (150 mg, 0.65 mmol), K₂CO₃ (108 mg, 0.78 mmol), and KI (catalytic amount). The reaction mixture was stirred at 60°C for 12h. The mixture was filtered and the solid residue washed with ethyl acetate (3 X 30 ml). The combined organic phases were washed with brine (30ml) and dried over MgS04. The solution was concentrated and then residue was purified by chromatography using Hexane: EtOAc 7:3 to afford (S)-1-tert-butyl 2-(2-(3,4-dimethoxyphenoxy)ethyl)-piperidine-1,2-dicarboxylate (200mg, 0.50 mmol, 77%).
TLC (Hexane:EtOAc 7:3): RF = 0.39
¹H NMR (600 MHz, CDCl₃) δ= 1.42 (d, 9H, J= 19.2 Hz), 1.57-1.65 (m, 4H), 2.17-2.23 (m, 1H), 2.86- 3.01 (m, 1H), 3.82 (s,3H), 3.83 (s,3H), 3.88-4.02 (m, 1H), 4.11 (t, 2H, J= 4.8 Hz), 4.45 (t, 2H, J= 4.8 Hz), 4.75 (s, 0.5H), 4.91 (s, 0.5H), 6.37 (dd, 1H, J= 3, 9 Hz), 6.51 (d, 1H, J= 1.2 Hz), 6.76 (d, 1H, J= 8.4 Hz).
¹³C NMR (150 MHz, CDCl₃) δ= 26.79, 28.37, 41.03, 42.08, 53.79, 54.86, 55.81, 56.39, 63.17, 66.49, 101.08, 103.94, 111.68, 143.82, 149.84, 153.02, 171.89.
MS (ESI) m/z 432.20[M + Na] ⁺, 448.20[M + K] ⁺, calculated 432.20 [M + Na] ⁺, 448.17[M + K] ⁺.

### Synthesis of (S)-2-(3,4-dimethoxyphenoxy)ethyl piperidine-2-carboxylate

(S)-1-tert-butyl 2-(2-(3,4-dimethoxyphenoxy)ethyl)-piperidine-1,2-dicarboxylate (200 mg, 0.488 mmol) was treated with 20% TFA in DCM at rt. The mixture was allowed to stir for 2h. TFA and DCM was evaporated under reduced pressure to yield (S)-2-(3,4-dimethoxyphenoxy)ethyl piperidine-2-carboxylate (150mg, 0.48mmol, 99%).
TLC (Hexane: EtOAc: TEA 7:2.8: 0.2): Rf = 0.16.
¹H NMR (600 MHz, CDCl₃) δ= 1.54-1.61 (m, 1 H), 1.82-1.97 (m, 4H), 2.24-2.28 (m, 1H), 2.99-3.04 (m, 1H), 3.55 (d, 1H, J= 12.6 Hz), 3.82 (s, 3H), 3.83 (s, 3H), 3.92 (dd, 1H, J= 3.6, 11.4 Hz), 4.11 (t, 2H, J= 4.2 Hz), 4.45-4.54(m, 2H), 6.35 (dd, 1H, J= 3, 9 Hz), 6.50 (d, 1H, J= 3 Hz), 6.76 (d, 1H, J= 9 Hz).
¹³C NMR (150 MHz, CDCl₃) δ= 21.50, 21.74, 25.60, 44.14, 55.81, 56.39, 56.83, 64.71, 65.85, 100.97, 103.94, 111.74, 143.98, 149.91, 152.71, 168.48.
MS (ESI) m/z 310.36 [M + H] ⁺, calculated 310.26 [M + H] ⁺.

### Synthesis of (S)-1-(9H-fluoren-9-yl)methyl 2-((R)-1-(3-(2-tert-butoxy-2-oxoethoxy) phenyl)-3-(3,4-dimethoxyphenyl)propyl) piperidine-1,2-dicarboxylate

A solution of tert-butyl 2-{3-[3-(3,4-dimethoxyphenyl)-1-hydroxypropyl]phenoxy}acetate (2g, 4.97 mmol), (S)-1-(((9H-fluoren-9-yl)methoxy)carbonyl)piperidine-2-carboxylic acid (1.9g, 5.47 mmol), and DMAP (0.06 g, 0.54mmol) in 30mL DCM at rt was treated with DCC (1.1g, 5.47 mmol). The mixture was stirred for 12h after which time the organic solvent was dried and the solid was dissolved in diethyl ether (50mL) and filtered through a plug of celite. The filtrate was concentrated and then flash chromatographed using Hexane: EtOAc 2:1 to afford (S)-1-(9H-fluoren-9-yl)methyl 2-((R)-1-(3-(2-tert-butoxy-2-oxoethoxy)phenyl)-3-(3,4-dimethoxyphenyl)propyl) piperidine-1,2-dicarboxylate (3.5g, 4.78 mmol, 96%).
TLC (Hexane :EtOAc 2:1): Rf = 0.4.
¹H NMR (600 MHz, CDCl₃) δ= 1.46 (s, 10H), 1.68-1.76 (m, 3H), 1.98-2.04 (m, 1H), 2.14-2.22 (m, 1 H), 2.29-2.33 (m, 1H), 2.41-2.58 (m, 2H), 2.96-3.15 (m, 1 H), 3.81 (s, 3H), 3.83 (s, 3H), 4.07-4.15 (m, 2H), 4.26-4.49 (m, 5H), 5.02 (d, 1 H, J= 5.4 Hz), 5.73-5.77 (m, 1H), 6.57-6.63 (m, 2H), 6.72-6.81 (m, 2H), 6.88 (s, 1H), 6.93-6.95 (m, 1H), 7.16-7.24 (m, 2H), 7.28-7.48 (m, 2H), 7.57-7.80 (m, 1H), 7.69-7.71 (m, 1H), 7.75-7.77 (m, 1H). ¹³C NMR (150 MHz, CDCl₃) δ= 15.35, 17.89, 20.80, 24.76, 26.81, 28.00, 31.10, 38.03, 42.02, 47.20, 55.77, 55.89, 63.75, 65.72, 67.77, 76.18, 82.35, 104.18, 111.26, 111.56, 111.65, 113.23, 113.90, 119.64, 119.88, 119.89, 119.93, 125.06, 127.02, 127.62, 127.66, 129.65, 133.46, 141.26, 141.67, 143.85, 144.08, 147.25, 148.81, 156.38, 158.01, 167.88, 170.86.
MS (ESI) m/z 758.60[M+Na] ⁺, 774.53[M+K] ⁺, calc. 758.33[M+Na] ⁺, 774.30 [M+K] ⁺.

### Synthesis of (S)-((R)-1-(3-(2-tert-butoxy-2-oxoethoxy)phenyl)-3-(3,4-dimethoxyphenyl)-propyl) piperidine-2-carboxylate

(S)-1-(9H-fluoren-9-yl)methyl 2-((R)-1-(3-(2-tert-butoxy-2-oxoethoxy)phenyl)-3-(3,4-dimethoxyphe-nyl)propyl) piperidine-1,2-dicarboxylate (3.5 g, 4.8 mmol) was treated with 20% 4-methylpiperidine in DCM at rt. The mixture was allowed to stir for 4h. 4-methylpiperidine and DCM was evaporated under reduced pressure. Saturated NH₄Cl solution was added to the filtrate and the solution was stirred for 10 min. The organic phase was separated and the aqueous phase was extracted with ethyl acetate (3X 100ml). The combined organic phases were washed with brine (30ml) and dried over MgSO₄ and the residue purified by chromatogrpahy using Hexane: EtOAc : TEA 7:2.8: 0.2 to yield (S)-((R)-1-(3-(2-tert-butoxy-2-oxoethoxy)phenyl)-3-(3,4-dimethoxyphenyl)-propyl) piperidine-2-carboxylate (2.2g, 4.3mmol, 91 %).
TLC (Hexane: EtOAc: TEA 7:2.8: 0.2): Rf = 0.33.
¹H NMR (600 MHz, CDCl₃) δ= 1.42 (s, 9H), 1.43-1.50 (m, 2H), 1.52-1.57 (m, 1H), 1.61-1.68 (m, 1H), 1.69-1.74 (m, 1H), 1.99-2.04 (m, 2H), 2.15-2.21 (m, 1H), 2.44-2.55 (m, 2H), 2.62-2.66 (m, 1H), 3.05-3.08 (m, 1H), 3.42 (dd, 1H, J= 3, 10.2 Hz), 3.78 (s, 3H), 3.79 (s, 3H), 4.45 (s, 2H), 5.65-5.71 (m, 1H), 6.58-6.62 (m, 2H), 6.70-6.74 (m, 2H), 6.81-6.89 (m, 2H), 7.18 (t, 1H, J= 8.4Hz).
¹³C NMR (150 MHz, CDCl₃) δ= 23.53, 24.78, 27.97, 28.41, 31.19, 37.83, 45.08, 55.80, 55.85, 58.08, 65.63, 75.83, 82.29, 112.27, 111.63, 113.07, 113.79, 119.72, 120.09, 129.56, 133.44, 141.60, 146.26, 148.81, 157.97, 167.84, 171.63.
MS (ESI) m/z 514.53[M + H] ⁺, calculated 514.27 [M + H] ⁺.

### Synthesis of (S)-1-(9H-fluoren-9-yl)methyl 2-((R)-3-(3,4-dimethoxyphenyl)-1-(3-(2-morpholinoethoxy)phenyl)propyl) piperidine-1,2-dicarboxylate

A solution of 3-(3,4-dimethoxyphenyl)-1-{3-[2-(morpholin-4-yl)ethoxy]phenyl}propan-1-ol (J. S. Grimley et al. / Bioorg. Med. Chem. Lett. 18 (2008) 759-761) (171 mg, 0.427mmol), (S)-1-(((9H-fluoren-9-yl)methoxy)carbonyl)piperidine-2-carboxylic acid (150mg, 0.427 mmol), and DMAP (5.7 mg, 0.047mmol) in 10mL DCM at rt was treated with DCC (113 mg, 0.51 mmol). The mixture was stirred for 12h after which the organic solvent was dried and the solid was dissolved in diethyl ether (50mL) and filtered through a plug of celite. The filtrate was concentrated and then flash chromatographed using DCM: MeOH 9.7:0.3 to afford (S)-1-(9H-fluoren-9-yl)methyl 2-((R)-3-(3,4-dimethoxyphenyl)-1-(3-(2-morph-olinoethoxy)phenyl)propyl) piperidine-1,2-dicarboxylate (280 mg, 0.38 mmol, 89%).
TLC (EtOAc: 1): Rf = 0.56.
¹H NMR (300 MHz, CDCl₃) δ= 1.31-1.51 (, 2H), 1.67-1.82 (m, 3H), 1.89-1.99 (m, 1H), 2.14-2.39 (m, 2H), 2.47-2.64 (m, 6H), 2.70-2.83 (m, 2H), 2.96-3.21 (m, 1H), 3.74 (s, 4H), 3.83 (s, 3H), 3.85 (s, 3H), 4.00-4.04 (m, 1H), 4.09-4.17 (m, 4H), 4.26-4.49 (m, 3H), 5.74-5.80 (m, 1H), 6.60-6.67 (m, 2H), 6.74-6.94 (m, 4H).
¹³C NMR (75 MHz, CDCl₃) δ= 21.04, 24.95, 25.63, 31.22, 38.09, 41.98, 47.22, 54.00, 55.81, 55.92, 57.59, 60.38, 65.57, 66.75, 67.78, 76.28, 111.33, 111.73, 113.04, 113.93, 118.95, 119.95, 120.10, 125.06, 127.06, 127.67, 129.64, 133.51, 141.27, 141.52, 141.71, 143.87, 144.08, 147.33, 148.87, 156.38, 158.77, 170.94.
MS (ESI) m/z 735.57 [M + H] ⁺, calculated 735.40 [M + H] ⁺.

### Synthesis of (S)-((R)-3-(3,4-dimethoxyphenyl)-1-(3-(2-morpholinoethoxy)phenyl)propyl) piperidine-2-carboxylate

Pipecolic ester 4 (250 mg, 0.34 mmol) was treated with 20% 4-methylpiperidine in DCM at rt. The mixture was allowed to stir for 4h. 4-methylpiperidine and DCM was evaporated under reduced pressure. Saturated NH₄Cl solution was added to the filtrate and the solution was stirred for 10 min. The organic phase was separated and the aqueous phase was extracted with ethyl acetate (3X 100ml). The combined organic phases were washed with brine (30ml) and dried over MgSO₄ and the residue purified by chromatogrpahy using Hexane: EtOAc : TEA 2: 7.8: 0.2 to yield (S)-((R)-3-(3,4-dimethoxyphenyl)-1-(3-(2-morpholinoethoxy)phen-yl)propyl) piperidine-2-carboxylate (160 mg, 0.31 mmol, 84%).
TLC (Hexane: EtOAc: TEA 7:2.8: 0.2): Rf = 0.3.
¹H NMR (300 MHz, CDCl₃) δ= 1.48-1.60 (m, 1H), 1.67-1.89 (m, 2H), 1.89-2.10 (m, 3H), 2.17-2.31 (m, 2H), 2.44-2.62 (m, 1H), 2.85 (s, 2H), 3.06 (s, 3H), 3.08 (s, 3H), 3.27-3.42 (m, 1H), 3.77-3.85 (m, 5H), 4.18-4.35 (m, 2H), 5.70 (t, 1H, J= 7.2 Hz), 6.62-6.66 (m, 2H), 6.72-6.80 (m, 2H), 6.86 (d, 1 H, J= 7.8Hz), 6.95 (s, 1H), 7.19 (t, 1H, J= 8.1 Hz). ¹³C NMR (75 MHz, CDCl₃) δ= 21.59, 22.00, 26.01, 31.15, 37.82, 44.12, 45.89, 55.91, 55.95, 56.67, 57.17, 64.55, 65.60, 77.65, 111.35, 111.83, 112.36, 111.72, 119.27, 120.18, 129.57, 133 .31, 140.98, 147.29, 148.84, 158.45, 167.88. MS (ESI) m/z 513.29 [M + H] ⁺, calculated 513.32 [M + H] ⁺.

### Example 5

### General procedure for the coupling of pipecolic esters with the building blocks C:

To a stirred solution of the free pipecolic amines in acetonitrile under argon was added sequentially N,N-Diisopropyl-ethylamine (DIPEA), HATU and the building blocks C. The reaction mixture was stirred for 16h at rt. Saturated NH₄Cl solution was added to the reaction and the solution was stirred for 10 min. The organic phase was separated and the aqueous phase was extracted with ethyl acetate (3* 100ml). The combined organic phases were washed with brine (10ml) and dried over MgSO₄ and the residue purified by column chromatogrpahy.

### Synthesis of 2-(3-((R)-3-(3,4-dimethoxyphenyl)-1-((S)-1-(2-((1S,2R)-1-hydroxy-2-methyl cyclohexyl)-2-oxoacetyl)piperidine-2-carbonyloxy)propyl)phenoxy) acetic acid

To (S)-((R)-1-(3-(2-tert-butoxy-2-oxoethoxy)phenyl)-3-(3,4-dimethoxyphenyl)-propyl) piperi dine-2-carboxylate (211mg, 0.412 mmol) was added DIPEA (160 mg, 1.24 mmol), HATU (234 mg, 0.618 mmol), 2-((1S,2R)-1-hydroxy-2-methylcyclohexyl)-2-oxoacetic acid (92 mg, 0.494 mmol) and the reaction was followed as described above. The residue obtained was purified by column chromatography using Hexane : EtOAc 6:4 to yield the protected material (46mg, 0.067 mmol, 20%).
TLC (Hexane: EtOAc 6:4): Rf = 0.41.
HPLC (Gradient A) retention time= 32.1-32.6 min
¹H NMR (600 MHz, CDCl₃) δ= 0.84 (t, 3H, J= 5.4 Hz), 1.30-1.41 (m, 6H), 1.47 (m, 9H), 1.61-1.85(m, 7H), 2.00-2.13 (m, 2H), 2.20-2.28(m, 1H), 2.36 (d. 1H, J= 13.8Hz), 2.47-2.61 (m, 2H), 3.10-3.17 (m, 1H), 3.49 (d, 1H, J= 13.2 HZ), 3.85 (s, 6H), 4.53 (s, 2H), 5.29 (s, 1H), 5.74-5.79 (m, 1H), 6.66-6.67 (m, 2H), 6.76-6.78 (m, 1H), 6.81-6.84 (m, 1H), 6.88-6.93 (m, 1H), 6.95-7.00 (m, 1H), 7.25-7.28 (m, 1H).
¹³C NMR (150 MHz, CDCl₃) δ= 16.14, 20.33, 20.92, 25.33, 26.24, 28.02, 29.97, 36.89, 38.05, 44.31, 51.53, 55.80, 65.70, 76.69, 81.21, 82.35, 111.27, 111.68, 113.18, 114.25, 119.85, 120.13, 129.73, 133.47, 141.38, 147.34, 148.83, 158.07, 166.55, 167.87, 169.27, 205.06.
MS (ESI) m/z 682.07 [M + H] ⁺, calculated 682.85 [M + H] ⁺.

The protected material (46 mg, 0.067 mmol) was treated with 20% TFA in DCM at rt. The mixture was allowed to stir for 6h. TFA and DCM was evaporated under reduced pressure to yield 2-(3-((R)-3-(3,4-dimethoxyphenyl)-1-((S)-1-(2-((1S,2R)-1-hydroxy-2-methyl cyclohexyl)-2-oxoacetyl)piperidine-2-carbonyloxy)propyl)phenoxy) acetic acid (32mg, 0.051 mmol, 77%).
TLC (Hexane: EtOAc: TFA 6:3.9: 0.1): Rf = 0.33.
HPLC (Gradient A) retention time= 24.6-25.1 min
¹H NMR (600 MHz, CDCl₃) δ= 0.82-0.88 (m, 3H), 1.36-1.92 (m, 13H), 2.03-2.13 (m, 2H), 2.23-2.38 (m, 2H), 2.50-2.67 (m, 2H), 3.24-3.31 (m, 1H), 3.48-3.55 (m, 1H), 3.85 (s, 3H), 3.86 (s, 3H), 4.67 (s, 2H), 5.25-5.27 (m, 2H), 5.74-5.77 (m, 1H), 6.56-6.70 (m, 2H), 6.77-6.80 (m, 1H), 6.82-6.87 (m, 1H), 6.89-6.96 (m, 2H), 7.26-7.29 (m, 1H). ¹³C NMR (150 MHz, CDCl₃) δ= 16.3, 20.16, 20.87, 24.79, 25.29, 26.55, 29.30, 31.35, 35.59, 37.60, 39.45, 44.28, 51.92, 55.87, 55.92, 65.07, 76.86, 82.24, 111.37, 111.70, 115.71, 116.21, 119.71, 120.20, 129.90, 133.21, 141.51, 147.41, 148.89, 157.74, 167.39, 169.20, 171.63, 205.23.
MS (ESI) m/z: found Rt 13.88 min. (Method LCMS), 648.45 [M + Na]⁺,
HRMS 626.2902 [M + H] ⁺, calculated 626.2887 [M + H] ⁺.

The diasteromeric mixture was further separated using preparative HPLC Gradient 62-77% B for 35min to yield diasteromer **1 a** (6mg) and **1 b** (9mg).

### 1a: HPLC (Gradient A) retention time= 24.6-24.8min

¹H NMR (600 MHz, CDCl₃) δ= 0.82 (d, 3H, J= 5.4 Hz), 1.38-1.43 (m, 2H), 1.44-1.48 (m, 2H), 1.53-1.58 (m, 2H), 1.64-1.70 (m, 3H), 1.74-1.81 (m, 2H), 2.04-2.12 (m, 2H), 2.22-2.28 (m, 1H), 2.52-2.67 (m, 2H), 2.98 (d, 1 h, J= 5.4 Hz), 3.08 (s, 1H), 3.12 (s, 1H), 3.25 (dt, 1H, J= 2.4, 13.2 Hz), 3.53 (d, 1H, J=13.2 Hz), 3.64-3.67 (m, 1H), 3.72 (s, 1H), 3.85 (s, 3H), 3.86 (s, 3H), 4.63 (s, 2H), 5.24 (d, 1H, J= 4.8Hz), 5.74-5.80 (m, 1H), 6.66-6.69 (m, 2H), 6.77-6.79 (m, 1H), 6.83-6.94 (m, 3H), 7.26-7.28 (m, 1H).
¹³C NMR (150 MHz, CDCl₃) δ= 16.15, 20.18, 21.06, 24.79, 25.27, 26.52, 29.68, 31.41, 35.57, 36.61, 37.64, 44.18, 51.88, 55.86, 55.92, 63.81, 81,38, 111.35, 111.68, 115.65, 115.66, 119.54, 120.16, 129.85, 133.19, 141.53, 147.45, 148.93, 157.92, 167.57, 169.26, 169.26, 205.46.
MS (ESI) m/z: found Rt 13.87 min. (Method LCMS), 648.40 [M + Na]⁺, calculated 648.45 [M + Na] ⁺.

### 1b: HPLC (Gradient A) retention time= 24.9-25.1 min

¹H NMR (600 MHz, CDCl₃) δ= 0.84 (d, 3H,J= 6.6Hz), 1.38-1.85 (m, 10H), 2.06 (s, 2H), 2.20-2.31 (m, 1H), 2.49-2.65 (m, 2H), 2.97 (d, 1H, J= 6.6 Hz), 3.05 (s, 1H), 3.12 (s, 1H), 3.25 (t, 1H, J= 12.6Hz), 3.48 (d, 1H, J= 10.8 Hz), 3.65 (s, 1H), 3.72 (s, 2H), 3.84 (s, 3H), 3.85 (s, 3H), 4.81 (s, 2H), 5.26 (s, 1H), 5.74 (s, 1H), 6.66-6.68 (m, 2H), 6.77-6.94 (m, 4H), 7.21-7.24 (m, 1H).
¹³C NMR (150 MHz, CDCl₃) δ= 16.15, 20.21, 20.94, 24.82, 25.31, 26.40, 29.68, 31.35, 35.31, 36.72, 37.15, 42.16, 43.25, 44.25, 44.54, 46.53, 48.81, 51.75, 55.86, 55.92, 56.79, 63.84, 81.66, 111.34, 111.70, 115.51, 119.59, 120.17, 129.82, 133.32, 141.58, 147.41, 148.91, 157.91, 167.37, 169.34, 205.95. MS (ESI) m/z: found Rt 13.91 min. (Method LCMS), 648.31 [M + Na]⁺, calculated 648.45 [M + Na] ⁺.

### Synthesis of 3,4-dimethoxyphenethyl 1-(2-((1S,2R)-1-hydroxy-2-methylcyclohexyl)-2-oxoacetyl)piperidine-2-carboxylate

To (S)-3,4-dimethoxyphenethyl piperidine-2-carboxylate (33mg, 0.112 mmol) was added DIPEA (43.4 mg, 0.336 mmol), HATU (40.5mg, 0.168 mmol) and 2-((1S,2R)-1-hydroxy-2-methylcyclohexyl)-2-oxoacetic acid (25 mg, 0.134 mmol) . The reaction was followed as described above. The residue obtained was purified by column chromatography using Hexane : EtOAc 6:4 to yield 3,4-dimethoxyphenethyl 1-(2-((1S,2R)-1-hydroxy-2-methylcyclohexyl)-2-oxoacetyl)piperidine-2-carboxylate (25mg, 0.054 mmol, 49%).
TLC (Hexane: EtOAc 6:4): Rf = 0.57.
HPLC (Gradient A) retention time= 25.8-26.2 min
¹H NMR (600 MHz, CDCl₃) δ= 0.83 (m, 3H), 1.39-1.77 (m, 14H), 2.07-2.12 (m, 2H), 2.26 (d, 1H, J= 14.4 Hz), 2.89-2.94 (m, 2H), 3.09-3.17 (m, 1H), 3.46 (t, 1H, J= 11.4 Hz), 3.86 (s, 3H), 3.87 (s, 3H), 4.31-4.43 (m, 2H), 5.24 (s, 1H), 6.72-6.81 (m, 3H).
¹³C NMR (150 MHz, CDCl₃) δ= 16.12, 20.35, 20.80, 24.95, 25.37, 26.30, 29.50, 34.56, 35.67, 36.81, 44.04, 51.46, 55.86, 55.91, 65.99, 81.23, 111.29, 111.99, 120.93, 129.85, 147.78, 148.95, 166.83, 169.99, 204.98.
MS (ESI) m/z: found Rt 14.16 min. (Method LCMS), 462.70 [M + H]⁺, 484.44 [M + Na]⁺, HRMS 462.2944 [M + H]⁺, calculated 462.2914 [M + H] ⁺.

### Synthesis of 2-(3,4-dimethoxyphenoxy)ethyl 1-(2-((1S,2R)-1-hydroxy-2-methylcyclohexyl)-2-oxoacetyl)piperidine-2-carboxylate

To (S)-2-(3,4-dimethoxyphenoxy)ethyl piperidine-2-carboxylate (34.6 mg, 0.112 mmol) was added DIPEA (43.4 mg, 0.336 mmol), HATU (40.5mg, 0.168 mmol) and 2-((1S,2R)-1-hydroxy-2-methylcyclohexyl)-2-oxoacetic acid (25 mg, 0.134 mmol). The reaction was followed as described above. The residue obtained was purified by column chromatography using Hexane : EtOAc 1:1 to yield 2-(3,4-dimethoxyphenoxy)ethyl 1-(2-((1S,2R)-1-hydroxy-2-methylcyclohexyl)-2-oxoacetyl)piperidine-2-carboxylate (33mg, 0.069 mmol, 62%).
TLC (Hexane: EtOAc 1:1): Rf = 0.28.
HPLC (Gradient A) retention time= 25.8-26.2 min
¹H NMR (600 MHz, CDCl₃) δ= 0.821 (dd, 3H, J= 6.6Hz), 1.41-1.79 (m, 13H), 2.04-2.13 (m, 1H), 2.35 (d, 1H, J= 13.8 Hz), 3.22- 3.34 (m, 1H), 3.51 (t, 1H, J= 12Hz), 3.83 (s, 3H), 3.85 (s, 3H), 4.13-4.15 (m, 2H), 4.43- 4.56 (m, 2H), 5.30 (d, 1H, J= 5.4Hz), 6.37-6.40 (m, 1H), 6.52 (t, 1H, J= 3Hz), 6.76 (d, 1H, J= 9Hz).
¹³C NMR (150 MHz, CDCl₃) δ= 16.13, 20.33, 21.06, 24.84, 26.40, 29.47, 34.60, 35.64, 36.82, 44.09, 51.49, 55.85, 56.40, 63.77, 66.35, 81.18, 101.09, 104.07, 111.71, 143.92, 149.88, 152.83, 166.98, 170.03, 205.03.
MS (ESI) m/z: found Rt 13.91 min. (Method LCMS), 478.35 [M + 1]⁺, 500.40 [M + Na]⁺, HRMS 478.2405 [M + H] ⁺, calculated 478.2403 [M + H] ⁺.

### Synthesis of 2-(3,4-dimethoxyphenoxy)ethyl 1-(2-((1 S,2R)-2-ethyl-1-hydroxycyclohexyl)-2-oxoacetyl) piperidine-2-carboxylate

To (S)-2-(3,4-dimethoxyphenoxy)ethyl piperidine-2-carboxylate (64.4 mg, 0.208 mmol) was added DIPEA (81 mg, 0.624 mmol), HATU (75 mg, 0.312 mmol) and 2-((1S,2R)-2-ethyl-1-hydroxycyclohexyl)-2-oxoacetic acid (50 mg, 0.250 mmol). The reaction was followed as described above. The residue obtained was purified by column chromatography using Hexane: EtOAc 1:1 to yield 2-(3,4-dimethoxyphenoxy)ethyl 1-(2-((1S,2R)-2-ethyl-1-hydroxycyclohexyl)-2-oxoacetyl) piperidine-2-carboxylate (25 mg, 0.051 mmol, 25%).
TLC (Hexane: EtOAc 6:4): Rf = 0.50.
HPLC (Gradient A) retention time= 25.5-25.9 min
¹H NMR (600 MHz, CDCl₃) δ= 0.85-0.88 (m, 3H), 1.41-1.88 (m, 18H), 2.35 (d, 1H, J= 12Hz), 3.22-3.29 (m, 1H), 3.49-3.53 (m, 1H), 3.83 (s, 3H), 3.85 (s, 3H), 4.13-4.16 (m, 2H), 4.35-4.57 (m, 2H), 5.31 (s, 1H), 6.37-6.40 (m, 1H), 6.51-6.53 (m, 1H), 6.77 (d, 1H, J= 9Hz). ¹³C NMR (150 MHz, CDCl₃) δ= 11.78, 20.66, 20.90, 23.67, 24.94, 25.46, 26.42, 35.91, 43.72, 44.06, 55.85, 56.40, 63.70, 66.36, 82.34, 101.10, 104.08, 111.72, 143.97, 149.88, 152.85, 152.90, 166.82, 170.01, 205.09. MS (ESI) m/z: found Rt 13.69min. (Method LCMS), 492.21 [M + H]⁺, calc. 492.24 [M + H] ⁺.

### Synthesis of (S)-((R)-3-(3,4-dimethoxyphenyl)-1-(3-(2-morpholinoethoxy)phenyl) propyl) 1-(2-((1S,2R)-1-hydroxy-2-methylcyclohexyl)-2-oxoacetyl)piperidine-2-carboxylate

To (S)-((R)-3-(3,4-dimethoxyphenyl)-1-(3-(2-morpholinoethoxy)phenyl)propyl) piperidine-2-carboxylate 60mg, 0.117 mmol) was added DIPEA (60 mg, 0.468 mmol), HATU (66mg, 0.175mmol) and 2-((1S,2R)-1-hydroxy-2-methylcyclohexyl)-2-oxoacetic acid (26 mg, 0.14 mmol) and the reaction was followed as described above. The residue obtained was purified by column chromatography using Hexane : EtOAc 1:1 to yield (S)-((R)-3-(3,4-dimethoxyphenyl)-1-(3-(2-morpholinoethoxy)phenyl)propyl) 1-(2-((1S,2R)-1-hydroxy-2-methylcyclohexyl)-2-oxoacetyl)piperidine-2-carboxylate (41 mg, 0.060mmol, 52%).
TLC (DCM: MeOH 9.3:0.7): Rf = 0.50.
HPLC (Gradient A) retention time= 21.1-21.7 min
¹H NMR (400 MHz, CDCl₃) δ= 0.79-0.86 (m, 3H), 1.40-1.74 (m, 13H), 1.98-2.38 (m, 7H), 2.48-2.61 (m, 2H), 2.83-2.87 (m, 1H), 2.96-3.05 (m, 2H), 3.09-3.18 (m, 1H), 3.47-3.51 (m, 1H), 3.77-3.85 (m, 11H), 4.17-4.24 (m, 2H), 5.29 (t, 1H, J= 4.4 Hz), 5.74-5.77 (m, 1H), 6.65-6.68 (m, 2H), 6.75-6.78 (m, 2H), 6.81-6.84 (m, 1H), 6.86-6.96 (m, 2H), 7.22-7.27 (m, 1H).
¹³C NMR (100 MHz, CDCl₃) δ= 16.12, 16.14, 20.25, 20.29, 20.90, 21.11, 24.88, 24.92, 25.32, 25,35, 26.23, 26.32, 29.34, 29.41, 31.17, 31.25, 34.85, 35.31, 36.89, 36.90, 38.01, 38.24, 44.24, 44.29, 51.49, 51.64, 53.67, 53.74, 55.81, 55.90, 57.31, 57.36, 64.82, 64.82, 65.83, 66.03, 77.22, 81.16, 81.23, 111.23,111.26, 111.65, 111.69, 112.73, 113.06, 114.08, 114.38, 119.23, 119.40, 120.11, 120.13, 129.73, 129.78, 133.30, 133.46, 141.41, 141.59, 147.33, 148.82, 148.85, 158.36, 158.42, 166.59, 166.73, 169.31, 169.40, 205.13, 205.47.
MS (ESI) m/z: found Rt 11.32min. (Method LCMS), 681.38 [M + H]⁺,
HRMS 681.4418 [M + Na] ⁺, calculated 681.4373.

### Synthesis of 2-(3-((R)-3-(3,4-dimethoxyphenyl)-1-((S)-1-(2-((1S,2R)-2-ethyl-1-hydroxy-cyclohexyl) -2-oxoacetyl)piperidine-2-carbonyloxy) propyl)phenoxy) acetic acid

To (S)-((R)-1-(3-(2-tert-butoxy-2-oxoethoxy)phenyl)-3-(3,4-dimethoxyphenyl)-propyl) pipe-ridine-2-carboxylate (214mg, 0.416 mmol) was added DIPEA (161 mg, 1.25 mmol), HATU (236 mg, 0.624 mmol) and 2-((1S,2R)-2-ethyl-1-hydroxycyclohexyl)-2-oxoacetic acid (100 mg, 0.499 mmol) and the reaction was followed as described above. The residue obtained was purified by column chromatography using Hexane : EtOAc 6:4 to yield the protected material (62mg, 0.089 mmol, 21 %).
TLC (Hexane: EtOAc 6:4): Rf = 0.71.
HPLC (Gradient A) retention time= 32.6-32.9 min
¹H NMR (600 MHz, CDCl₃) δ= 0.87 (t, 3H, J= 7.2Hz), 1.28-1.37 (m, 4H), 1.48 (s, 9H), 1.65-1.88 (m, 11H), 2.00-2.09 (m, 1H), 2.20-2.27 (m, 1H), 2.37 (d, 1H, J =13.8Hz), 2.47-2.62 (m, 2H), 3.05-3.20 (m, 1H), 3.49-3.53 (m, 1H), 3.85 (s, 3H), 3.86 (s, 3H), 4.52 (d, 2H, J=4.8 Hz), 5.31 (d, 1H, J= 5.4 Hz), 5.75-5.79 (m, 1H), 6.65-6.67 (m, 2H), 6.76-6.78 (m, 1H), 6.81-6.83 (m, 1H), 6.87-7.00 (m, 2H), 7.25-7.28 (m, 1H).
¹³C NMR (150 MHz, CDCl₃) δ= 11.78, 20.65, 23.65, 23.66, 24.92, 25.27, 26.26, 28.02, 29.66, 31.17, 35.27, 38.04, 44.28, 51.65, 55.80, 55.90, 65.70, 76.69, 81.99, 82.36, 111.27, 111.68, 113.44, 114.22, 119.87, 120.13, 129.72, 133.48, 141.37, 147.27, 148.83, 156.05, 158.06, 166.58, 167.83, 169.26, 205.17.
MS (ESI) m/z 696.84 [M + H] ⁺, calculated 696.72 [M + H] ⁺.

The protected material (62 mg, 0.089 mmol) was treated with 20% TFA in DCM at rt. The mixture was allowed to stir for 6h. TFA and DCM was evaporated under reduced pressure to yield the free acid 2-(3-((R)-3-(3,4-dimethoxyphenyl)-1-((S)-1-(2-((1S,2R)-2-ethyl-1-hydroxy-cyclohexyl)-2-oxoacetyl)piperidine-2-carbonyloxy)propyl)phenoxy)acetic acid (40mg, 0.062mmol, 80%).

TLC (Hexane: EtOAc: TFA 1:1: 0.2): Rf = 0.45.
HPLC (Gradient A) retention time= 25.3-25.9 min
MS (ESI) m/z: found Rt 15.93 min. (Method LCMS), 662.63 [M + Na]⁺.
HRMS 640.3739[M + H] ⁺, calculated 640.3043 [M + H] ⁺.

The diasteromeric mixture was further separated using preparative HPLC Gradient 65-70% B for 15min to yield diasteromer **6a** (5mg) and **6b** (7mg).

### 6a: HPLC (Gradient A) retention time= 25.3-25.5min

HRMS 640.3773[M + H] ⁺, calculated 640.3043 [M + H] ⁺.

### 6b: HPLC (Gradient A) retention time= 25.7-25.9min

HRMS 640.3764[M + H] ⁺, calculated 640.3043 [M + H] ⁺.

### Synthesis of (S)-((R)-3-(3,4-dimethoxyphenyl)-1-(3-(2-morpholinoethoxy)phenyl) propyl)1-(2-((1S,2R)-2-ethyl-1-hydroxycyclohexyl)-2-oxoacetyl)piperidine-2-carboxylate

To (S)-((R)-3-(3,4-dimethoxyphenyl)-1-(3-(2-morpholinoethoxy)phenyl)propyl) piperidine-2-carboxylate (60 mg, 0.117 mmol) was added DIPEA (55 mg, 0.425 mmol), HATU (60mg, 0.158 mmol) and 2-((1S,2R)-2-ethyl-1-hydroxycyclohexyl)-2-oxoacetic acid (28 mg, 0.139 mmol) and the reaction was followed as described above. The residue obtained was purified by column chromatography using Hexane : EtOAc 1:1 to yield (S)-((R)-3-(3,4-dimethoxyphenyl)-1-(3-(2-morpholinoethoxy)phenyl)propyl)1-(2-((1S,2R)-2-ethyl-1-hydroxycyclohexyl)-2-oxoacetyl)piperidine-2-carboxylate (20mg, 0.028 mmol, 25%).
TLC (DCM: MeOH 9.7: 0.3): Rf = 0.48.
HPLC (Gradient A) retention time= 21.7-22.3 min
¹H NMR (300 MHz, CDCl₃) δ= 0.77-0.86 (m, 3H), 1.41-1.74 (m, 15H), 1.96-2.36 (m, 7H), 2.45-2.61 (m, 2H), 2.87-2.89 (m, 1H), 3.00-3.10 (m, 2H), 3.12-3.19 (m, 1H), 3.47-3.51 (m, 1H), 3.77-3.86 (m, 11H), 4.19-4.25 (m, 2H), 5.29 (t, 1H, J= 4.8 Hz), 5.74-5.77 (m, 1H), 6.65-6.69 (m, 2H), 6.76-6.78 (m, 2H), 6.81-6.84 (m, 1H), 6.84-6.98 (m, 2H), 7.23-7.28 (m, 1H).
¹³C NMR (75 MHz, CDCl₃) δ=11.83, 12.40, 20.51, 20.63, 21.55, 22.68, 23.63, 24.93, 25.25, 25.45, 29.36, 30.57, 31.19, 31.29, 31.92, 34.27, 35.56, 38.61, 43.69, 43.77, 44.31, 51.50, 51.62, 53.65, 55.87, 55.95, 57.31, 61.77, 65.56, 70.36, 70.61, 77.22, 82.04, 111.38, 111.78, 113.11, 114.10, 114.42, 120.20, 129.83, 130.91, 133.39, 133.53, 141.61, 147.42, 148.94, 158.32, 158.45, 166.65, 166.69, 169.34, 169.43, 205.13, 205.41. MS (ESI) m/z: found Rt 8.95 min. (Method LCMS), 695.45 [M + H] ⁺, HRMS 695.4492 [M + H] ⁺, calculated 695.4429 [M + H] ⁺.

### Synthesis of 2-(3-((R)-3-(3,4-dimethoxyphenyl)-1-((S)-1-(2-((1S,2S)-1-hydroxy-2-(hydroxylmethyl)cyclohexyl)-2-oxoacetyl)piperidine-2-carbonyloxy)propyl)phen oxy)acetic acid

To (S)-((R)-1-(3-(2-tert-butoxy-2-oxoethoxy)phenyl)-3-(3,4-dimethoxyphenyl)-propyl) piperidine-2-carboxylate (208mg, 0.404 mmol) was added DIPEA (158 mg, 1.22 mmol), HATU (230 mg, 0.608 mmol) and 2-((1S,2S)-1-hydroxy-2-((methoxymethoxy)methyl)cyclohexyl)-2-oxoacetic acid (120 mg, 0.487 mmol) and the reaction was followed as described above. The residue obtained was purified by column chromatography using Hexane : EtOAc 1:1 to yield the protected material (60mg, 0.080 mmol, 20%).
TLC (Hexane: EtOAc 1:1): Rf = 048.
¹H NMR (600 MHz, CDCl₃) δ= 1.26-1.37 (m, 4H), 1.46 (s, 9H), 1.51-1.63 (m, 3H), 1.64-1.79 (m, 5H), 1.96- 2.08 (m, 1H), 2.15-2.26 (m, 2H), 2.33 (d, 1H, J= 14.4 Hz), 2.45-2.62 (m, 2H), 3.13-3.21 (m, 1H), 3.25 (s, 1H), 3.27-3.29 (m, 1H), 3.30 (s, 1H), 3.48-3.53 (m, 1H), 3.55-3.58 (m, 1H), 3.64-3.67 (m, 1H), 3.83-2.84 (m, 6H), 4.44-4.48 (m, 1H), 4.50 (s, 2H), 4.51-4.59 (m, 1H), 5.28 (t, 1H, J= 5.4 Hz), 5.72-5.78 (m, 1H), 6.64-6.67 (m, 2H), 6.75-6.77 (m, 1H), 6.79-6.82 (m, 1H), 6.88-7.00 (m, 2H), 7.23-7.26 (m, 1H).
¹³C NMR (150 MHz, CDCl₃) δ= 14.09, 20.38, 20.40, 22.67, 24.67, 24.72, 24.97, 24.99, 25.12, 25.17, 26.32, 26.46, 29.35, 29.67, 29.69, 31.13, 31.91, 31.92, 35.50, 36.16, 37.98, 38.03, 41.50, 41.64, 44.13, 44.20, 51.60, 51.67, 55.29, 55.35, 55.80, 55.89, 65.69, 69.84, 69.89, 76.56, 76.58, 81.26, 81.28, 82.36, 82.38, 96.39, 96.49, 111.26, 111.67, 113.27, 114.06, 114.13, 119.77, 119.89, 120.13, 129.62, 129.69, 133.40, 133.48, 141.35, 141.54, 147.26, 147.29, 148.82, 148.83, 159.01, 158.04, 165.87, 166.07, 166.81, 167.09, 167.89, 167.90, 169.47, 169.50, 205.48, 206.00 MS (ESI) m/z 764.51 [M + Na] ⁺, calculated 764.36 [M + Na] ⁺.

The protected material (60 mg, 0.080 mmol) was treated with 20% TFA in DCM at rt. The mixture was allowed to stir for 6h. TFA and DCM was evaporated under reduced pressure to yield the free acid 2-(3-((R)-3-(3,4-dimethoxyphenyl)-1-((S)-1-(2-((1S,2S)-1-hydroxy-2-(hydroxyl methyl) cyclohexyl)-2-oxoacetyl)piperidine-2-carbonyloxy) propyl) phenoxy) acetic acid (16mg, 0.024mmol, 31%).
TLC (Hexane: EtOAc: TFA 6: 4: 0.1): Rf = 0.41.
HPLC (Gradient A) retention time= 25.7-26.1 min
¹H NMR (600 MHz, CDCl₃) δ= 1.32-1.88 (m, 13H), 2.02-2.10 (m, 1H), 2.16-2.28 (m, 2H), 2.37-2.40 (m, 1 H), 2.51-2.65 (m, 2H), 3.20-3.26 (m, 1 H), 3.51-3.59 (m, 1 H), 3.60-3.67 (m, 1 H), 3.73-3.80 (m, 1 H), 3.85 (s, 6H), 4.63-4.70 (m, 2H), 5.32 (d, 1 H, J= 5.4 Hz), 5.73-5.79 (m, 1 H), 6.66-6.70 (m, 2H), 6.77-6.80 (m, 1 H), 6.84-6.89 (m, 2H), 6.92-6.96 (m, 1 H), 7.25-7.29 (m, 1H).
¹³C NMR (150 MHz, CDCl₃) δ= 20.67, 20.80, 21.19, 23.60, 24.92, 25.47, 26.60, 29.68, 31.29, 34.48, 44.50, 44.56, 51.92, 55.91, 65.06, 69.07, 76.67, 81.87, 111.35, 111.72, 115.07, 115.16, 119.99, 120.18, 129.84, 133.26, 141.63, 147.40, 148.85, 157.77, 166.31, 169.38, 169.47, 204.17.
MS (ESI) m/z: found Rt 13.12 min. (Method LCMS), 642.90 [M + H]⁺.
HRMS 642.3570 [M + H] ⁺, calculated 642.3536 [M + H] ⁺.

### Synthesis of 2-(3-((R)-3-(3,4-dimethoxyphenyl)-1-((S)-1-(2-((1R,2R)-1-hydroxy-2-methyl cyclohexyl)-2-oxoacetyl) piperidine-2-carbonyloxy) propyl) phenoxy) acetic acid

To (S)-((R)-1-(3-(2-tert-butoxy-2-oxoethoxy)phenyl)-3-(3,4-dimethoxyphenyl)-propyl) piperidine-2-carboxylate (210mg, 0.412 mmol) was added DIPEA (160 mg, 1.24 mmol), HATU (234 mg, 0.618 mmol) and 2-((1R,2R)-1-hydroxy-2-methylcyclohexyl)-2-oxoacetic acid (92 mg, 0.494 mmol) and the reaction was followed as described above. The residue obtained was purified by column chromatography using Hexane : EtOAc 6:4 to yield the protected material (38mg, 0.055 mmol, 14%).

The protected material(38 mg, 0.055 mmol) was treated with 20% TFA in DCM at rt. The mixture was allowed to stir for 6h. TFA and DCM was evaporated under reduced pressure to yield the free acid 2-(3-((R)-3-(3,4-dimethoxyphenyl)-1-((S)-1-(2-((1R,2R)-1-hydroxy-2-methyl cyclohexyl) -2-oxoacetyl) piperidine-2-carbonyloxy) propyl) phenoxy)acetic acid (27mg, 0.043mmol, 77%).
HPLC (Gradient A) retention time=27.3-27.7min
MS (ESI) m/z: found Rt 15.6 min. (Method LCMS), 648.45 [M + Na]⁺,
calculated 648.45 [M + Na] ⁺.

The diasteromeric mixture was further separated using preparative HPLC Gradient 61-71% B for 20min to yield diasteromer **9a** (5mg) and **9b** (8mg).

### 9a: HPLC (Gradient A) retention time= 27.2-27.4min

MS (ESI) m/z: found Rt 15.51 min. (Method LCMS), 648.59 [M + Na]⁺, calculated 648.45 [M + Na] ⁺.

### 9b: HPLC (Gradient A) retention time= 27.3-27.6min

MS (ESI) m/z: found Rt 15.67 min. (Method LCMS), 648.51 [M + Na]⁺, calculated 648.45 [M + Na] ⁺.

### Synthesis of 2-(3-((R)-3-(3,4-dimethoxyphenyl)-1-((S)-1-(2-((1R,2R)-2-ethyl-1-hydroxy cyclohexyl)-2-oxoacetyl) piperidine-2-carbonyloxy) propyl) phenoxy) acetic acid

To (S)-((R)-1-(3-(2-tert-butoxy-2-oxoethoxy)phenyl)-3-(3,4-dimethoxyphenyl)-propyl) piperidine-2-carboxylate (21.4mg, 0.041 mmol) was added DIPEA (15.1 mg, 0.117 mmol), HATU (23.7 mg, 0.063 mmol) and 2-((1R,2R)-2-ethyl-1-hydroxycyclohexyl)-2-oxoacetic acid (10.0 mg, 0.05 mmol) and the reaction was followed as described above. The residue obtained was purified by column chromatography using Hexane : EtOAc 6:4 to yield the protected material (6.6 mg, 0.009 mmol, 22%).
TLC (Hexane: EtOAc 6: 4): Rf = 0.46.
HPLC (Gradient A) retention time= 31.5-31.9 min
MS (ESI) m/z 662.55 [M - tBu + Na] ⁺, calculated 662.54 [M - tBu + Na] ⁺.

The protected material (6.6 mg, 0.009 mmol) was treated with 20% TFA in DCM at rt. The mixture was allowed to stir for 6h. TFA and DCM was evaporated under reduced pressure to yield the free acid 2-(3-((R)-3-(3,4-dimethoxyphenyl)-1-((S)-1-(2-((1R,2R)-2-ethyl-1-hydroxy cyclohexyl)-2-oxoacetyl)piperidine-2-carbonyloxy)propyl) phenoxy)acetic acid (5.7mg, 0.007mmol, 91 %).
TLC (Hexane: EtOAc: TFA 1:1: 0.1): Rf = 0.35.
HPLC (Gradient A) retention time= 25.6-26.1 min
MS (ESI) m/z: found Rt 14.29min. (Method LCMS), 662.55 [M + Na]⁺,
HRMS 640.3063 [M + H] ⁺, calculated 640.3043 [M + H] ⁺.

### Example 6

### Synthesis of the heterocyclic precursors of the general formula (I):

### Synthesis of (S)-methyl 1-(3,3-dimethyl-2-oxopentanoyl)piperidine-2-carboxylate:

The compound was prepared as described previously (Holt et al. / J. Am. Chem. SOC., Vol. 115, No. 22, 1993).

### Synthesis of (S)-methyl 1-(3,3-dimethyl-2-oxopentanoyl)pyrrolidine-2-carboxylate:

Prepared from the methyl ester of L-Proline in an analogous manner to the synthesis of (S)-methyl 1-(3,3-dimethyl-2-oxopentanoyl)piperidine-2-carboxylate.

### Final coupling step in the synthesis of compounds according to the general formula (I)

The method is in accordance with the methods described in Example 4. The following compounds have been synthesized by this strategy:
2-(3-((R)-3-(3,4-dimethoxyphenyl)-1-((S)-1-(3,3-dimethyl-2-oxopentanoyl) piperidine-2-carbonyloxy)propyl)phenoxy)acetic acid
has been published in: Kozany et al., ChemBioChem 10, 8, 2009, 1402-1410; and T. Keenan et al. Bioorg. Med. Chem. 1998, 6, 1309-1335.
2-(3-((R)-3-(3,4-dimethoxyphenyl)-1-((S)-1-(3,3-dimethyl-2-oxopentanoyl)py - rrolidine-2-carbonyloxy)propyl)phenoxy)acetic acid
   TLC (Hexane: EtOAc: MeOH: AcOH: 6:3: 0.5: 0.5): Rf = 0.25.
   HPLC (Gradient A) retention time= 24.8-25.2min
   ¹H NMR (600 MHz, CDCl₃) δ= 0.79 (t, 3H, J= 7.2Hz), 1.14 (s, 3H), 1.16 (s, 3H), 1.59-1.70 (s, 2H), 1.89-2.05 (m, 4H), 2.15-2.24 (m, 2H), 2.48-2.60 (m, 2H), 3.44-3.70 (m, 2H), 3.80 (s, 3H), 3.82 (s, 3H), 4.55 (s, 2H), 4.58-4.61 (m, 1H), 5.66-5.72 (m, 1H), 6.62-6.66 (m, 2H), 6.74 (d, 1H, J= 8.4Hz), 6.78-6.81 (m, 1H), 6.85- 6.89 (m, 2H), 7.18-7.21 (m, 1H).
   ¹³C NMR (150 MHz, CDCl₃) δ= 9.05, 23.20, 23.76, 24.99, 29.89, 31.36, 32.33, 38.26, 47.05, 47.46, 56.04, 58.78, 65.74, 76.39, 111.52, 112.03, 112.56, 114.58, 119.63, 120.39, 129.86, 133.76, 141.91, 147.48, 149.02, 158.05, 163.74, 165.53, 170.83, 207.13.
   MS (ESI) m/z: found Rt 11.52 min. (Method LCMS), 592.32 [M + Na]⁺.
   HRMS 570.3268[M + H]⁺, calculated 570.3225 [M + H] ⁺.
2-(3-((R)-3-(3,4-dimethoxyphenyl)-1-((S)-1-(3,3-dimethyl-2-oxopentanoyl)-1,2,3,6-tetrahydropyridine-2-carbonyloxy)propyl)phenoxy)acetic acid
   TLC (Hexane: EtOAc: AcOH : 5:5: 0.5): Rf = 0.35.
   HPLC (Gradient A) retention time= 25.4-25.9min
   ¹H NMR (300 MHz, CDCl₃) δ= 0.89 (t, 3H, J= 7.5 Hz), 1.22 (s, 3H), 1.23 (s, 3H), 1.67-1.75 (m, 3H), 1.99-2.29 (m, 2H), 2.51-2.64 (m, 3H), 3.73 (t, 1H, J= 18.8 Hz), 3.85 (s, 3H), 3.86 (s, 3H), 3.97 (d, 1H, J= 18.7 Hz), 4.37-4.38 (m, 1H), 4.68 (s, 2H), 5.50-5.89 (m, 3H), 6.66 (s, 1H), 6.69 (s, 1H), 6.77-6.81 (m, 1H), 6.83- 6.94 (m, 3H), 7.17-7.24 (m, 1H).
   ¹³C NMR (75 MHz, CDCl₃) δ= 9.06, 23.56, 23.66, 27.38, 29.37, 31.44, 32.73, 38.31, 43.26, 54.75, 56.10, 56.17, 65.36, 76.88, 111.64, 112.06, 112.82, 114.92, 120.48, 122.57, 123.98, 125.53, 130.07, 133.61, 141.88, 147.63, 149.14, 158.07, 167.34, 169.54, 172.76, 207.48.
   MS (ESI) m/z: found Rt 11.12 min. (Method LCMS), 604.92 [M + Na]⁺.
   HRMS 582.3266 [M + H]⁺, calculated 582.3225 [M + H] ⁺.

(S)-((R)-3-(3,4-dimethoxyphenyl) -1-(3-(2-morpholinoethoxy)phenyl) propyl) 1-(3,3-dimethyl-2-oxopentanoyl)piperidine-2-carboxylate
TLC (DCM : MeOH 9.7:0.3): Rf = 0.62.
HPLC (Gradient A) retention time= 18.87-19.32 min
¹H NMR (400 MHz, CDCl₃) δ= 0.87 (t, 3H, J= 7.6 Hz), 1.19 (s, 3H), 1.21 (s, 3H), 1.56-1.76 (m, 7H), 1.99-2.08 (m, 1H), 2.19-2.37 (m, 2H), 2.47-2.59 (m, 6H), 2.80 (t, 2H, J= 5.2Hz), 3.14 (dt,, 1H, J= 3.2, 13.2 Hz), 3.34 (d, 1H, J= 13.2 Hz), 3.73 (t, 4H, J= 4.4 Hz). 3.84 (s, 3H), 3.85 (s, 3H), 4.11, (t, 2H, J= 5.6 Hz), 5.30 (d, 1H, J= 5.2 Hz), 5.75 (q, 1H, J= 2.4, 5.6 Hz), 6.65-6.68 (m, 2H), 6.75-6.78 (m, 1H), 6.81-6.84 (m, 1H), 6.87-6.92 (m, 2H), 7.22-7.26 (m, 1H).
¹³C NMR (100 MHz, CDCl₃) δ= 8.74, 21.18, 23.09, 23.49, 24.94, 26.41, 31.24, 32.45, 38.01, 44.12, 46.66, 51.23, 54.04, 55.80, 55.89, 57.63, 65.68, 66.81, 77.02, 111.25, 111.69, 113.01, 114.20, 119.04, 120.11, 129.64, 133.43, 141.31, 147.29, 148.83, 158.82, 167.19, 169.65, 207.76.
MS (ESI) m/z: found Rt 10.90 min. (Method LCMS), 639.45 [M + H]⁺.
HRMS 639.3566 [M + H]⁺, calculated 639.3567 [M + H]⁺.

(S)-((R)-3-(3,4-dimethoxyphenyl)-1-(3-(2-morpholinoethoxy)phenyl)propyl) 1-(3,3-dimethyl-2-oxopentanoyl)pyrrolidine-2-carboxylate
TLC (DCM: MeOH 9.7:0.3): Rf = 0.38.
HPLC (Gradient A) retention time= 18.79-19.19 min
¹H NMR (400 MHz, CDCl₃) δ= 0.81-0.86 (m, 3H), 1.19-1.22 (m, 6H), 1.56-1.75 (m, 3H), 1.89-2.01 (m, 4H), 2.17-2.25 (m, 1 H), 2.45-2.61 (m, 6H), 2.79 (s, 2H), 3.45-3.54 (m, 2H), 3.72 (t, 4H, J= 4.8 Hz), 3.83 (s, 3H), 3.85 (s, 3H), 4.11 (t, 2H, J= 5.6 Hz), 4.56-4.66 (m, 1H), 5.65-5.77 (m, 1H), 6.64-6.66 (m, 1H), 6.67 (s, 1H), 6.75-6.77 (m, 1H), 6.79-6.84 (m, 1H), 6.88-6.90 (m, 2H), 7.20-7.25 (m, 1H).
¹³C NMR (100 MHz, CDCl₃) δ= 8.92, 23.23, 23.59, 24.93, 31.07, 32.20, 33.93, 38.04, 46.86, 47.14, 49.06, 54.04, 55.81, 57.63, 65.67, 66.85, 76.19, 111.21, 111.79, 112.70, 114.08, 118.75, 120.11, 129.50, 133.63, 141.48, 147.21, 148.78, 158.78, 165.04, 170.62, 207.01.
MS (ESI) m/z: found Rt 10.59 min. (Method LCMS), 625.47 [M + H]⁺,
HRMS 625.3421 [M + H]⁺ calculated 625.3411 [M + H]⁺.

3,3-dimethyl-2-oxopentanamide (1R)-1-(3-aminophenyl) -3-(3,4-dimethoxyphenyl) propyl cyclohexanecarboxylate

Obtained from commercialsource (Cayman Chemicals, Compound name: SLF, Cat no. 1000974).

2-(3-((R)-3-(3,4-dimethoxyphenyl)-1-((S)-1-(2-oxo-2-(3,4,5-trimethoxyphen yl)acetyl)piperidine-2-carbonyloxy)propyl)phenoxy)acetic acid

To a stirred solution of (S)-((R)-1-(3-(2-tert-butoxy-2-oxoethoxy)phenyl)-3-(3,4-dimethoxyphenyl)-propyl) piperidine-2-carboxylate (51 mg, 0.1mmol) in DCM under argon was added sequentially N,N-Diisopropyl-ethylamine (DIPEA) (15.7mg, 0.125mmol) to which was added 2-oxo-2-(3,4,5-trimethoxyphenyl)acetyl chloride (26mg, 0.1mmol). The reaction mixture was stirred for 1 h at room temperature. Saturated NH₄Cl solution was added to the reaction and the solution was stirred for 10 min. The organic phase was separated and the aqueous phase was extracted with ethyl acetate (3x 100ml). The combined organic phases were washed with brine (10ml), dried over MgSO₄ and the residual solid was purified by column chromatography using Hexane: EtOAc 4:1 to afford the protected product (35.8mg, 0.048mmol, 49%).

TLC (Hexane: EtOAc 4:1): Rf = 0.45.
HPLC (Gradient A) retention time= 18.92-19.16min
¹H NMR (600 MHz, CDCl₃) δ= 1.48 (s, 9H), 1.58-1.66 (m, 1H), 1.78-1.85 (m, 3H), 2.06-2.12 (m, 1H), 2.24-2.30 (m, 2H), 2.44 (d, 1H, J= 12.6 Hz), 2.52-2.57 (m, 1H), 2.59-2.64 (m, 1H), 3.27 (t, 1H, J= 12.6 Hz), 3.48 (d, 1H, J= 12.6Hz), 3.82 (s, 6H), 3.85 (s, 6H), 3.92 (s, 3H), 4.52 (s, 2H), 5.41 (d, 1H, J= 4.8Hz), 5.73 (t, 1H, J= 6.6Hz), 6.63-6.70 (m, 3H), 6.76-6.81 (m, 2H), 6.90 (s, 1H), 6.95 (d, 1H, J= 7.8 Hz), 7.09 (s, 1H), 7.28-7.35 (m, 1H).

The protected material (35.8mg, 0.048mmol) was treated with 20% TFA in DCM at room temperature. The mixture was stirred for 6h. TFA and DCM were evaporated under reduced pressure to yield 2-{3-[(1R)-3-(3,4-dimethoxyphenyl)-1-({1-[2-oxo-2-(3,4,5-trimethoxyphenyl)acetyl]piperidin-2-yl}carbonyloxy)propyl]phenoxy}acetic acid (30.9mg, 0.045mmol, 96%).

TLC (Hexane: EtOAc: AcOH 5:5: 0.5): Rf = 0.37.
HPLC (Gradient A) retention time= 23.8-24.6min
¹H NMR (600 MHz, CDCl₃) δ= 1.50-1.56 (m, 1H), 1.61-1.66(m, 1H), 1.83-1.91 (m, 2H), 1.98-2.10 (m, 2H), 2.22-2.28 (m, 1H), 2.42 (d, 1H, J= 13.8 Hz), 2.56-2.61 (m, 1H), 2.64-2.69 (m, 1H), 3.25-3.30 (m, 1H), 3.45 (d, 1H, J= 12 Hz), 3.71 (s, 6H), 3.84 (s, 3H), 3.85 (s, 3H), 3.88 (s, 3H), 4.58 (s, 2H), 5.40 (d, 1H, J= 5.4 Hz), 5.56-5.66 (m, 1H), 6.66-6.67 (m, 2H), 6.75-6.78 (m, 1H), 6.82 (d, 1H, J= 7.8 Hz), 6.87-6.88 (m, 2H), 7.16 (s, 2H), 7.22 (t, 1H, j= 7.8Hz).
¹³C NMR 150 MHz, CDCl₃) δ= 24.84, 28.41, 28.44, 31.65, 38.45, 44.56, 52.17, 56.06, 56.14, 56.39, 61.14, 77.40, 107.09, 111.43, 111.58, 111.89, 114.92, 119.85, 120.41, 127.87, 129.85, 133.48, 142.25, 144.19, 147.64, 149.15, 153.63, 158.17, 168.36, 170.23, 177.39, 190.70
MS (ESI) m/z: found Rt 13.29 min. (Method LCMS), 702.22 [M + Na]⁺.
HRMS 680.3277 [M + H]⁺, calculated 680.3129 [M + H]⁺.

### Example 7

### Generation of biological data: Fluorescence polarization assay

The binding affinites for FKBPs were dermined as described in Kozany et al., ChemBioChem 10, 8, 2009, 1402-1410 using tracer 2b.

**Table 1: Chemical compounds according to formula (I) used in fluorescence polarization assay: Binding affinity to FKBP51 (FK1 domain) and FKBP52 (FK1 domain)**

| **Compd. No.** | **Structure** | **FKBP51 FK1** | **FKBP52FK1** |
|---|---|---|---|
| | | **IC₅₀(µM)** | |
| **Ref(**)** | 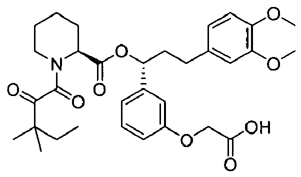 | 8.36 ± 0.98 | 10.5 ± 1.5 |
| **1*** | 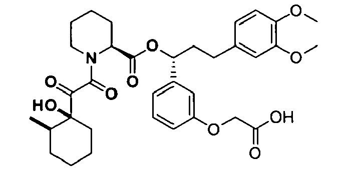 | 4.20 ± 0.11 | 2.13 ± 0.21 |
| **2*** | 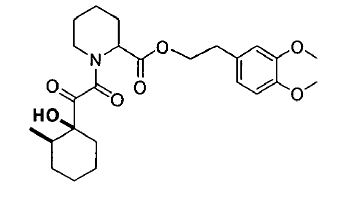 | >100 | >100 |
| **3*** | 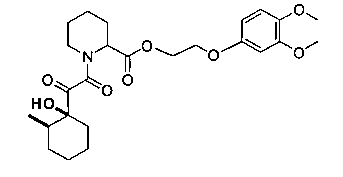 | 29.39 ± 8.5 | 11.7 ± 6.4 |
| **4*** | 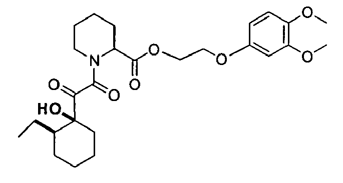 | >100 | >100 |
| **5*** | 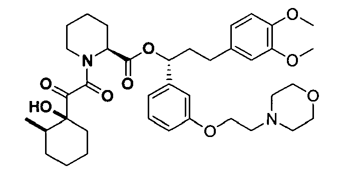 | 2.02 ± 0.14 | 0.89 ± 0.06 |
| **6*** | 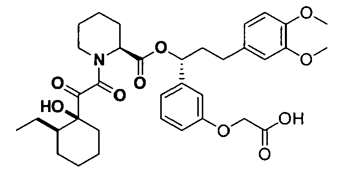 | 3.9 ± 1.2 | 9.5 ± 1.3 |
| **6a** | 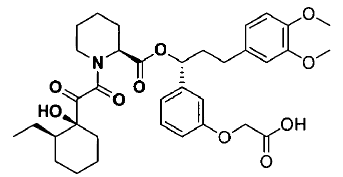 | 5.8 ± 0.6 | 4.2 ± 0.3 |
| **6b** | 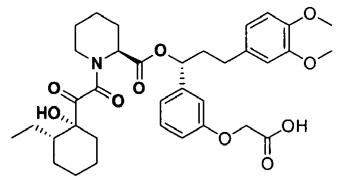 | 3.9 ± 0.6 | 3.5 ± 0.6 |
| **7*** | 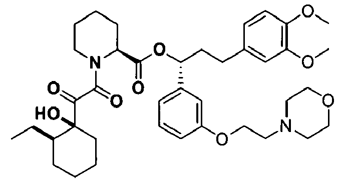 | 9.66 ± 0.83 | 3.72 ± 1.02 |
| **8** | 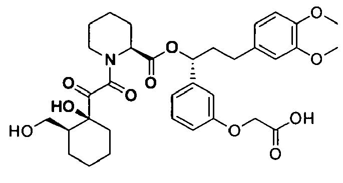 | 8.5 ± 0.6 | 6.2 ± 0.5 |
| **9*** | 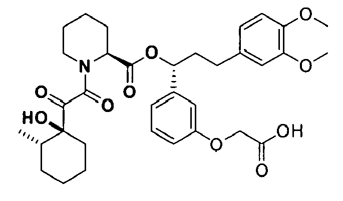 | 4.13 ± 0.20 | 2.64 ± 0.19 |
| **1a** | 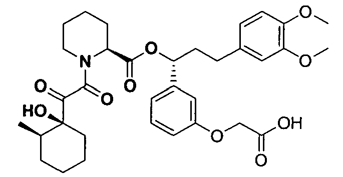 | 4.27 ± 0.19 | 2.44 ± 0.17 |
| **1b** | 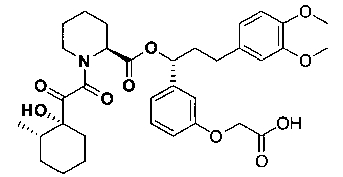 | 4.54 ± 0.24 | 2.88 ± 0.21 |
| **9a** | 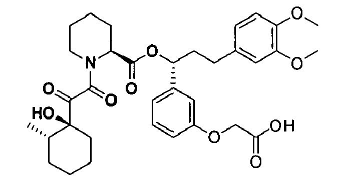 | 4.18 ± 0.15 | 2.20 ± 0.13 |
| **9b** | 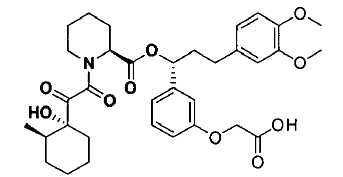 | 4.96 ± 0.25 | 2.64 ± 0.25 |
| **10*** | 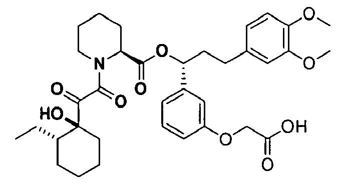 | 9.13 ± 0.59 | 9.77 ± 1.48 |
| **11** | 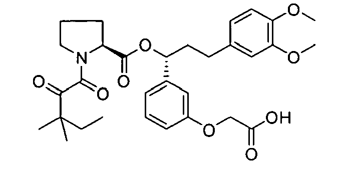 | 51.5 ± 31.9 | 41.6 ± 15.8 |
| **12** | 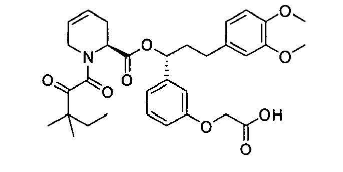 | 32.73 ± 12.3 | 49.2 ± 24.6 |
| **13** | 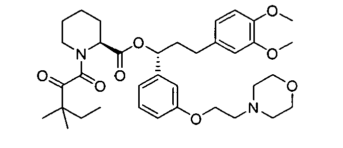 | 4.15 ± 1.45 | 2.8 ± 1.10 |
| **14** | 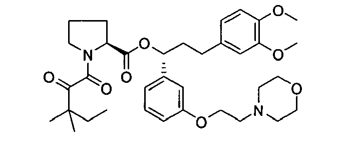 | 15.34 ± 1.94 | 5.55 ± 1.16 |
| **15** | 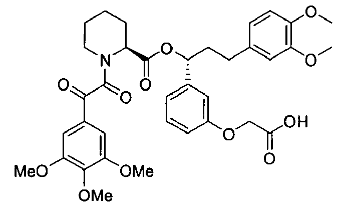 | 19.3 ± 6.6 | 11.6 ± 1.6 |

| | | | |
|---|---|---|---|
| * Mixture of Diasteromers. ** Kozany et al., ChemBioChem 10, 8, 2009, 1402-1410; T. Keenan et al. Bioorg. Med. Chem. 1998, 6, 1309-1335. | | | |

## Claims

1. Compound of the general formula (III) 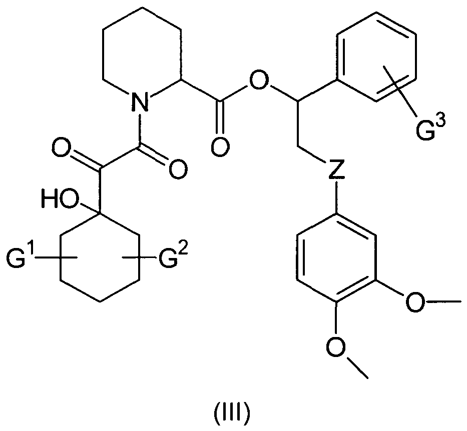 wherein
**Z** represents -CH₂-, -O-; **G¹-G³** represent independently of each other -**G^{C}**, -**G^{D}**, -H, -OH, -OCH₃, -OC₂H₅, -OC₃H₇, -O-cyclo-C₃H₅, -CH₂-OH, -OCH(CH₃)₂, -OC(CH₃)₃, -OC₄H₉, -OCH₂-COOH, -O-(CH₂)ₙ-COO**G^{C}**, -OPh, -OCH₂-Ph, -OCPh₃, -SH, -SCH₃, -SC₂H₅, -SC₃H₇, -S-cyclo-C₃H₅, -SCH(CH₃)₂, -SC(CH₃)₃, -NO₂, -F, -Cl, -Br, -I, -P(O)(OH)₂, -P(O)(OCH₃)₂, -P(O)(OC₂H₅)₂, -P(O)(OCH(CH₃)₂)₂, -C(OH)[P(O)(OH)₂]₂, -Si(CH₃)₂(C(CH₃)₃), -Si(C₂H₅)₃, -Si(CH₃)₃, -N₃, -CN, -OCN, -NCO, -SCN, -NCS, -CHO, -COCH₃, -COC₂H₅, -COC₃H₇, -CO-cyclo-C₃H₅, -COCH(CH₃)₂, -COC(CH₃)₃, -COOH, -COCN, -COOCH₃, -COOC₂H₅, -COOC₃H₇, -COO-cyclo-C₃H₅, -COOCH(CH₃)₂, -COOC(CH₃)₃, -OOC-CH₃, -OOC-C₂H₅, -OOC-C₃H₇, -OOC-cyclo-C₃H₅, -OOC-CH(CH₃)₂, -OOC-C(CH₃)₃, -CONH₂, -CONHCH₃, -CONHC₂H₅, -CONHC₃H₇, -CONH-cyclo-C₃H₅, -CONH[CH(CH₃)₂], -CONH[C(CH₃)₃], -CON(CH₃)₂, -CON(C₂H₅)₂, -CON(C₃H₇)₂, -CON(cyclo-C₃H₅)₂, -CON[CH(CH₃)₂]₂, -CON[C(CH₃)₃]₂, -NHCOCH₃, -NHCOC₂H₅, -NHCOC₃H₇, -NHCO-cyclo-C₃H₅, -NHCO-CH(CH₃)₂, -NHCO-C(CH₃)₃, -NHCO-OCH₃, -NHCO-OC₂H₅, -NHCO-OC₃H₇, -NHCO-O-cyclo-C₃H₅, -NHCO-OCH(CH₃)₂, -NHCO-OC(CH₃)₃, -NH₂, -NHCH₃, -NHC₂H₅, -NHC₃H₇, -NH-cyclo-C₃H₅, -NHCH(CH₃)₂, -NHC(CH₃)₃, -N(CH₃)₂, -N(C₂H₅)₂, -N(C₃H₇)₂, -N(cyclo-C₃H₅)₂, -N[CH(CH₃)₂]₂, -N[C(CH₃)₃]₂, -SOCH₃, -SOC₂H₅, -SOC₃H₇, -SO-cyclo-C₃H₅, -SOCH(CH₃)₂, -SOC(CH₃)3, -SO₂CH₃, -SO₂C₂H₅, -SO₂C₃H₇, -SO₂-cyclo-C₃H₅, -SO₂CH(CH₃)₂, -SO₂C(CH₃)₃, -SO₃H, -SO₃CH₃, -SO₃C₂H₅, -SO₃C₃H₇, -SO₃-cyclo-C₃H₅, -SO₃CH(CH₃)₂, -SO₃C(CH₃)₃, -SO₂NH₂, -SO₂NHCH₃, -SO₂NHC₂H₅, -SO₂NHC₃H₇, -SO₂NH-cyclo-C₃H₅, -SO₂NHCH(CH₃)₂, -SO₂NHC(CH₃)₃, -SO₂N(CH₃)₂, -SO₂N(C₂H₅)₂, -SO₂N(C₃H₇)₂, -SO₂N(cyclo-C₃H₅)₂, -SO₂N[CH(CH₃)₂]₂, -SO₂N[C(CH₃)₃]₂, -O-S(=O)CH₃, -O-S(=O)C₂H₅, -O-S(=O)C₃H₇, -O-S(=O)-cyclo-C₃H₅, -O-S(=O)CH(CH₃)₂, -O-S(=O)C(CH₃)₃, -S(=O)(=NH)CH₃, -S(=O)(=NH)C₂H₅, -S(=O)(=NH)C₃H₇, -S(=O)(=NH)-cyclo-C₃H₅, -S(=O)(=NH)CH(CH₃)₂, -S(=O)(=NH)C(CH₃)₃, -NH-SO₂-CH₃, -NH-SO₂-C₂H₅, -NH-SO₂-C₃H₇, -NH-SO₂-cyclo-C₃H₅, -NH-SO₂-CH(CH₃)₂, -NH-SO₂-C(CH₃)₃, -O-SO₂-CH₃, -O-SO₂-C₂H₅, -O-SO₂-C₃H₇, -O-SO₂-cyclo-C₃H₅, -O-SO₂-CH(CH₃)₂, -O-SO₂-C(CH₃)₃, -OCF₃, -CH₂-OCF₃, -C₂H₄-OCF₃, -C₃H₆-OCF₃, -OC₂F₅, -CH₂-OC₂F₅, -C₂H₄-OC₂F₅, -C₃H₆-OC₂F₅, -O-COOCH₃, -O-COOC₂H₅, -O-COOC₃H₇, -O-COO-cyclo-C₃H₅, -O-COOCH(CH₃)₂, -O-COOC(CH₃)₃, -NH-CO-NH₂, -NH-CO-NHCH₃, -NH-CO-NHC₂H₅, -NH-CS-N(C₃H₇)₂, -NH-CO-NHC₃H₇, -NH-CO-N(C₃H₇)₂, -NH-CO-NH[CH(CH₃)₂], -NH-CO-NH[C(CH₃)₃], -NH-CO-N(CH₃)₂, -NH-CO-N(C₂H₅)₂, -NH-CO-NH-cyclo-C₃H₅, -NH-CO-N(cyclo-C₃H₅)₂, -NH-CO-N[CH(CH₃)₂]₂, -NH-CS-N(C₂H₅)₂, -NH-CO-N[C(CH₃)₃]₂, -NH-CS-NH₂, -NH-CS-NHCH₃, -NH-CS-N(CH₃)₂, -NH-CS-NHC₂H₅, -NH-CS-NHC₃H₇, -NH-CS-NH-cyclo-C₃H₅, -NH-CS-NH[CH(CH₃)₂], -NH-CS-NH[C(CH₃)₃], -NH-CS-N(cyclo-C₃H₅)₂, -NH-CS-N[CH(CH₃)₂]₂, -NH-CS-N[C(CH₃)₃]₂, -NH-C(=NH)-NH₂, -NH-C(=NH)-NHCH₃, -NH-C(=NH)-NHC₂H₅, -NH-C(=NH)-NHC₃H₇, -O-CO-NH-cyclo-C₃H₅, -NH-C(=NH)-NH-cyclo-C₃H₅, -NH-C(=NH)-NH[CH(CH₃)₂], -O-CO-NH[CH(CH₃)₂], -NH-C(=NH)-NH[C(CH₃)₃], -NH-C(=NH)-N(CH₃)₂, -NH-C(=NH)-N(C₂H₅)₂, -NH-C(=NH)-N(C₃H₇)₂, -NH-C(=NH)-N(cycylo-C₃H₅)₂, -O-CO-NHC₃H₇, -NH-C(=NH)-N[CH(CH₃)₂]₂, -NH-C(=NH)-N[C(CH₃)₃]₂, -O-CO-NH₂, -O-CO-NHCH₃, -O-CO-NHC₂H₅, -O-CO-NH[C(CH₃)₃], -O-CO-N(CH₃)₂, -O-CO-N(C₂H₅)₂, -O-CO-N(C₃H₇)₂, -O-CO-N(cyclo-C₃H₅)₂, -O-CO-N[CH(CH₃)₂]₂, -O-CO-N[C(CH₃)₃]₂, -O-CO-OCH₃, -O-CO-OC₂H₅, -O-CO-OC₃H₇, -O-CO-O-cyclo-C₃H₅, -O-CO-OCH(CH₃)₂, -O-CO-OC(CH₃)₃, 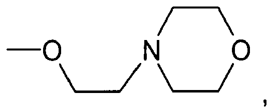 **G^{C}** and **G^{D}** represent independently of each other -CH₂-OCH₃, -C₂H₄-OCH₃, -C₃H₆-OCH₃, -CH₂-OC₂H₅, -C₂H₄-OC₂H₅, -C₃H₆-OC₂H₅, -CH₂-OC₃H₇, -C₂H₄-OC₃H₇, -C₃H₆-OC₃H₇, -CH₂-O-cyclo-C₃H₅, -C₂H₄-O-cyclo-C₃H₅, -C₃H₆-O-cyclo-C₃H₅, -CH₂-OCH(CH₃)₂, -C₂H₄-OCH(CH₃)₂, -C₃H₆-OCH(CH₃)₂, -CH₂-OC(CH₃)₃, -C₂H₄-OC(CH₃)₃, -C₃H₆-OC(CH₃)₃, -CH₂-OC₄H₉, -C₂H₄-OC₄H₉, -C₃H₆-OC₄H₉, -CH₂-OPh, -C₂H₄-OPh, -C₃H₆-OPh, -CH₂-OCH₂-Ph, -C₂H₄-OCH₂-Ph, -C₃H₆-OCH₂-Ph, -CH₂F, -CHF₂, -CF₃, -CH₂Cl, -CH₂Br, -CH₂I, -CH₂-CH₂F, -CH₂-CHF₂, -CH₂-CF₃, -CH₂-CH₂Cl, -CH₂-CH₂Br, -CH₂-CH₂I, -cyclo-C₈H₁₅, -Ph, -CH₂-Ph, -CH₂-CH₂-Ph, -CH=CH-Ph, -CPh₃, -CH₃, -C₂H₅, -C₃H₇, -CH(CH₃)₂, -C₄H₉, -CH₂-CH(CH₃)₂, -CH(CH₃)-C₂H₅, -C(CH₃)₃, -C₅H₁₁, -CH(CH₃)-C₃H₇, -CH₂-CH(CH₃)-C₂H₅, -CH(CH₃)-CH(CH₃)₂, -C(CH₃)₂-C₂H₅, -CH₂-C(CH₃)₃, -CH(C₂H₅)₂, -C₂H₄-CH(CH₃)₂, -C₆H₁₃, -C₇H₁₅, -C₈H₁₇, -C₃H₆-CH(CH₃)₂, -C₂H₄-CH(CH₃)-C₂H₅, -CH(CH₃)-C₄H₉, -CH₂-CH(CH₃)-C₃H₇, -CH(CH₃)-CH₂-CH(CH₃)₂, -CH(CH₃)-CH(CH₃)-C₂H₅, -CH₂-CH(CH₃)-CH(CH₃)₂, -CH₂-C(CH₃)₂-C₂H₅, -C(CH₃)₂-C₃H₇, -C(CH₃)₂-CH(CH₃)₂, -C₂H₄-C(CH₃)₃, -CH(CH₃)-C(CH₃)₃, -CH=CH₂, -CH₂-CH=CH₂, -C(CH₃)=CH₂, -CH=CH-CH₃, -C₂H₄-CH=CH₂, -CH₂-CH=CH-CH₃, -CH=CH-C₂H₅, -CH₂-C(CH₃)=CH₂, -CH(CH₃)-CH=CH, -CH=C(CH₃)₂, -C(CH₃)=CH-CH₃, -CH=CH-CH=CH₂, -C₃H₆-CH=CH₂, -C₂H₄-CH=CH-CH₃, -CH₂-CH=CH-C₂H₅, -CH=CH-C₃H₇, -CH₂-CH=CH-CH=CH₂, -CH=CH-CH=CH-CH₃, -CH=CH-CH₂-CH=CH₂, -C(CH₃)=CH-CH=CH₂, -CH=C(CH₃)-CH=CH₂, -CH=CH-C(CH₃)=CH₂, -C₂H₄-C(CH₃)=CH₂, -CH₂-CH(CH₃)-CH=CH₂, -CH(CH₃)-CH₂-CH=CH₂, -CH₂-CH=C(CH₃)₂, -CH₂-C(CH₃)=CH-CH₃, -CH(CH₃)-CH=CH-CH₃, -CH=CH-CH(CH₃)₂, -CH=C(CH₃)-C₂H₅, -C(CH₃)=CH-C₂H₅, -C(CH₃)=C(CH₃)₂, -C(CH₃)₂-CH=CH₂, -CH(CH₃)-C(CH₃)=CH₂, -C(CH₃)=CH-CH=CH₂, -CH=C(CH₃)-CH=CH₂, -CH=CH-C(CH₃)=CH₂, -C₄H₈-CH=CH₂, -C₃H₆-CH=CH-CH₃, -C₂H₄-CH=CH-C₂H₅, -CH₂-CH=CH-C₃H₇, -CH=CH-C₄H₉, -C₃H₆-C(CH₃)=CH₂, -C₂H₄-CH(CH₃)-CH=CH₂, -CH₂-CH(CH₃)-CH₂-CH=CH₂, -C₂H₄-CH=C(CH₃)₂, -CH(CH₃)-C₂H₄-CH=CH₂, -C₂H₄-C(CH₃)=CH-CH₃, -CH₂-CH(CH₃)-CH=CH-CH₃, -CH(CH₃)-CH₂-CH=CH-CH₃, -CH₂-CH=CH-CH(CH₃)₂, -CH₂-CH=C(CH₃)-C₂H₅, -CH₂-C(CH₃)=CH-C₂H₅, -CH(CH₃)-CH=CH-C₂H₅, -CH=CH-CH₂-CH(CH₃)₂, -CH=CH-CH(CH₃)-C₂H₅, -CH=C(CH₃)-C₃H₇, -C(CH₃)=CH-C₃H₇, -CH₂-CH(CH₃)-C(CH₃)=CH₂, -C[C(CH₃)₃]=CH₂, -CH(CH₃)-CH₂-C(CH₃)=CH₂. -CH(CH₃)-CH(CH₃)-CH=CH₂, -CH=CH-C₂H₄-CH=CH₂, -CH₂-C(CH₃)₂-CH=CH₂, -C(CH₃)₂-CH₂-CH=CH₂, -CH₂-C(CH₃)=C(CH₃)₂, -CH(CH₃)-CH=C(CH₃)₂, -C(CH₃)₂-CH=CH-CH₃, -CH=CH-CH₂-CH=CH-CH₃, -CH(CH₃)-C(CH₃)=CH-CH₃, -CH=C(CH₃)-CH(CH₃)₂, -C(CH₃)=CH-CH(CH₃)₂, -C(CH₃)=C(CH₃)-C₂H₅, -CH=CH-C(CH₃)₃, -C(CH₃)₂-C(CH₃)=CH₂, -CH(C₂H₅)-C(CH₃)=CH₂, -C(CH₃)(C₂H₅)-CH=CH₂, -CH(CH₃)-C(C₂H₅)=CH₂, -CH₂-C(C₃H₇)=CH₂, -CH₂-C(C₂H₅)=CH-CH₃, -CH(C₂H₅)-CH=CH-CH₃, -C(C₄H₉)=CH₂, -C(C₃H₇)=CH-CH₃, -C(C₂H₅)=CH-C₂H₅, -C(C₂H₅)=C(CH₃)₂, -C[CH(CH₃)(C₂H₅)]=CH₂, -C[CH₂-CH(CH₃)₂]=CH₂, -C₂H₄-CH=CH-CH=CH₂, -CH₂-CH=CH-CH₂-CH=CH₂, -C₃H₆-C≡C-CH₃, -CH₂-CH=CH-CH=CH-CH₃, -CH=CH-CH=CH-C₂H₅, -CH₂-CH=CH-C(CH₃)=CH₂, -CH₂-CH=C(CH₃)-CH=CH₂, -CH₂-C(CH₃)=CH-CH=CH₂, -CH(CH₃)-CH₂-C=CH, -CH(CH₃)-CH=CH-CH=CH₂, -CH=CH-CH₂-C(CH₃)=CH₂, -CH(CH₃)-C≡C-CH₃, -CH=CH-CH(CH₃)-CH=CH₂, -CH=C(CH₃)-CH₂-CH=CH₂, -C₂H₄-CH(CH₃)-C≡CH, -C(CH₃)=CH-CH₂-CH=CH₂, -CH=CH-CH=C(CH₃)₂, -CH₂-CH(CH₃)-CH₂-C≡CH, -CH=CH-C(CH₃)=CH-CH₃, -CH=C(CH₃)-CH=CH-CH₃, -CH₂-CH(CH₃)-C≡CH, -C(CH₃)=CH-CH=CH-CH₃, -CH=C(CH₃)-C(CH₃)=CH₂, -C(CH₃)=CH-C(CH₃)=CH₂, -C(CH₃)=C(CH₃)-CH=CH₂, -CH=CH-CH=CH-CH=CH₂, -C≡CH, -C≡C-CH₃, -CH₂-C≡CH, -C₂H₄-C≡CH, -CH₂-C≡C-CH₃, -C≡C-C₂H₅, -C₃H₆-C≡CH, -C₂H₄-C≡C-CH₃, -CH₂-C≡C-C₂H₅, -C≡C-C₃H₇, -CH(CH₃)-C≡CH, -C₄H₈-C≡CH, -C₂H₄-C≡C-C₂H₅, -CH₂-C≡C-C₃H₇, -C≡C-C₄H₉, -C≡C-C(CH₃)₃, -CH(CH₃)-C₂H₄-C≡CH, -CH₂-CH(CH₃)-C≡C-CH₃, -CH(CH₃)-CH₂-C≡C-CH₃, -CH(CH₃)-C≡C-C₂H₅, -CH₂-C≡C-CH(CH₃)₂, -C≡C-CH(CH₃)-C₂H₅, -C≡C-CH₂-CH(CH₃)₂, -CH(C₂H₅)-C≡C-CH₃, -C(CH₃)₂-C≡C-CH₃, -CH(C₂H₅)-CH₂-C≡CH, -CH₂-CH(C₂H₅)-C≡CH, -C(CH₃)₂-CH₂-C≡CH, -CH₂-C(CH₃)₂-C≡CH, -CH(CH₃)-CH(CH₃)-C≡CH, -CH(C₃H₇)-C≡CH, -C(CH₃)(C₂H₅)-C≡CH, -CH₂-CH(C≡CH)₂, -C≡C-C≡CH, -CH₂-C≡C-C≡CH, -C≡C-C≡C-CH₃, -CH(C≡CH)₂, -C₂H₄-C≡C-C≡CH, -CH₂-C≡C-CH₂-C≡CH, -C≡C-C₂H₄-C≡CH, -CH₂-C≡C-C≡C-CH₃, -C≡C-CH₂-C≡C-CH₃, -C≡C-C≡C-C₂H₅, -C(C≡CH)₂-CH₃, -C≡C-CH(CH₃)-C≡CH, -CH(CH₃)-C≡C-C≡CH, -CH(C≡CH)-CH₂-C≡CH, -CH(C≡CH)-C≡C-CH₃;
n is 1, 2, 3 or 4,
and enantiomers, stereoisomeric forms, mixtures of enantiomers, anomers, deoxy-forms, diastereomers, mixtures of diastereomers, prodrugs, tautomers, hydrates, solvates and racemates of the above mentioned compounds and pharmaceutically acceptable salts thereof.

2. Compound according to claim 1 for use as pharmaceutically active agent in medicine.

3. Compound according to claim 1 for the inhibition of FK506-binding proteins.

4. Compound according to claim 1 selected from the group consisting of:
2-(3-((R)-3-(3,4-dimethoxyphenyl)-1-((S)-1-(2-((1S,2R)-1-hydroxy-2-methyl-cyclohexyl)-2-oxoacetyl)piperidine-2-carbonyloxy)propyl)phenoxy)acetic acid
3,4-dimethoxyphenethyl 1-(2-((1 S,2R)-1-hydroxy-2-methylcyclohexyl)-2-oxo-acetyl)-piperidine-2-carboxylate
2-(3,4-dimethoxyphenoxy)ethyl 1-(2-((1S,2R)-1-hydroxy-2-methylcyclohexyl)-2-oxoacetyl)piperidine-2-carboxylate
2-(3,4-dimethoxyphenoxy)ethyl 1-(2-((1 S,2R)-2-ethyl-1-hydroxycyclohexyl)-2-oxoacetyl)piperidine-2-carboxylate
(S)-((R)-3-(3,4-dimethoxyphenyl)-1-(3-(2-morpholinoethoxy)phenyl)propyl) 1-(2-((1S, 2R)-1-hydroxy-2-methylcyclohexyl)-2-oxoacetyl)piperidine-2-carboxylate
2-(3-((R)-3-(3,4-dimethoxyphenyl)-1-((S)-1-(2-((1S,2R)-2-ethyl-1-hydroxy-cyclohexyl)-2-oxoacetyl)piperidine-2-carbonyloxy)propyl)phenoxy)acetic acid
(S)-((R)-3-(3,4-dimethoxyphenyl)-1-(3-(2-morpholinoethoxy)phenyl)propyl)1-(2-((1S, 2R) -2-ethyl-1-hydroxycyclohexyl)-2-oxoacetyl)piperidine-2-carboxylate
2-(3-((R)-3-(3,4-dimethoxyphenyl)-1-((S)-1-(2-((1S,2S)-1-hydroxy-2-(hydroxyl-methyl) cyclohexyl)-2-oxoacetyl)piperidine-2-carbonyloxy)propyl)phenoxy)acetic acid
2-(3-((R)-3-(3,4-dimethoxyphenyl)-1-((S)-1-(2-((1R,2R)-1-hydroxy-2-methylcyclohexyl)-2-oxoacetyl)piperidine-2-carbonyloxy)propyl)phenoxy)acetic acid
2-(3-((R) -3-(3,4-dimethoxyphenyl)-1-((S)-1-(2-((1R,2R)-2-ethyl-1-hydroxy cyclohexyl)-2-oxoacetyl)piperidine-2-carbonyloxy)propyl)phenoxy)acetic acid

5. Compound according to the general formula (I): 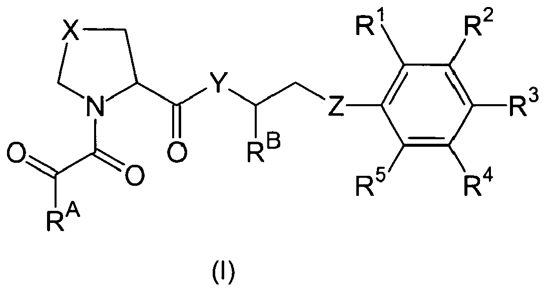 wherein
X represents -CH₂-, -CH₂-CH₂-, -CH=CH-, -CH₂-S-;
Y represents -NH-, -O-;
Z represents -CH₂-, -O-;
**R**^{A} **represents** -CH₃, -C₂H₅, -C₃H₇, -CH(CH₃)₂, -C₄H₉, -CH₂-CH(CH₃)₂, -CH(CH₃)-C₂H₅, -C(CH₃)₃, -C₅H₁₁, -CH(CH₃)-C₃H₇, -CH₂-CH(CH₃)-C₂H₅, -CH(CH₃)-CH(CH₃)₂, -C(CH₃)₂-C₂H₅, -CH₂-C(CH₃)₃, -CH(C₂H₅)₂, -C₂H₄-CH(CH₃)₂, -C₆H₁₃, -C₇H₁₅, -C₈H₁₇, -C₃H₆-CH(CH₃)₂, -C₂H₄-CH(CH₃)-C₂H₅, -CH(CH₃)-C₄H₉, -CH₂-CH(CH₃)-C₃H₇, -CH(CH₃)-CH₂-CH(CH₃)₂, -CH(CH₃)-CH(CH₃)-C₂H₅, -CH₂-CH(CH₃)-CH(CH₃)₂, -CH₂-C(CH₃)₂-C₂H₅, -C(CH₃)₂-C₃H₇, -C(CH₃)₂-CH(CH₃)₂, -C₂H₄-C(CH₃)₃, -CH(CH₃)-C(CH₃)₃, -CH=CH₂, -CH₂-CH=CH₂, -C(CH₃)=CH₂, -CH=CH-CH₃, -C₂H₄-CH=CH₂, -CH₂-CH=CH-CH₃, -CH=CH-C₂H₅, -CH₂-C(CH₃)=CH₂, -CH(CH₃)-CH=CH, -CH=C(CH₃)₂, -C(CH₃)=CH-CH₃, -CH=CH-CH=CH₂, -C₃H₆-CH=CH₂, -C₂H₄-CH=CH-CH₃, -CH₂-CH=CH-C₂H₅, -CH=CH-C₃H₇, -CH₂-CH=CH-CH=CH₂, -CH=CH-CH=CH-CH₃, -CH=CH-CH₂-CH=CH₂, -C(CH₃)=CH-CH=CH₂, -CH=C(CH₃)-CH=CH₂, -CH=CH-C(CH₃)=CH₂, -C₂H₄-C(CH₃)=CH₂, -CH₂-CH(CH₃)-CH=CH₂, -CH(CH₃)-CH₂-CH=CH₂, -CH₂-CH=C(CH₃)₂, -CH₂-C(CH₃)=CH-CH₃, -CH(CH₃}-CH=CH-CH₃, -CH=CH-CH(CH₃)₂, -CH=C(CH₃)-C₂H₅, -C(CH₃)=CH-C₂H₅, -C(CH₃)=C(CH₃)₂, -C(CH₃)₂-CH=CH₂, -CH(CH₃)-C(CH₃)=CH₂, -C(CH₃)=CH-CH=CH₂, -CH=C(CH₃)-CH=CH₂, -CH=CH-C(CH₃)=CH₂, -C₄H₈-CH=CH₂, -C₃H₆-CH=CH-CH₃, -C₂H₄-CH=CH-C₂H₅, -CH₂-CH=CH-C₃H₇, -CH=CH-C₄H₉, -C₃H₆-C(CH₃)=CH₂, -C₂H₄-CH(CH₃)-CH=CH₂, -CH₂-CH(CH₃)-CH₂-CH=CH₂, -C₂H₄-CH=C(CH₃)₂, -CH(CH₃)-C₂H₄-CH=CH₂, -C₂H₄-C(CH₃)=CH-CH₃, -CH₂-CH(CH₃)-CH=CH-CH₃, -CH(CH₃)-CH₂-CH=CH-CH₃, -CH₂-CH=CH-CH(CH₃)₂, -CH₂-CH=C(CH₃)-C₂H₅, -CH₂-C(CH₃)=CH-C₂H₅, -CH(CH₃)-CH=CH-C₂H₅, -CH=CH-CH₂-CH(CH₃)₂, -CH=CH-CH(CH₃)-C₂H₅, -CH=C(CH₃)-C₃H₇, -C(CH₃)=CH-C₃H₇, -CH₂-CH(CH₃)-C(CH₃)=CH₂, -C[C(CH₃)₃]=CH₂, -CH(CH₃)-CH₂-C(CH₃)=CH₂, -CH(CH₃)-CH(CH₃)-CH=CH₂, -CH=CH-C₂H₄-CH=CH₂, -CH₂-C(CH₃)₂-CH=CH₂, -C(CH₃)₂-CH₂-CH=CH₂, -CH₂-C(CH₃)=C(CH₃)₂, -CH(CH₃)-CH=C(CH₃)₂, -C(CH₃)₂-CH=CH-CH₃, -CH=CH-CH₂-CH=CH-CH₃, -CH(CH₃)-C(CH₃)=CH-CH₃, -CH=C(CH₃)-CH(CH₃)₂, -C(CH₃)=CH-CH(CH₃)₂, -C(CH₃)=C(CH₃)-C₂H₅, -CH=CH-C(CH₃)₃, -C(CH₃)₂-C(CH₃)=CH₂, -CH(C₂H₅)-C(CH₃)=CH₂, -C(CH₃)(C₂H₅)-CH=CH₂, -CH(CH₃)-C(C₂H₅)=CH₂, -CH₂-C(C₃H₇)=CH₂, -CH₂-C(C₂H₅)=CH-CH₃, -CH(C₂H₅)-CH=CH-CH₃, -C(C₄H₉)=CH₂, -C(C₃H₇)=CH-CH₃, -C(C₂H₅)=CH-C₂H₅, -C(C₂H₅)=C(CH₃)₂, -C[CH(CH₃)(C₂H₅)]=CH₂, -C[CH₂-CH(CH₃)₂]=CH₂, -C₂H₄-CH=CH-CH=CH₂, -CH₂-CH=CH-CH₂-CH=CH₂, -C₃H₆-C≡C-CH₃, -CH₂-CH=CH-CH=CH-CH₃, -CH=CH-CH=CH-C₂H₅, -CH₂-CH=CH-C(CH₃)=CH₂, -CH₂-CH=C(CH₃)-CH=CH₂, -CH₂-C(CH₃)=CH-CH=CH₂, -CH(CH₃)-CH₂-C≡CH, -CH(CH₃)-CH=CH-CH=CH₂, -CH=CH-CH₂-C(CH₃)=CH₂, -CH(CH₃)-C≡C-CH₃, -CH=CH-CH(CH₃)-CH=CH₂, -CH=C(CH₃)-CH₂-CH=CH₂, -C₂H₄-CH(CH₃)-C≡CH, -C(CH₃)=CH-CH₂-CH=CH₂, -CH=CH-CH=C(CH₃)₂, -CH₂-CH(CH₃)-CH₂-C≡CH, -CH=CH-C(CH₃)=CH-CH₃, -CH=C(CH₃)-CH=CH-CH₃, -CH₂-CH(CH₃)-C≡CH, -C(CH₃)=CH-CH=CH-CH₃, -CH=C(CH₃)-C(CH₃)=CH₂, -C(CH₃)=CH-C(CH₃)=CH₂, -C(CH₃)=C(CH₃)-CH=CH₂, -CH=CH-CH=CH-CH=CH₂, -C≡CH, -C≡C-CH₃, -CH₂-C≡CH, -C₂H₄-C≡CH, -CH₂-C≡C-CH₃, -C≡C-C₂H₅, -C₃H₆-C≡CH, -C₂H₄-C≡C-CH₃, -CH₂-C≡C-C₂H₅, -C≡C-C₃H₇, -CH(CH₃)-C≡CH, -C₄H₈-C≡CH, -C₂H₄-C≡C-C₂H₅, -CH₂-C≡C-C₃H₇, -C≡C-C₄H₉, -C≡C-C(CH₃)₃, -CH(CH₃)-C₂H₄-C≡CH, -CH₂-CH(CH₃)-C≡C-CH₃, -CH(CH₃)-CH₂-C≡C-CH₃, -CH(CH₃)-C≡C-C₂H₅, -CH₂-C≡C-CH(CH₃)₂, -C≡C-CH(CH₃)-C₂H₅, -C≡C-CH₂-CH(CH₃)₂, -CH(C₂H₅)-C≡C-CH₃, -C(CH₃)₂-C≡C-CH₃, -CH(C₂H₅)-CH₂-C≡CH, -CH₂-CH(C₂H₅)-C≡CH, -C(CH₃)₂-CH₂-C≡CH, -CH₂-C(CH₃)₂-C≡CH, -CH(CH₃)-CH(CH₃)-C≡CH, -CH(C₃H₇)-C=CH, -C(CH₃)(C₂H₅)-C≡CH, -CH₂-CH(C≡CH)₂, -C≡C-C≡CH, -CH₂-C≡C-C≡CH, -C≡C-C≡C-CH₃, -CH(C≡CH)₂, -C₂H₄-C≡C-C≡CH, -CH₂-C≡C-CH₂-C≡CH, -C≡C-C₂H₄-C≡CH, -CH₂-C≡C-C≡C-CH₃, -C≡C-CH₂-C≡C-CH₃, -C≡C-C≡C-C₂H₅, -C(C≡CH)₂-CH₃, -C≡C-CH(CH₃)-C≡CH, -CH(CH₃)-C≡C-C≡CH, -CH(C≡CH)-CH₂-C≡CH, -CH(C≡CH)-C≡C-CH₃, 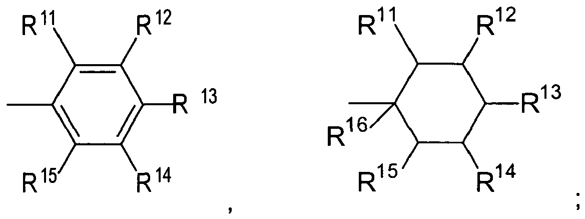 **R^{B}** represents the following -H or 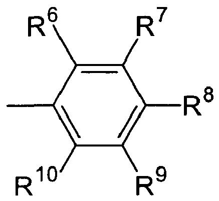 **R¹-R¹⁶** represent independently of each other -H, -OH, -OCH₃, -OC₂H₅, -OC₃H₇, -O-cyclo-C₃H₅, -OCH(CH₃)₂, -OC(CH₃)₃, -OC₄H₉, -OCH₂-COOH, -O-(CH₂)ₙ-COO**R^{C}** (with n = 1-4), -OPh, -OCH₂-Ph, -OCPh₃, -CH₂-OCH₃, -CH₂-OH, -C₂H₄-OCH₃, -C₃H₆-OCH₃, -CH₂-OC₂H₅, -C₂H₄-OC₂H₅, -C₃H₆-OC₂H₅, -CH₂-OC₃H₇, -C₂H₄-OC₃H₇, -C₃H₆-OC₃H₇, -CH₂-O-cyclo-C₃H₅, -C₂H₄-O-cyclo-C₃H₅, -C₃H₆-cyclo-C₃H₅, -CH₂-OCH(CH₃)₂, -C₂H₄-OCH(CH₃)₂, -C₃H₆-OCH(CH₃)₂, -CH₂-OC(CH₃)₃, -C₂H₄-OC(CH₃)₃, -C₃H₆-OC(CH₃)₃, -CH₂-OC₄H₉, -C₂H₄-OC₄H₉, -C₃H₆-OC₄H₉, -CH₂-OPh, -C₂H₄-OPh, -C₃H₆-OPh, -CH₂-OCH₂-Ph, -C₂H₄-OCH₂-Ph, -C₃H₆-OCH₂-Ph, -SH, -SCH₃, -SC₂H₅, -SC₃H₇, -S-cyclo-C₃H₅, -SCH(CH₃)₂, -SC(CH₃)₃, -NO₂, -F, -Cl, -Br, -I, -P(O)(OH)₂, -P(O)(OCH₃)₂, -P(O)(OC₂H₅)₂, -P(O)(OCH(CH₃)₂)₂, -C(OH)[P(O)(OH)₂]₂, -Si(CH₃)₂(C(CH₃)₃), -Si(C₂H₅)₃, -Si(CH₃)₃, -N₃, -CN, -OCN, -NCO, -SCN, -NCS, -CHO, -COCH₃, -COC₂H₅, -COC₃H₇, -CO-cyclo-C₃H₅, -COCH(CH₃)₂, -COC(CH₃)₃, -COOH, -COCN, -COOCH₃, -COOC₂H₅, -COOC₃H₇, -COO-cyclo-C₃H₅, -COOCH(CH₃)₂, -COOC(CH₃)₃, -OOC-CH₃, -OOC-C₂H₅, -OOC-C₃H₇, -OOC-cyclo.C₃H₅, -OOC-CH(CH₃)₂, -OOC-C(CH₃)₃, -CONH₂, -CONHCH₃, -CONHC₂H₅, -CONHC₃H₇, -CONH-cyclo-C₃H₅, -CONH[CH(CH₃)₂], -CONH[C(CH₃)₃], -CON(CH₃)₂, -CON(C₂H₅)₂, -CON(C₃H₇)₂, -CON(cyclo-C₃H₅)₂, -CON[CH(CH₃)₂]₂, -CON[C(CH₃)₃]₂, -NHCOCH₃, -NHCOC₂H₅, -NHCOC₃H₇, -NHCO-cyclo-C₃H₅, -NHCO-CH(CH₃)₂, -NHCO-C(CH₃)₃, -NHCO-OCH₃, -NHCO-OC₂H₅, -NHCO-OC₃H₇, -NHCO-O-cyclo-C₃H₅, -NHCO-OCH(CH₃)₂, -NHCO-OC(CH₃)₃, -NH₂, -NHCH₃, -NHC₂H₅, -NHC₃H₇, -NH-cyclo-C₃H₅, -NHCH(CH₃)₂, -NHC(CH₃)₃, -N(CH₃)₂, -N(C₂H₅)₂, -N(C₃H₇)₂, -N(cyclo-C₃H₅)₂, -N[CH(CH₃)₂]₂, -N[C(CH₃)₃]₂, -SOCH₃, -SOC₂H₅, -SOC₃H₇, -SO-cycl-C₃H₅, -SOCH(CH₃)₂, -SOC(CH₃)₃, -SO₂CH₃, -SO₂C₂H₅, -SO₂C₃H₇, -SO₂-cyclo-C₃H₅, -SO₂CH(CH₃)₂, -SO₂C(CH₃)₃, -SO₃H, -sO₃CH₃, -SO₃C₂H₅, -SO₃C₃H₇, -SO₃-cyclo-C₃H₅, -SO₃CH(CH₃)₂, -SO₃C(CH₃)₃, -SO₂NH₂, -SO₂NHCH₃, -SO₂NHC₂H₅, -SO₂NHC₃H₇, -SO₂NH-cyclo-C₃H₅, -SO₂NHCH(CH₃)₂, -SO₂NHC(CH₃)₃, -SO₂N(CH₃)₂, -SO₂N(C₂H₅)₂, -SO₂N(C₃H₇)₂, -SO₂N(cyclo-C₃H₅)₂, -SO₂N[CH(CH₃)₂]₂, -SO₂N[C(CH₃)₃]₂, -O-S(=O)CH₃, -O-S(=O)C₂H₅, -O-S(=O)C₃H₇, -O-S(=O)-cyclo-C₃H₅, -O-S(=O)CH(CH₃)₂, -O-S(=O)C(CH₃)₃, , -S(=O)(=NH)CH₃, -S(=O)(=NH)C₂H₅, -S(=O)(=NH)C₃H₇, -S(=O)(=NH)-cyclo-C₃H₅, -S(=O)(=NH)CH(CH₃)₂, -S(=O)(=NH)C(CH₃)₃, -NH-SO₂-CH₃, -NH-SO₂-C₂H₅, -NH-SO₂-C₃H₇, -NH-SO₂-cyclo-C₃H₅, -NH-SO₂-CH(CH₃)₂, -NH-SO₂-C(CH₃)₃, -O-SO₂-CH₃, -O-SO₂-C₂H₅, -O-SO₂-C₃H₇, -O-SO₂-cyclo-C₃-H₅, -O-SO₂-CH(CH₃)₂, -O-SO₂-C(CH₃)₃, -OCF₃, -CH₂-OCF₃, -C₂H₄-OCF₃, -C₃H₆-OCF₃, -OC₂F₅, -CH₂-OC₂F₅, -C₂H₄-OC₂F₅, -C₃H₆-OC₂F₅, -O-COOCH₃, -O-COOC₂H₅, -O-COOC₃H₇, -O-COO-cyclo-C₃H₅, -O-COOCH(CH₃)₂, -O-COOC(CH₃)₃, -NH-CO-NH₂, -NH-CO-N HCH₃, -NH-CO-NHC₂H₅, -NH-CS-N(C₃H₇)₂, -NH-CO-NHC₃H₇, -NH-CO-N(C₃H₇)₂, -NH-CO-NH[CH(CH₃)₂], -NH-CO-NH[C(CH₃)₃], -NH-CO-N(CH₃)₂, -NH-CO-N(C₂H₅)₂, -NH-CO-NH-cyclo-C₃H₅, -NH-CO-N(cyclo-C₃H₅)₂, -NH-CO-N[CH(CH₃)₂]₂, -NH-CS-N(C₂H₅)₂, -NH-CO-N[C(CH₃)₃]₂, -NH-CS-NH₂, -NH-CS-NHCH₃, -NH-CS-N(CH₃)₂, -NH-CS-NHC₂H₅, -NH-CS-NHC₃H₇, -NH-CS-NH-cyclo-C₃H₅, -NH-CS-NH[CH(CH₃)₂], -NH-CS-NH[C(CH₃)₃], -NH-CS-N(cyclo-C₃H₅)₂, -NH-CS-N[CH(CH₃)₂]₂, -NH-CS-N[C(CH₃)₃]₂, -NH-C(=NH)-NH₂, -NH-C(=NH)-NHCH₃, -NH-C(=NH)-NHC₂H₅, -NH-C(=NH)-NHC₃H₇, -O-CO-NH-cyclo-C₃H₅, -NH-C(=NH)-NH-cyclo-C₃H₅, -NH-C(=NH)-NH[CH(CH₃)₂], -O-CO-NH[CH(CH₃)₂], -NH-C(=NH)-NH[C(CH₃)₃], -NH-C(=NH)-N(CH₃)₂, -NH-C(=NH)-N(C₂H₅)₂, -NH-C(=NH)-N(C₃H₇)₂, -NH-C(=NH)-N(cyclo-C₃H₅)₂, -O-CO-NHC₃H₇, -NH-C(=NH)N[CH(CH₃)₂]₂, -NH-C(=NH)-N[C(CH₃)₃]₂, -O-CO-NH₂, -O-CO-NHCH₃, -O-CO-NHC₂H₅, -O-CO-NH[C(CH₃)₃], -O-CO-N(CH₃)₂, -O-CO-N(C₂H₅)₂, -O-CO-N(C₃H₇)₂, -O-CO-N(cyclo-C₃H₅)₂, -O-CO-N[CH(CH₃)₂]₂, -O-CO-N[C(CH₃)₃]₂, -O-CO-OCH₃, -O-CO-OC₂H₅, -O-CO-OC₃H₇, -O-CO-O-cyclo-C₃H₅, -O-CO-OCH(CH₃)₂, -O-CO-OC(CH₃)₃, -CH₂F, -CHF₂, -CF₃, -CH₂Cl, -CH₂Br, -CH₂l, -CH₂-CH₂F, -CH₂-CHF₂, -CH₂-CF₃, -CH₂-CH₂Cl, -CH₂-CH₂Br, -CH₂-CH₂l, -cyclo-C₈H₁₅, -Ph, -CH₂-Ph, -CH₂-CH₂-Ph, -CH=CH-Ph, -CPh₃, -CH₃, -C₂H₅, -C₃H₇, -CH(CH₃)₂, -C₄H₉, -CH₂-CH(CH₃)₂, -CH(CH₃)-C₂H₅, -C(CH₃)₃, -C₅H₁₁, -CH(CH₃)-C₃H₇, -CH₂-CH(CH₃)-C₂H₅, -CH(CH₃)-CH(CH₃)₂, -C(CH₃)₂-C₂H₅, -CH₂-C(CH₃)₃, -CH(C₂H₅)₂, -C₂H₄-CH(CH₃)₂, -C₆H₁₃₉ -C₇H₁₅, -C₈H₁₇, -C₃H₆-CH(CH₃)₂, —C₂H₄—CH(CH₃)—C₂H₅, -CH(CH₃)-C₄H₉, -CH₂-CH(CH₃)-C₃H₇, -CH(CH₃)-CH₂-CH(CH₃)₂, -CH(CH₃)-CH(CH₃)-C₂H₅, -CH₂-CH(CH₃)-CH(CH₃)₂, -CH₂-C(CH₃)₂-C₂H₅, -C(CH₃)₂-C₃H₇, -C(CH₃)₂-CH(CH₃)₂, -C₂H₄-C(CH₃)₃, -CH(CH₃)-C(CH₃)₃, -CH=CH₂, -CH₂-CH=CH₂, -C(CH₃)=CH₂, -CH=CH-CH₃, -C₂H₄-CH=CH₂, -CH₂-CH=CH-CH₃, -CH=CH-C₂H₅, -CH₂-C(CH₃)=CH₂, -CH(CH₃)-CH=CH, -CH=C(CH₃)₂, -C(CH₃)=CH-CH₃, -CH=CH-CH=CH₂, -C₃H₆-CH=CH₂, -C₂H₄-CH=CH-CH₃, -CH₂-CH=CH-C₂H₅, -CH=CH-C₃H₇, -CH₂-CH=CH-CH=CH₂, -CH=CH-CH=CH-CH₃, -CH=CH-CH₂-CH=CH₂, -C(CH₃)=CH-CH=CH₂, -CH=C(CH₃)-CH=CH₂, -CH=CH-C(CH₃)=CH₂, -C₂H₄-C(CH₃)=CH₂, -CH₂-CH(CH₃)-CH=CH₂, -CH(CH₃)-CH₂-CH=CH₂, -CH₂-CH=C(CH₃)₂, -CH₂-C(CH₃)=CH-CH₃, -CH(CH₃)-CH=CH-CH₃, -CH=CH-CH(CH₃)₂, -CH=C(CH₃)-C₂H₅, -C(CH₃)=CH-C₂H₅, -C(CH₃)=C(CH₃)₂, -C(CH₃)₂-CH=CH₂, -CH(CH₃)-C(CH₃)=CH₂, -C(CH₃)=CH-CH=CH₂, -CH=C(CH₃)-CH=CH₂, -CH=CH-C(CH₃)=CH₂, -C₄H₈-CH=CH₂, -C₃H₆-CH=CH-CH₃, -C₂H₄-CH=CH-C₂H₅, -CH₂-CH=CH-C₃H₇, -CH=CH-C₄H₉, -C₃H₆-C(CH₃)=CH₂, -C₂H₄-CH(CH₃)-CH=CH₂, -CH₂-CH(CH₃)-CH₂-CH=CH₂, -C₂H₄-CH=C(CH₃)₂, -CH(CH₃)-C₂H₄-CH=CH₂, -C₂H₄-C(CH₃)=CH-CH₃, -CH₂-CH(CH₃)-CH=CH-CH₃, -CH(CH₃)-CH₂-CH=CH-CH₃, -CH₂-CH=CH-CH(CH₃)₂, -CH₂-CH=C(CH₃)-C₂H₅, -CH₂-C(CH₃)=CH-C₂H₅, -CH(CH₃)-CH=CH-C₂H₅, -CH=CH-CH₂-CH(CH₃)₂, -CH=CH-CH(CH₃)-C₂H₅, -CH=C(CH₃)-C₃H₇, -C(CH₃)=C_{H}-C₃H₇, -CH₂-CH(CH₃)-C(CH₃)=CH₂, -C[C(CH₃)₃]=CH₂, -CH(CH₃)-CH₂-C(CH₃)=CH₂, -CH(CH3)-CH(CH₃)-CH=CH₂, -CH=CH-C₂H₄-CH=CH₂, -CH₂-C(CH₃)₂-CH=CH₂, -C(CH₃)₂-CH₂-CH=CH₂, -CH₂-C(CH₃)=C(CH₃)₂, -CH(CH₃)-CH=C(CH₃)₂, -C(CH₃)₂-CH=CH-CH₃, -CH=CH-CH₂-CH=CH-CH₃, -CH(CH₃)-C(CH₃)=CH-CH₃, -CH=C(CH₃)-CH(CH₃)₂, -C(CH₃)=CH-CH(CH₃)₂, -C(CH₃)=C(CH₃)-C₂H₅, -CH=CH-C(CH₃)₃, -C(CH₃)₂-C(CH₃)=CH₂, -CH(C₂H₅)-C(CH₃)=CH₂, -C(CH₃)(C₂H₅)-CH=CH₂, -CH(CH₃)-C(C₂H₅)=CH₂, -CH₂-C(C₃H₇)=CH₂, -CH₂-C(C₂H₅)=CH-CH₃, -CH(C₂H₅)-CH=CH-CH₃, -C(C₄H₉)=CH₂, -C(C₃H₇)=CH-CH3, -C(C₂H₅)=CH-C₂H₅, -C(C₂H₅)=C(CH₃)₂, -C[CH(CH₃)(C₂H₅)]=CH₂, -C[CH₂-CH(CH₃)₂]=CH₂, -C₂H₄-CH=CH-CH=CH₂, -CH₂-CH=CH-CH₂-CH=CH₂, -C₃H₆-C≡C-CH₃, -CH₂-CH=CH-CH=CH-CH₃, -CH=CH-CH=CH-C₂H₅, -CH₂-CH=CH-C(CH₃)=CH₂, -CH₂-CH=C(CH₃)-CH=CH₂, -CH₂-C(CH₃)=CH-CH=CH₂, -CH(CH₃)-CH₂-C≡CH, -CH(CH₃)-CH=CH-CH=CH₂, -CH=CH-CH₂≡C(CH₃)=CH₂, -CH(CH₃)-C≡C-CH₃, -CH=CH-CH(CH₃-CH=CH₂, -CH=C(CH₃)-CH₂-CH=CH₂, -C₂H₄-CH(CH₃)-C≡CH, -C(CH₃)=CH-CH₂-CH=CH₂, -CH=CH-CH=C(CH₃)₂, -CH₂-CH(CH₃)-CH₂-C≡CH, -CH=CH-C(CH₃)=CH-CH₃, -CH=C(CH₃)-CH=CH-CH₃, -CH₂-CH(CH₃)-C≡CH, -C(CH₃)=CH-CH=CH-CH₃, -CH=C(CH₃)-C(CH₃)=CH₂, -C(CH₃)=CH-C(CH₃)=CH₂, -C(CH₃)=C(CH₃)-CH=CH₂, -CH=CH-CH=CH-CH=CH₂, -C=CH, -C=C-CH₃, -CH₂-C=CH, -C₂H₄-C=CH, -CH₂-C=C-CH₃, -C=C-C₂H₅, -C₃H₆-C=CH, -C₂H₄-C=C-CH₃, -CH₂-C≡C-C₂H₅, -C=C-C₃H₇, -CH(CH₃)-C=CH, -C₄H₈-C≡CH, -C₂H₄-C≡C-C₂H₅, -CH2-C≡C-C₃H₇, -C=C-C₄H₉, -C=C-C(CH₃)₃, -CH(CH₃)-C₂H₄-C≡CH, -CH₂-CH(CH₃)-C≡C-CH₃, -CH(CH₃)-CH₂-C≡C-CH₃, -CH(CH₃)-C=C-C₂H₅, -CH₂-C≡C-CH(CH₃)₂, -C≡C-CH(CH₃)-C₂H₅, -C=C-CH₂-CH(CH₃)₂, -CH(C₂H₅)-C≡C-CH₃, -C(CH₃)₂-C≡C-CH₃, -CH(C₂H₅)-CH₂-C=CH, -CH₂-CH(C₂H₅)-C≡CH, -C(CH₃)₂-CH₂-C≡CH, -CH₂-C(CH₃)₂-C≡CH, -CH(CH₃)-CH(CH₃)-C≡CH, -CH(C₃H₇)-C≡CH, -C(CH₃)(C₂H₅)-C≡CH, -CH₂-CH(C≡CH)₂, -C≡C-C≡CH, -CH₂-C≡C-C≡CH, -C≡C-C≡C-CH₃, -CH(C=CH)₂, -C₂H₄-C≡C-C≡CH, -CH₂-C≡C-CH₂-C≡CH, -C≡C-C₂H₄-C≡CH, -CH₂-C≡C-C≡C-CH₃, -C≡C-CH₂-C≡C-CH₃, -C≡C-C≡C-C₂H₅, -C(C≡CH)₂-CH₃, -C≡C-CH(CH₃)-C≡CH, -CH(CH₃)-C≡C-C≡CH, -CH(C≡CH)-CH₂-C≡CH, -CH(C≡CH)-C≡C-CH₃, 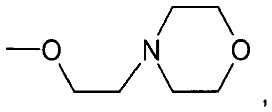 R^{c} represents -CH₂-OCH₃, -C₂H₄-OCH₃, -C₃H₆-OCH₃, -CH₂-OC₂H₅, -C₂H₄-OC₂H₅, -C₃H₆-OC₂H₅, -CH₂-OC₃H₇, -C₂H₄-OC₃H₇, -C₃H₆-OC₃H₇, -CH₂-O-cyclo-C₃H₅, -C₂H₄-O-cyclo-C₃H₅, -C₃H₆O-cyclo-C₃H₅, -CH₂-OCH(CH₃)₂, -C₂H₄-OCH(CH₃)₂, -C₃H₆-OCH(CH₃)₂, -CH₂-OC(CH₃)₃, -C₂H₄-OC(CH₃)₃, -C₃H₆-OC(CH₃)₃, -CH₂-OC₄H₉, -C₂H₄-OC₄H₉, -C₃H₆-OC₄H₉, -CH₂-OPh, -C₂H₄-OPh, -C₃H₆-OPh, -CH₂-OCH₂-Ph, -C₂H₄-OCH₂-Ph, -C₃H₆-OCH₂-Ph, -CH₂F, -CHF₂, -CF₃, -CH₂Cl, -CH₂Br, -CH₂I, -CH₂-CH₂F, -CH₂-CHF₂, -CH₂-CF₃, -CH₂-CH₂Cl, -CH₂-CH₂Br, -CH₂-CH₂I, -cyclo-C₈H₁₅, -Ph, -CH₂-Ph, -CH₂-CH₂-Ph, -CH=CH-Ph, -CPh₃, -CH₃, -C₂H₅, -C₃H₇, -CH(CH₃)₂, -C₄H₉, -CH₂-CH(CH₃)₂, -CH(CH₃)-C₂H₅, -C(CH₃)₃, -C₅H₁₁, -CH(CH₃)-C₃H₇, -CH₂-CH(CH₃)-C₂H₅, -CH(CH₃)-CH(CH₃)₂, -C(CH₃)₂-C₂H₅, -CH₂-C(CH₃)₃, -CH(C₂H₅)₂, -C₂H₄-CH(CH₃)₂, -C₆H_{13,} -C₇H₁₅, -C₈H_{17,} -C₃H₆-CH(CH₃)₂, -C₂H₄-CH(CH₃)-C₂H₅, -CH(CH₃)-C₄H₉, -CH₂-CH(CH₃)-C₃H₇, -CH(CH₃)-CH₂-CH(CH₃)₂, -CH(CH₃)-CH(CH₃)-C₂H₅, -CH₂-CH(CH₃)-CH(CH₃)₂, -CH₂-C(CH₃)₂-C2H₅, -C(CH₃)₂-C₃H₇, -C(CH₃)₂-CH(CH₃)₂, -C₂H₄-C(CH₃)₃, -CH(CH₃)-C(CH₃)₃, -CH=CH₂, -CH₂-CH=CH₂, -C(CH₃)=CH₂, -CH=CH-CH₃, -C₂H₄-CH=CH₂, -CH₂-CH=CH-CH₃, -CH=CH-C₂H₅, -CH₂-C(CH₃)=CH₂, -CH(CH₃)-CH=CH, -CH=C(CH₃)₂, -C(CH₃)=CH-CH₃, -CH=CH-CH=CH₂, -C₃H₆-CH=CH₂, -C₂H₄-CH=CH-CH₃, -CH₂-CH=CH-C₂H₅, -CH=CH-C₃H₇, -CH₂-CH=CH-CH=CH₂, -CH=CH-CH=CH-CH₃, -CH=CH-CH₂-CH=CH₂, -C(CH₃)=CH-CH=CH₂, -CH=C(CH₃)-CH=CH₂, -CH=CH-C(CH₃)=CH₂, -C₂H₄-C(CH₃)=CH₂, -CH₂-CH(CH₃)-CH=CH₂, -CH(CH₃)-CH₂-CH=CH₂, -CH₂-CH=C(CH₃)₂, -CH₂-C(CH₃)=CH-CH₃, -CH(CH₃)-CH=CH-CH₃, -CH=CH-CH(CH₃)₂, -CH=C(CH₃)-C₂H₅, -C(CH₃)=CH-C₂H₅, -C(CH₃)=C(CH₃)₂, -C(CH₃)₂-CH=CH₂, -CH(CH₃)-C(CH₃)=CH₂, -C(CH₃)=CH-CH=CH₂, -CH=C(CH₃)-CH=CH₂, -CH=CH-C(CH₃)=CH₂, -C₄H₈-CH=CH₂, -C₃H₆-CH=CH-CH₃, -C₂Ha-CH=CH-C₂H₅, -CH₂-CH=CH-C₃H₇, -CH=CH-C₄H₉, -C₃H₆-C(CH₃)=CH₂, -C₂H₄-CH(CH₃)-CH=CH₂, -CH₂-CH(CH₃)-CH₂-CH=CH₂, -C₂H₄-CH=C(CH₃)₂, -CH(CH₃)-C₂H₄-CH=CH₂, -C₂H₄-C(CH₃)=CH-CH₃, -CH₂-CH(CH₃)-CH=CH-CH₃, -CH(CH₃)-CH₂-CH=CH-CH₃, -CH₂-CH=CH-CH(CH₃)₂, -CH₂-CH=C(CH₃)-C₂H₅, -CH₂-C(CH₃)=CH-C₂H₅, -CH(CH₃)-CH=CH-C₂H₅, -CH=CH-CH₂-CH(CH₃)₂, -CH=CH-CH(CH₃)-C₂H₅, -CH=C(CH₃)-C₃H₇, -C(CH₃)=CH-C₃H₇, -CH₂-CH(CH₃)-C(CH₃)=CH₂, -C[C(CH₃)₃]=CH₂, -CH(CH₃)-CH₂-C(CH₃)=CH₂, -CH(CH₃)-CH(CH₃)-CH=CH₂, -CH=CH-C₂H₄-CH=CH₂, -CH₂-C(CH₃)₂-CH=CH₂, -C(CH₃)₂-CH₂-CH=CH₂, -CH₂-C(CH₃)=C(CH₃)₂, -CH(CH₃)-CH=C(CH₃)₂, -C(CH₃)₂-CH=CH-CH₃, -CH=CH-CH₂-CH=CH-CH₃, -CH(CH₃)-C(CH₃)=CH-CH₃, -CH=C(CH₃)-CH(CH₃)₂, -C(CH₃)=CH-CH(CH₃)₂, -C(CH₃)=C(CH₃)-C₂H₅, -CH=CH-C(CH₃)₃, -C(CH₃)₂-C(CH₃)=CH₂, -CH(C₂H₅)-C(CH₃)=CH₂, -C(CH₃)(C₂H₅)-CH=CH₂, -CH(CH₃)-C(C₂H₅)=CH₂, -CH₂-C(C₃H₇)=CH₂, -CH₂-C(C₂H₅)=CH-CH₃, -CH(C₂H₅)-CH=CH-CH₃, -C(C₄H₉)=CH₂, -C(C₃H₇)=CH-CH₃, -C(C₂H₅)=CH-C₂H₅, -C(C₂H₅)=C(CH₃)₂, -C[CH(CH₃)(C₂H₅)]=CH₂, -C[CH₂-CH(CH₃)₂]=CH₂, -C₂H₄-CH=CH-CH=CH₂, -CH₂-CH=CH-CH₂-CH=CH₂, -C₃H₆-C≡C-CH₃, -CH₂-CH=CH-CH=CH-CH₃, -CH=CH-CH=CH-C₂H₅, -CH₂-CH=CH-C(CH₃)=CH₂, -CH₂-CH=C(CH₃)-CH=CH₂, -CH₂-C(CH₃)=CH-CH=CH₂, -CH(CH₃)-CH₂-C≡CH, -CH(CH₃)-CH=CH-CH=CH₂, -CH=CH-CH₂-C(CH₃)=CH₂, -CH(CH₃)-C≡C-CH₃, -CH=CH-CH(CH₃)-CH=CH₂, -CH=C(CH₃)-CH₂-CH=CH₂, -C₂H₄-CH(CH₃)-C≡CH, -C(CH₃)=CH-CH₂-CH=CH₂, -CH=CH-CH=C(CH₃)₂, -CH₂-CH(CH₃)-CH₂-C≡CH, -CH=CH-C(CH₃)=CH-CH₃, -CH=C(CH₃)-CH=CH-CH₃, -CH₂-CH(CH₃)-C≡CH, -C(CH₃)=CH-CH=CH-CH₃, -CH=C(CH3)-C(CH3)=CH₂, -C(CH₃)=CH-C(CH₃)=CH₂, -C(CH₃)=C(CH₃)-CH=CH₂, -CH=CH-CH=CH-CH=CH₂, -C≡CH, -C≡C-CH₃, -CH₂-C≡CH, -C₂H₄-C≡CH, -CH₂-C≡C-CH₃, -C≡C-C₂H₅, -C₃H₆-C≡CH, -C₂H₄-C≡C-CH₃, -CH₂-C≡C-C₂H₅, -C≡C-C₃H₇, -CH(CH₃)-C≡CH, -C₄H₈-C≡CH, -C₂H₄-C≡C-C₂H₅, -CH₂-C≡C-C₃H₇, -C≡C-C₄H₉, -C≡C-C(CH₃)₃, -CH(CH₃)-C₂H₄-C≡CH, -CH₂-CH(CH₃)-C≡C-CH₃, -CH(CH₃)-CH₂-C≡C-CH₃, -CH(CH₃)-C≡C-C₂H₅, -CH₂-C≡C-CH(CH₃)₂, -C≡C-CH(CH₃)-C₂H₅, -C≡C-CH₂-CH(CH₃)₂, -CH(C₂H₅)-C≡C-CH₃, -C(CH₃)₂-C≡C-CH₃, -CH(C₂H₅)-CH₂-C≡CH, -CH₂-CH(C₂H₅)-C≡CH, -C(CH₃)₂-CH₂-C≡CH, -CH₂-C(CH₃)₂-C≡CH, -CH(CH₃)-CH(CH₃)-C≡CH, -CH(C₃H₇)-C=CH, -C(CH₃)(C₂H₅)-C≡CH, -CH₂-CH(C≡CH)₂, -C≡C-C≡CH, -CH₂-C≡C-C≡CH, -C≡C-C≡C-CH₃, -CH(C≡CH)₂, -C₂H₄-C≡C-C≡CH, -CH₂-C≡C-CH₂-C≡CH, -C≡C-C₂H₄-C≡CH, -CH₂-C≡C-C≡C-CH₃, -C≡C-CH₂-C≡C-CH₃, -C≡C-C≡C-C₂H₅, -C(C≡CH)₂-CH₃, -C≡C-CH(CH₃)-C≡CH, -CH(CH₃)-C≡C-C≡CH, -CH(C≡CH)-CH₂-C≡CH, -CH(C≡CH)-C≡C-CH₃, and enantiomers, stereoisomeric forms, mixtures of enantiomers, anomers, deoxy-forms, diastereomers, mixtures of diastereomers, prodrugs, tautomers, hydrates, solvates and racemates of the above mentioned compounds and pharmaceutically acceptable salts thereof, for use as inhibitor of the FK506-binding protein FKBP51 and FKBP52.

6. Compound according to claim 5 selected from the group consisting of:
2-(3-((R)-3-(3,4-dimethoxyphenyl)-1-((S)-1-(2-oxo-2-(3,4,5-trimethoxyphen yl)acetyl)piperidine-2-carbonyloxy)propyl)phenoxy)acetic acid
(S)-((R) -3-(3,4-dimethoxyphenyl) -1-(3-(2-morpholinoethoxy)phenyl) propyl) 1-(3,3-dimethyl-2-oxopentanoyl)pyrrolidine-2-carboxylate
(S)-((R) -3-(3,4-dimethoxyphenyl) -1-(3-(2-morpholinoethoxy) phenyl) propyl) 1-(3,3-dimethyl-2-oxopentanoyl)piperidine-2-carboxylate
2-(3-((R) -3-(3,4-dimethoxyphenyl) -1-((S)-1-(3,3-dimethyl-2-oxopentanoyl)-1,2,3,6-tetrahydropyridine-2-carbonyloxy) propyl)phenoxy)acetic acid
2-(3-((R)-3-(3,4-dimethoxyphenyl)-1-((S)-1-(3,3-dimethyl-2-oxopentanoyl)pyrrolidine-2-carbonyloxy)propyl)phenoxy)acetic acid
2-(3-((R)-3-(3,4-dimethoxyphenyl)-1-((S)-1-(3,3-dimethyl-2-oxopentanoyl) piperidine-2-carbonyloxy)propyl)phenoxy)acetic acid
3,3-dimethyl-2-oxopentanamide (1R)-1-(3-aminophenyl) -3-(3,4-dimethoxyphenyl)propyl cyclohexanecarboxylate
2-{3-[(1R)-3-(3,4-dimethoxyphenyl)-1-({1-[2-oxo-2-(3,4,5-trimethoxyphenyl)acetyl]piperidin-2-yl}carbonyloxy)propyl]phenoxy}acetic acid
2-(3-((R)-3-(3,4-dimethoxyphenyl)-1-((S)-1-(2-((1S,2R)-1-hydroxy-2-methyl cyclohexyl)-2-oxoacetyl)piperidine-2-carbonyloxy)propyl)phenoxy)acetic acid
3,4-dimethoxyphenethyl 1-(2-((1S,2R)-1-hydroxy-2-methytcyc)ohexy!)-2-oxo-acetyl)-piperidine-2-carboxylate
2-(3,4-dimethoxyphenoxy)ethyl 1-(2-((1S,2R)-1-hydroxy-2-methylcyclohexyl)-2-oxoacetyl)piperidine-2-carboxylate
2-(3,4-dimethoxyphenoxy)ethyl 1-(2-((1S,2R)-2-ethyl-1-hydroxycyclohexyl)-2-oxoacetyl)piperidine-2-carboxylate
(S)-((R)-3-(3,4-dimethoxyphenyl)-1-(3-(2-morpholinoethoxy)phenyl)propyl) 1-(2-((1S, 2R)-1-hydroxy-2-methylcyclohexyl)-2-oxoacetyl)piperidine-2-carboxylate
2-(3-((R)-3-(3,4-dimethoxyphenyl)-1-((S)-1-(2-((1S,2R)-2-ethyl-1-hydroxy-cyclohexyl)-2-oxoacetyl)piperidine-2-carbonyloxy)propyl)phenoxy)acetic acid
(S)-((R)-3-(3,4-dimethoxyphenyl)-1-(3-(2-morpholinoethoxy)phenyl)propyl)1-(2-((1 S, 2R) -2-ethyl-1-hydroxycyclohexyl)-2-oxoacetyl)piperidine-2-carboxylate
2-(3-((R)-3-(3,4-dimethoxyphenyl)-1-((S)-1-(2-((1S,2S)-1-hydroxy-2-(hydroxyl methyl) cyclohexyl)-2-oxoacetyl)piperidine-2-carbonyloxy)propyl)phenoxy)acetic acid
2-(3-((R)-3-(3,4-dimethoxyphenyl)-1-((S)-1-(2-((1R,2R)-1-hydroxy-2-methyl cyclohexyl)-2-oxoacetyl)piperidine-2-carbonyloxy)propyl)phenoxy)acetic acid
2-(3-((R)-3-(3,4-dimethoxyphenyl)-1-((S)-1-(2-((1R,2R)-2-ethyl-1-hydroxy cyclo hexyl)-2-oxoacetyl)piperidine-2-carbonyloxy)propyl)phenoxy)acetic acid

7. Compound according to claim 5 or 6 for use in treatment or prophylaxis of a disease selected from psychiatric disorder, neurological disorder, cancer, transplant rejection or metabolic disorders, glucocorticoid hyposensitivity syndrome, peripheral glucocorticoid resistance, infectious diseases, alopecia, abnormally elevated intraocular pressure, macular degeneration, oxidative damage to eye tissues, vision disorder, memory impairment, and
for neuroprotection, neuroregeneration, promoting hair growth, stimulating neurite growth, wound healing, antiglaucomatous medications, improving vision, enhancing memory performance, and
for the use in treatment or prevention of multi-drug resistance,
for the use in induced abortion and the inhibition of embryo implantation,
for the use in limiting or preventing hemorrhage or neovascularization, and
for the use in treatment of diseases relating to neurodegeneration.

8. Compound according to claim 7, wherein the psychiatric disease is an affective disorder or an anxiety disorder.

9. Compound according to claim 8, wherein the affective disorder is selected from the group consisting of: depression, bipolar disorder, mania, substance induced mood disorder and seasonal affective disorder (SAD).

10. Compound according to claim 8, wherein the anxiety disorder is selected from the group comprising or consisting of generalized anxiety disorder, panic disorder, panic disorder with agoraphobia, phobias, obsessive-compulsive disorder, post-traumatic stress disorder, separation anxiety and childhood anxiety disorders.

11. Compound according to claim 7, wherein the infectious disease is selected from the group consisting of: malaria and Legionnaires' disease.

12. Pharmaceutical composition comprising at least one compound according to claim 5 together with at least one pharmaceutically acceptable carrier, solvent or excipient.

13. Pharmaceutical composition according to claim 12 further comprising at least one active agent selected from the group consisting of an anti-depressant and other psychotropic drugs.

14. Pharmaceutical composition according to claim 12 or 13, wherein the anti-depressant is selected from amitriptyline, amioxide clomipramine, doxepine, duloxetine, imipramine trimipramine, mirtazapine, reboxetine, citaloprame, fluoxetine, moclobemide and sertraline.
